# EUROPEAN PATENT APPLICATION

(11) **EP 4 721 768 A1**
(43) Date of publication of application: **08.04.2026**
(21) Application number: 24809205.8
(22) Date of filing: 24.05.2024
(51) Int. Cl.: A61K 51/08, A61K 47/62, A61K 47/54, A61P 35/00, A61P 25/00, A61P 9/10

(54) **NOVEL TRIFUNCTIONAL COMPOUND AND USE THEREOF**

(30) Priority: 24.05.2023 WO PCT/CN2023/096106
(71) Applicant: Beijing Changping Laboratory, Beijing 102206 (CN)
(72) Inventor: LIU, Zhibo, Beijing 100871 (CN); CUI, Xiyang, Beijing 100871 (CN)
(74) Representative: Gramm, Lins & Partner Patent- und Rechtsanwälte PartGmbB
(86) International application number: PCT/CN2024/095234
(87) International publication number: WO 2024/240251

(57) **Abstract**

A novel trifunctional compound, comprising at least one target object T, and at least one payload P. In addition to a covalent warhead which may be carried in the target object T, the trifunctional compound further comprises at least one covalent warhead C which can be reversibly or irreversibly covalently linked to a protein or tissue (such as FAP protein or other biomolecules in vivo). The present invention also relates to a pharmaceutical composition and kit comprising the trifunctional compound, and the use of the trifunctional compound for diagnosing or treating a disease.

## Description

### TECHNICAL FIELD

The present disclosure relates to the field of medicine, and specifically, to a trifunctional compound containing a covalent warhead, its pharmaceutical composition, and its use in diagnosis or treatment of diseases.

### BACKGROUND

Radiotherapy, surgery, and chemotherapy are traditionally three major first-line therapy modalities used by humans to combat cancer. Radiopharmaceuticals ("RP Drug" for short) can be viewed as internal irradiation in radiotherapy. Compared with conventional radiotherapy, radiopharmaceuticals have an advantage of so-called powerful "crossfire effect" and are one of the few treatment options for patients with advanced cancer and systemic metastases. From the perspective of mechanism of radiopharmaceuticals, high-energy ionizing radiation is generated via decay of medical isotopes to break DNA strands, thereby inducing death of cancer cells. Therefore, the radiopharmaceuticals need to have extremely high tumor selectivity/targeting ability, as well as a certain level of tumor uptake and retention, to deliver sufficient radiation specifically to tumors only while restricting damage to surrounding normal tissues.

Radionuclide Ligand Conjugates (RLCs) are a major type of radiopharmaceutical. They are generally binary conjugates of chelator-linker-targeting ligand. The ligand moiety can be a small molecule, a polypeptide, an aptamer, an antibody, or the like. The linker mainly functions as a spacer to prevent the conjugated chelator from greatly lowering affinity of the targeting molecule, with certain regulatory effect on pharmacokinetic properties. According to different diagnostic and therapeutic needs, the chelator part can coordinate different diagnostic/therapeutic nuclides with the same molecule or by replacing the chelator, which has the advantage of "integration of diagnosis and treatment". Therefore, some molecules with general targeting and safety but high tumor uptake still have the potential for molecular imaging diagnosis. Diagnostic radionuclides can be classified into two categories: positron-emitting radionuclides (for example, ¹⁸F, ⁶⁸Ga, ⁶⁴Cu, ⁸⁶Y, and ⁸⁹Zr) that can be traced by positron emission tomography-computed tomography (PET-CT); and radionuclides (for example, ^{99m}Tc, ¹³³Xe, ¹²³I, and ^{91m}Kr) that emit γ-rays and that can be traced by single-photon emission computed tomography-computed tomography (SPECT-CT). Therapeutic radionuclides can be classified into β-emitters (for example, ⁹⁰Y, ¹²⁴I, ¹⁵¹Tb, and ¹⁷⁷Lu) and α-emitters (for example, ²¹¹At, ²¹²Pb, ²¹³Bi, ²²³Ra, and ²²⁵Ac). In addition, replacing the chelating agent moiety with optical dyes with absorption-emission at different wavelengths to obtain optical probes is also used for optical imaging at the cellular, *in vivo,* and other levels.

In recent years, diagnostic/therapeutic RLCs based on prostate-specific membrane antigen (PSMA) and somatostatin receptor (SSTR) have obtained approval from the FDA and the European Union, which encourages more RLC drugs to enter clinical research. Because the PSMA and SSTR are only expressed in prostatic carcinoma and neuroendocrine tumors, respectively, and do not have a broad tumor spectrum, researchers around the world are striving to develop RLC drugs with broader applicability.

As a type II membrane-bound glycoprotein and also a member of the serine protease family, fibroblast activation protein (FAP-α) is overexpressed in cancer-associated fibroblasts (CAPs) and activated fibroblasts at wound healing sites/inflammatory sites. As a broad-spectrum cancer target, the fibroblast activation protein is expressed in over 90% of microenvironments of epithelial tumors, including pancreatic cancer, colon cancer, breast cancer, ENT (ear, nose, and throat) cancer, and the like. In 2019, Kratochwil et al. clinically confirmed that the quinoline-containing small-molecule nuclear drug ⁶⁸Ga-FAPI-04 can detect as many as 28 different types of cancer *(*J. Nucl. Med. 2019, 60, 801*).* Due to its extremely low expression in normal tissues, FAP-α can be used as not only a biomarker for tumor diagnosis and prognoses but also as a promising therapeutic target for radiopharmaceuticals. After Lindner et al. first applied FAPI-04 in clinical practice in 2018 *(*J. Nucl. Med. 2018, 59, 1415*),* PET imaging and radiotherapy based on FAP-α inhibitors (FAPI) have been studied for their use in various cancerous and non-cancerous diseases.

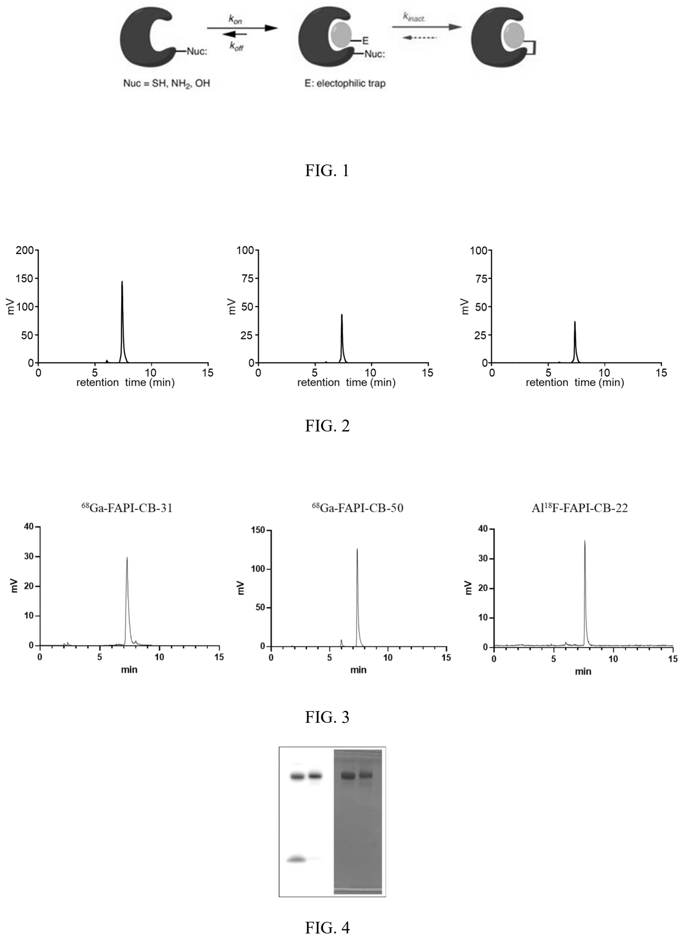

Covalent inhibitors are a type of inhibitors that exert their biological functions by irreversibly binding to target protein residues via covalent bonds. Most covalent inhibitors are small-molecule kinase inhibitors from the perspective of their forms *(*Eur. J. Med. Chem. 2017, 138, 96). In general, covalent inhibition is a two-step process (FIG. 1): First, the inhibitor binds reversibly to the target enzyme, so that the warhead in the small molecule gets closer to an active residue in an enzyme; and second, a bond-forming reaction occurs between the warhead of the inhibitor and the residue to form a covalent bond. Common nucleophilic amino acid residues (nucleophiles) that can undergo covalent reactions include cysteine, serine, tyrosine, lysine, arginine, glutamic acid, and the like. Common covalent warheads are generally electrophiles. Based on reversible or irreversible covalent bonds formed by the covalent warheads, the covalent warheads can be classified into two categories: one category includes warheads that generally form irreversible covalent bonds, for example acrylamide, epoxide, a chloroacetyl group, and sulfonyl fluoride; and the other category includes warheads that generally form reversible covalent bonds, for example a cyano group and a ketone carbonyl group. Covalent warheads that can be used *in vivo* and eventually developed into drugs need to balance activity and stability. Among them, a promising class of covalent warheads is "latent electrophiles", which are activated to accelerate covalent bond formation only when bound to specific proteins, thereby minimizing off-target toxicity associated with warhead activity. In 2014, Sharpless et al. developed a new type of "latent electrophiles", namely, fluorosulfates based on hexavalent sulfur click chemistry (SuFEx) *(*Angew. Chem. Int. Ed. 2014, 53, 9430*).* Compared with the most common acrylamide-based covalent warheads that react only with cysteine residues, fluorosulfates not only exhibit better *in vivo* stability, but also can form covalent bonds with more widely present adjacent lysine, histidine, tyrosine, and serine residues after binding to the target protein, showing the potential as a new class of drug covalent warheads. Similarly, promising covalent warheads for drugs further include phosphorus (V) fluoride-based covalent warheads, for example (hetero)aryl phosphoramidofluoridates.

### SUMMARY OF THE INVENTION

On the basis of the prior art, innovative research has been conducted in the present disclosure. By introducing a covalent warhead containing a (hetero)aryl-substituted halosulfate or a (hetero)aryl-substituted phosphoramide fluoride into a drug including a targeting moiety and a payload, the following unexpected effects are achieved: the pharmacokinetics of the drug are adjusted, and as a result, the uptake and retention of the drug in an organ where a target is located (target organ), for example a tumor site, is significantly improved, while the uptake and retention in non-target organs remain consistently relatively low. For example, uptake in the liver, kidneys, and/or blood pool can be continuously reduced. Therefore, compared with conventional drug modification strategies, the drug engineered via the strategy of the present disclosure has a higher target-to-background ratio, and can optimize performance (including sensitivity and/or specificity) of probe drugs in diagnosis, expand a safe therapeutic window in treatment, and improve bioavailability, thereby improving therapeutic efficacy while ensuring safety.

An aspect of this disclosure provides a trifunctional compound, or a pharmaceutically acceptable salt, stereoisomer or solvate thereof, where the trifunctional compound includes at least one targeting moiety T and at least one payload P, and in addition to any covalent warhead optionally present in the targeting moiety T itself, the trifunctional compound includes at least one covalent warhead C capable of forming a reversible or irreversible covalent bond with a protein or tissue (for example, FAP protein or another biomolecule *in vivo)* targeted by the targeting moiety T, and the covalent warhead C is preferably a covalent warhead C that includes a (hetero)aryl-substituted halosulfate or a (hetero)aryl-substituted phosphoramide fluoride.

In the present disclosure, the trifunctional compound containing the foregoing three functional groups is used, which improves the uptake and/or retention in the target organ (for example, tumors), enhances the sensitivity of the diagnostic probe and efficacy of the therapeutic drug without significantly increasing the binding capacity to plasma albumin, thereby maintaining uptake and retention in the blood pool, liver, kidneys, and other vital organs at a low level and ensuring the specificity of the diagnostic probe and controllable toxicity. Finally, a compound with high diagnostic performance or a broad therapeutic window is provided.

Preferably, by using the trifunctional compound containing the covalent warhead C, the present disclosure enables the compound to significantly improve its uptake and retention in targets such as tumor tissues (enhancing drug efficacy) while retaining a certain degree of targeting (ensuring drug safety).

More preferably, by adjusting or controlling the chain length and conformation of the linker, the trifunctional compound in the present disclosure can achieve a better target-to-background ratio.

More preferably, in the present disclosure, higher detection sensitivity is achieved for targets (for example, tumor tissues) when the payload P is a diagnostic group, and drug efficacy is better achieved when the payload P is a therapeutic group.

In another aspect, the present disclosure introduces a covalent warhead to another modification site, for example, introducing an additional covalent warhead to the payload P, which achieves a better pharmacokinetic effect.

An aspect of the present disclosure relates to a trifunctional compound, or a pharmaceutically acceptable salt, stereoisomer or solvate thereof, where the trifunctional compound includes at least one targeting moiety T and at least one payload P, and in addition to any covalent warhead optionally present in the targeting moiety T itself, the trifunctional compound includes at least one covalent warhead C described as follows.

The payload P is any optical functional group, radiolabeled functional group, radiolabelable functional group, or functional group of a small-molecule cytotoxic drug, which is usable for optical imaging, positron emission tomography, single-photon emission computed tomography, chemotherapy, or radiotherapy.

The targeting moiety T includes a targeting moiety capable of targeting a protein or tissue (for example, FAP protein or other biomolecules *in vivo),* and the targeting moiety is a small molecule.

The covalent warhead C is capable of forming a reversible or irreversible covalent bond with the protein or tissue targeted by the targeting moiety T.

The covalent warhead C is selected from the group consisting of: 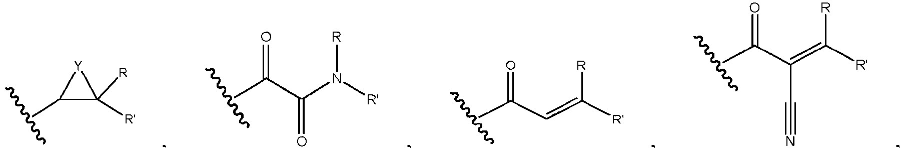 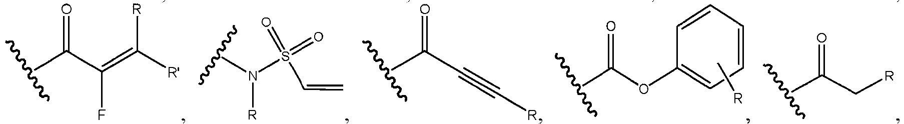 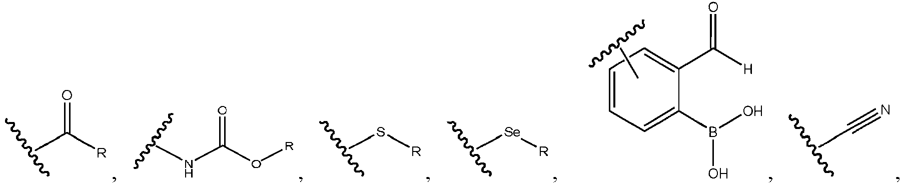 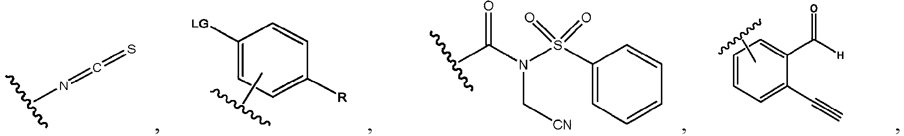 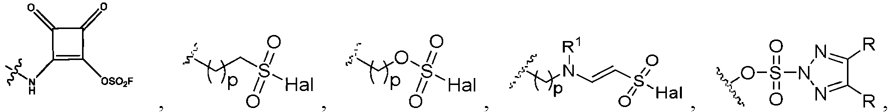 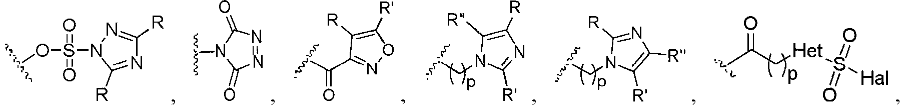 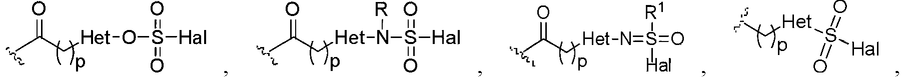 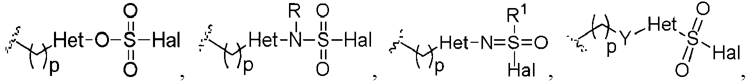 where:
Y is selected from the group consisting of O, S, and NR¹;
LG is a leaving group that can be displaced by a protein residue, and is preferably halogen or Ots, where Ts is a p-toluenesulfonyl group;
R, R' and R" are each independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
Het is an optionally substituted heterocyclic group or heteroaryl group, where when the heteroaryl group contains an N atom, the N atom may be present in a free form or an onium salt form;
Hal is halogen, preferably F or Cl;
each p is independently an integer from 0 to 12; and
the at least one targeting moiety T, at least one payload P, and at least one covalent warhead C are connected via a linker moiety.

In some embodiments of the present invention, the covalent warhead C in the trifunctional compound may also be selected from

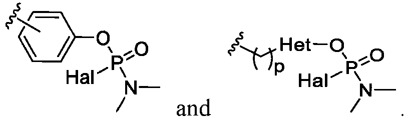

In some embodiments, Hal may also be another leaving group, for example OH or NH₂.

In a preferred embodiment of the present invention, the covalent warhead C in the trifunctional compound is selected from the group consisting of: 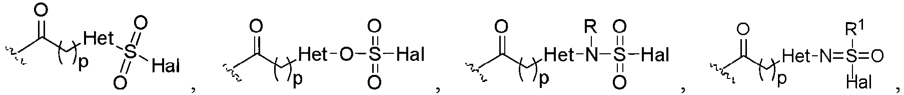 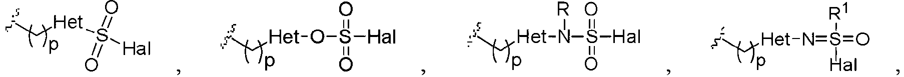 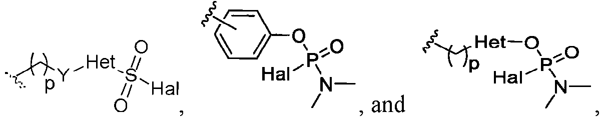
where Het is an optionally substituted C₄-C₁₂ heterocyclic group or an optionally substituted C₃-C₁₂ heteroaryl group, where when the heteroaryl group contains an N atom, the N atom may be present in a free form or an onium salt form;
Hal is F;
each p is independently an integer from 0 to 6; and
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group.

In a preferred embodiment of the present invention, C in the trifunctional compound is selected from the group consisting of: 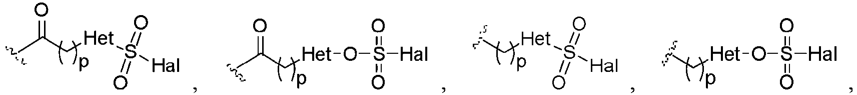 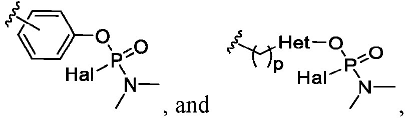 where:
Het is a C₄, C₅, or C₆ heterocyclic group or a C₅ or C₆ heteroaryl group, and the heterocyclic group or heteroaryl group contains one or two heteroatoms selected from N, O, or S, where when the heteroaryl group contains an N atom, the N atom may be present in a free form or an onium salt form, preferably in the free form; and more preferably, Het is an azetidinyl group, a pyridyl group, a pyrrolyl group, or an N-oxidopyridyl group;
Hal is F;
each p is independently an integer from 0 to 4.

In a preferred embodiment of the present invention, the trifunctional compound includes 1 to 3 (for example, 1 or 2) payloads P and 1 to 3 (for example, 1 or 2) targeting moieties T, and in addition to any covalent warhead optionally present in the targeting moiety T itself, the trifunctional compound further includes 1 to 3 (for example, 1 or 2) covalent warheads C, the linker moieties each independently includes 0 to 6 linker units, and the linker unit may be a divalent linker (for example, a single bond) or a trivalent linker.

In a preferred embodiment of the present invention, the trifunctional compound has a structure of General Formula (I), General Formula (II), or General Formula (III): 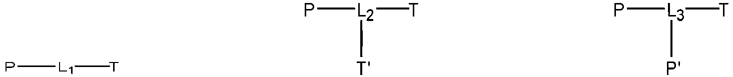 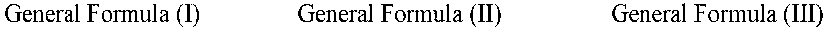 where:
payloads P and P' are each independently the same or different moieties selected from a radionuclide-containing moiety, a chelating moiety capable of chelating a radionuclide, an optical dye moiety, or a small-molecule cytotoxic drug moiety;
targeting moieties T and T' are each independently the same or different targeting moieties capable of targeting a protein or a tissue (for example, FAP proteins or other biomolecules *in vivo),* and the targeting moiety is a small molecule;
in General Formula (I), General Formula (II), and General Formula (III), in addition to any covalent warhead that may be present in the targeting moiety T itsef, the compound further comprises one, two, or three identical or different covalent warheads C, each independently connected to P, T, L₁, T', or P' in the general formulas via the same or different linker moieties L₄; and
each of L₁, L₂, and L₃ is a linker moiety and independently includes 0 to 6 linker units.

In a preferred embodiment of the present invention, the trifunctional compound has a structure of General Formula (Ia), General Formula (Ib), General Formula (Ic), General Formula (IIa), or General Formula (IIIa): 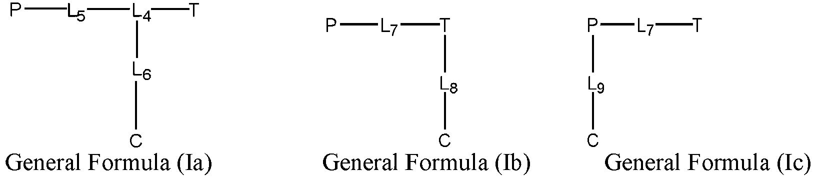 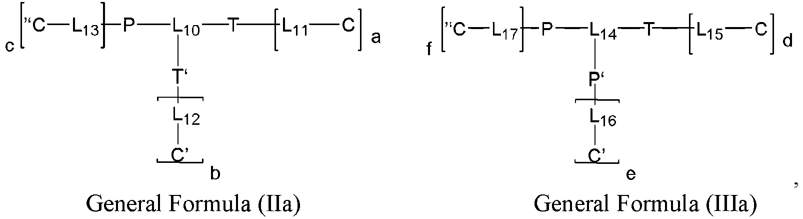 where:
L₄, L₅, L₆, L₇, L₈, L₉, L₁₀, L₁₁, L₁₂, L₁₃, L₁₄, L₁₅, L₁₆, and L₁₇ are each independently a linker moiety, and each independently contains 0 to 6 linker units;
a, b, c, d, e, and f are each independently 0 or 1, and 0 means that the group labeled by the number in the parenthesis does not exist, where the sum of a, b, and c is at least 1, and the sum of c, d, and e is at least 1; and
C, C', and C" each independently has the same meaning as defined above.

In a preferred embodiment of the present invention, the targeting moiety in the trifunctional compound targets fibroblast activation protein-α (FAP-α).

In a preferred embodiment of the present invention, the targeting moiety in the trifunctional compound has General Formula (IV), preferably General Formula (Iva), more preferably, General Formula (IVc), General Formula (IVd), General Formula (IVe), or General Formula (IVf), 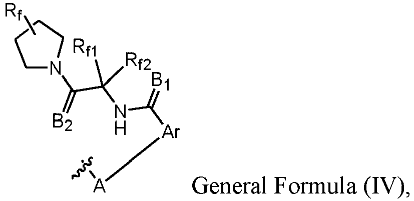
where in the General Formula (IV), A is selected from O, S, and NR_{A}, and R_{A} is selected from H and a C₁-C₆ alkyl group;
there are multiple R_{f} groups on the pyrrolidine ring, each R_{f} group is independently selected from H, F, Cl, -CN, -B(OH)₂, a C₁-C₆ alkyl group, and an α-chloroketone group, and optionally, two R_{f} groups on adjacent carbon atoms may be connected to form a cycloalkyl group, preferably a C₃-C₇ cycloalkyl group;
B₁ and B₂ are each independently selected from O and S;
R_{f1} and R_{f2} are each independently selected from H, D, and a C₁-C₄ alkyl group;
Ar is a C₆-C₁₀ heteroaryl group containing one N atom; 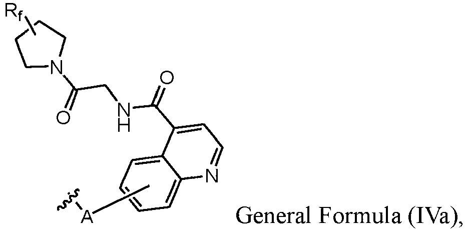
where in the General Formula (IVa), A is selected from O, S, and NR_{A}, and R_{A} is selected from H and a C₁-C₆ alkyl group; and
there are multiple R_{f} groups on the pyrrolidine ring, each R_{f} group is independently selected from H, F, Cl, -CN, a C₁-C₆ alkyl group, and an α-chloroketone group, and optionally, two R_{f} groups on adjacent carbon atoms may be connected to form a cycloalkyl group, preferably a C₃-C₇ cycloalkyl group; 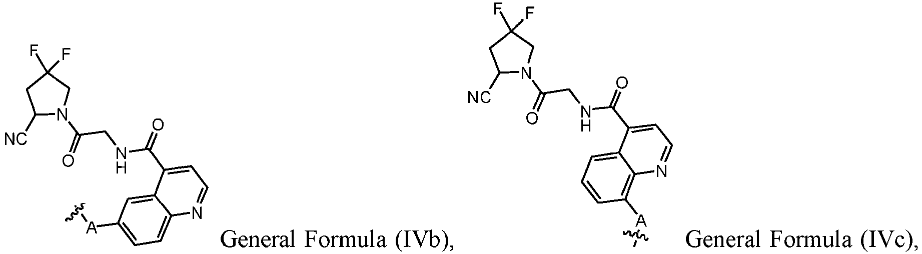 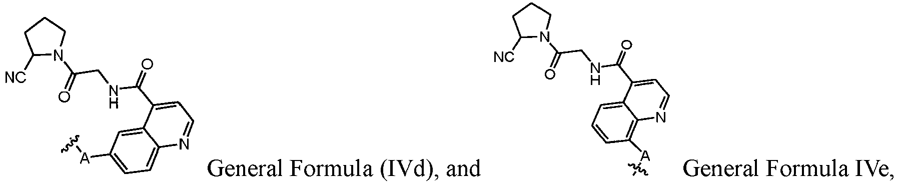
where in General Formula (IVb), (IVc), (IVd) or (IVe), A is selected from O, S, and NR_{A}, and R_{A} is selected from H, a methyl group, an ethyl group, an n-propyl group, and an isopropyl group.

In a preferred embodiment of the present invention, the targeting moiety in the trifunctional compound has General Formula (V), 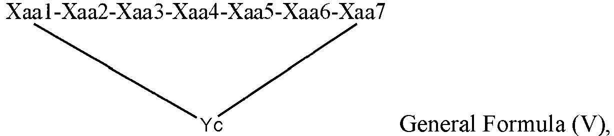
where the peptide sequence is depicted from left to right in a direction from the N-terminus to the C-terminus;
Xaa1 is a residue of an amino acid of General Formula (VI), 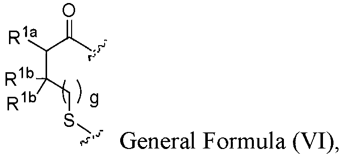
where:
   R^{1a} is -NH-R^{1a'}, R^{1a'} is a protecting group or an amino acid, and preferably, the protecting group is R^{1a}''-C(O)- or R^{1a}"-S(O₂)-, and R^{1a}" is a C₁-C₆ alkyl group, and optionally, one -CH₂- group is replaced with -S- or -O-;
   R^{1b} is H or a methyl group;
   g is 0 or 1;
   the carbonyl group of Xaa1 is covalently linked to a nitrogen atom of Xaa2;
   the sulfur atom of Xaa1 is covalently linked to Yc as a thioether;
   Xaa2 and Xaa3 are each independently selected from a residue of an amino acid of General Formula (VII) or General Formula (VIII), 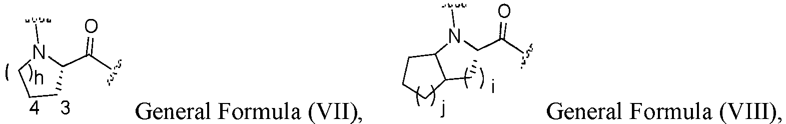
   where:
      h is 0, 1, or 2;
      i is 1 or 2;
      j is 1, 2, or 3;
      the amino acid of the General Formula (VII) may be substituted with one or two substituents selected from a methyl group, OH, NH₂, and F at the 3- and 4-positions of the ring;
      Xaa4 is a residue of an amino acid of General Formula (IX), 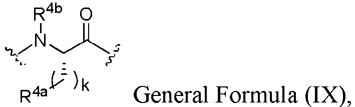
      where:
         R^{4a} is selected from H, OH, COOH, and CONH₂;
         q is 1, 2, or 3, and optionally, one or two hydrogen atoms of the 1, 2, or 3 CH₂ groups are each independently substituted by a methyl group or an ethyl group;
         R^{4b} is H or a methyl group;
         Xaa5 is a residue of an amino acid of General Formula (X), 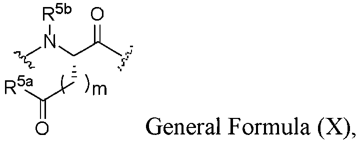
         where:
            R^{5a} is OH or NH₂;
            m is 1, 2, or 3;
            R^{5b} is H or a methyl group;
            Xaa6 is an amino acid selected from aromatic L-α-amino acids, and is preferably a residue of an amino acid of General Formula (XI), 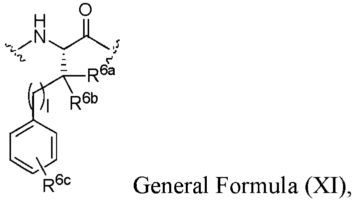
            where:
               R^{6a} and R^{6b} are each independently selected from H, a methyl group, an ethyl group, a propyl group, and an isopropyl group, preferably H;
               R^{6c} represents 0 to 3 substituents, each independently selected from F, Cl, Br, NO₂, NH₂, CN, CHF₃, OH, OR^{6d} and a C₁-C₄ alkyl group;
               R^{6d} is selected from a methyl group, an ethyl group, a propyl group, and an isopropyl group;
               l is 0 or 1, preferably 0;
               the CR^{6a}R^{6b}(CH₂)₁PhR^{6c} moiety may form a portion of the covalent warhead C in the General Formula (I), (II), or (III); and
               Xaa7 is a residue of an aminothiol or an amino acid of General Formula (XII), 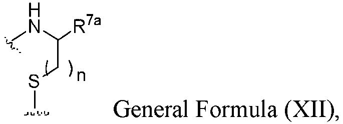
               where:
                  R^{7a} is H, -COOH, -CONH₂, or CH₂OH;
                  n is 1 or 2; and
                  Yc has a structure of General Formula (XIII), 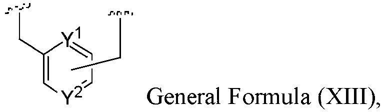
                  Yc connects the S atom of Xaa1 and the S atom of Xaa7 by forming two thioether bonds, thereby forming a cyclic structure of General Formula (XIV), 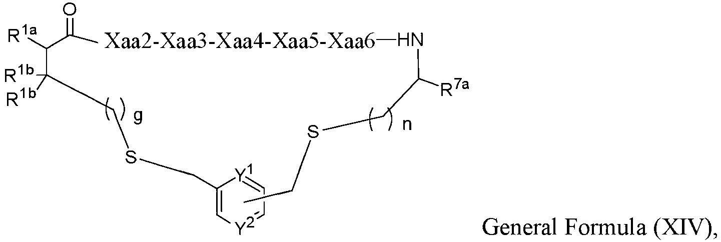
                  where a substitution pattern of the aromatic group in General Formula (XIII) is ortho, para, or meta;
                  g is 0 or 1;
                  n is 1 or 2;
                  Y¹ is CH or N;
                  Y² is CR^{L1};
                  R^{L1} is a linker unit connected to another portion of General Formula (I), and
                  the targeting moiety preferably has Formula (Va),

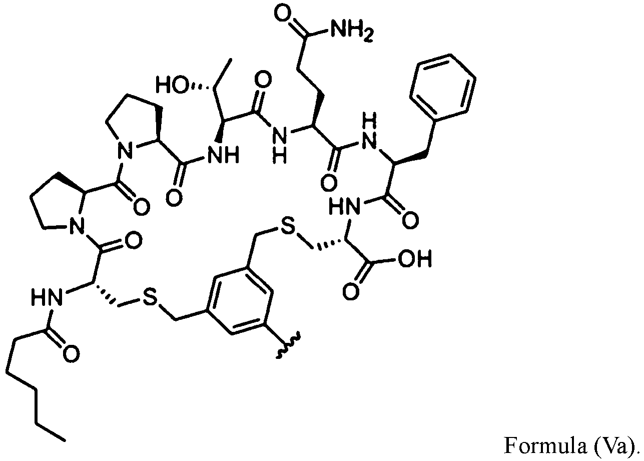

In a preferred embodiment of the present invention, the payload P in the trifunctional compound includes at least one moiety selected from the following groups of moieties:
Group 1: moieties with at least one radionuclide, selected from: 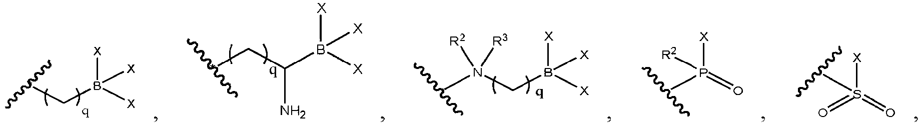 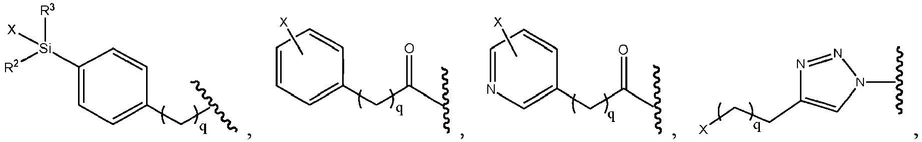 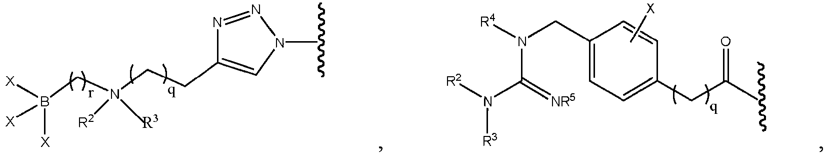 and 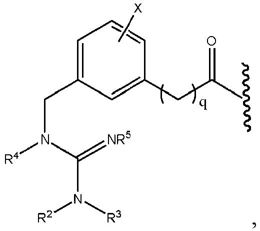
   where R², R³, R⁴, and R⁵ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
   X is selected from ¹⁸F, ¹²³I, ¹²⁴I, ¹²⁵I, ¹³¹I, and ²¹¹At, or a non-radioactive isotope thereof;
   q and r are each independently an integer from 0 to 4; and
   the N on the triazole ring at the linking site shown herein may not be present on the payload P, but on the linker unit instead;
Group 2: chelating moieties capable of chelating a radionuclide, selected from: 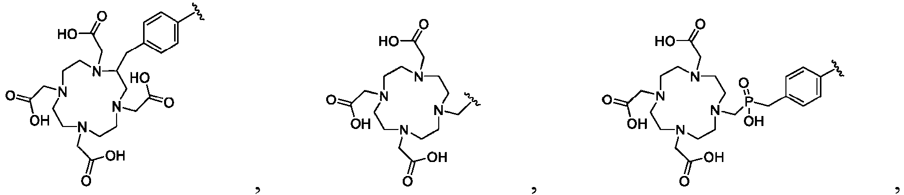 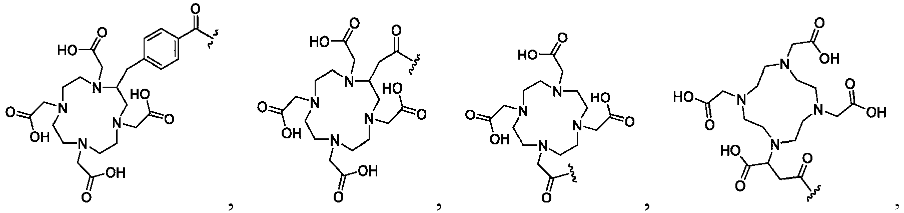 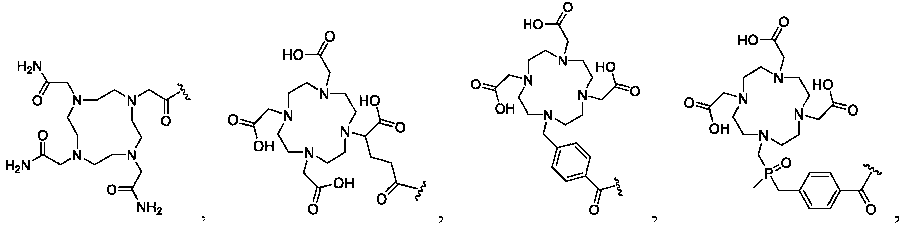 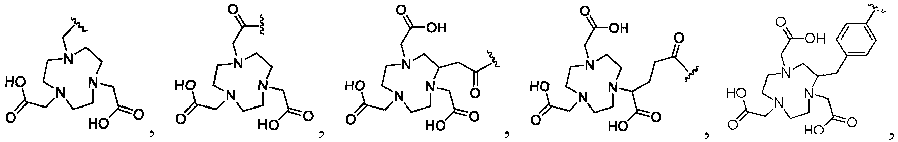 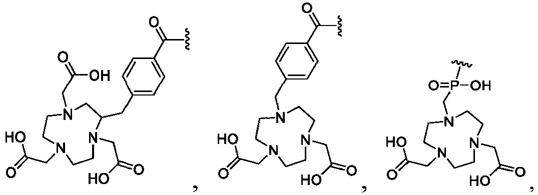 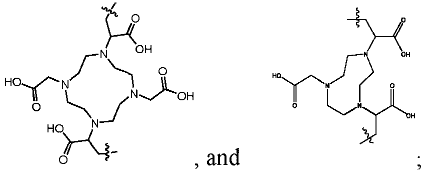
Group 3: Optical dye moieties, selected from: 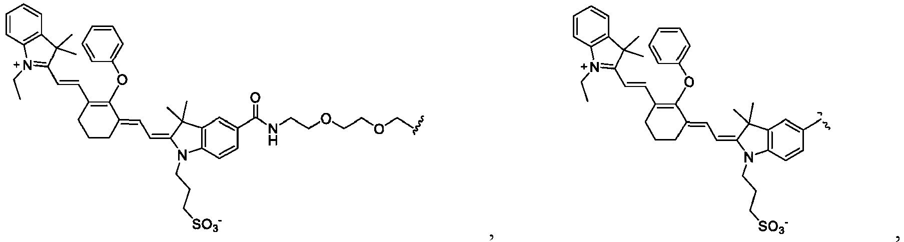 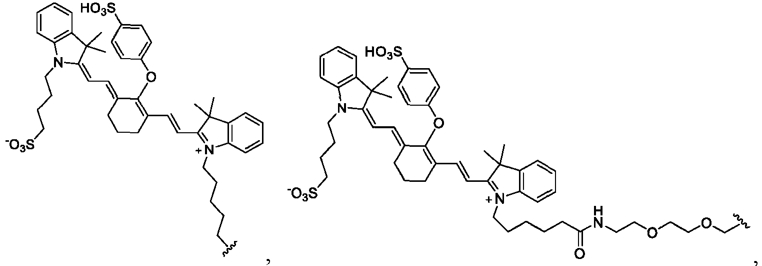 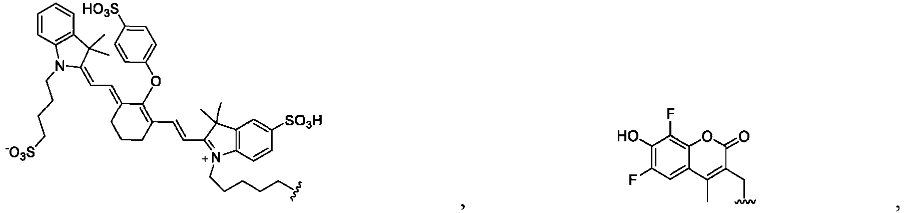 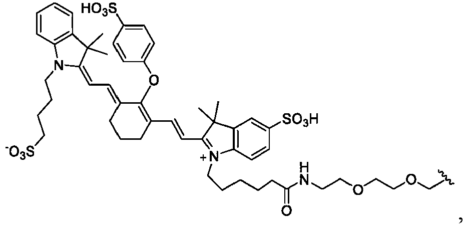 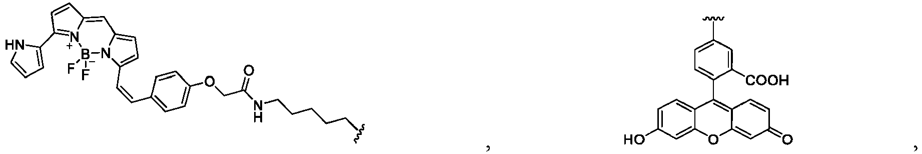 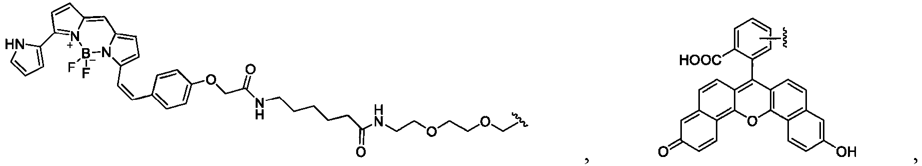 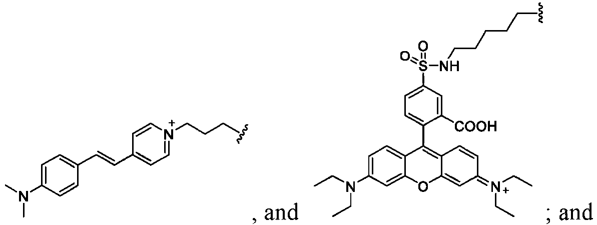
Group 4: Small-molecule cytotoxic drug moieties, selected from:

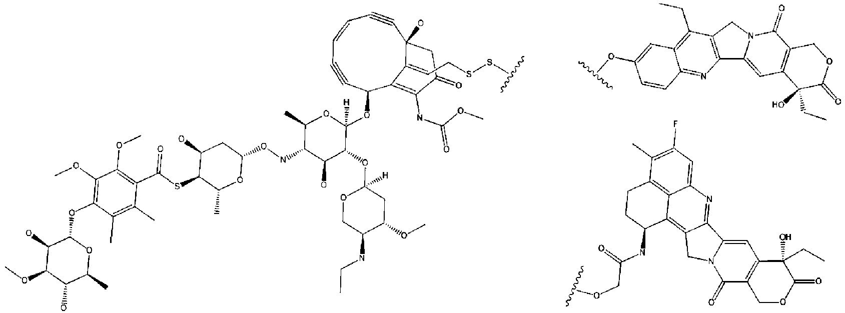

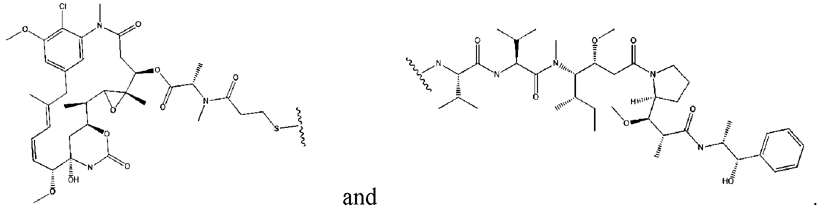

In a preferred embodiment of the present invention, in the trifunctional compound,
when L₁ is a trivalent linker, that is, when L₁ is linked to the covalent warhead C, L₄ is independently selected from: , 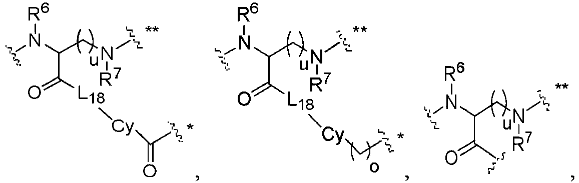 and 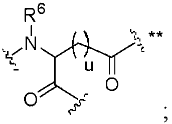
divalent linker units contained in L₁ to L₁₇ are each independently selected from: a single bond, 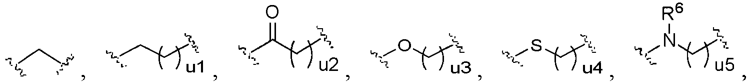 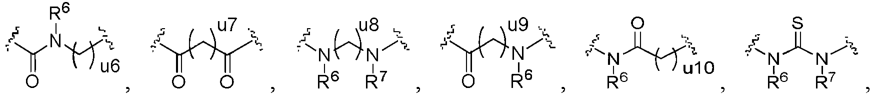 and 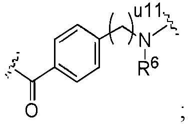
where the methylene group in the listed divalent linker units may be substituted with one or more -COR^{L2} or -COOR^{L2} groups, where each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 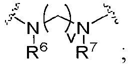
Cy is selected from 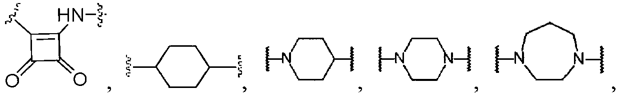 and 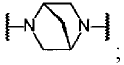
u, u1, u2, u3, u4, u5, u6, u7, u8, u9, u10, u11, v, and o are each independently 1, 2, 3, 4, or 5;
R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
*is a site for linking the targeting moiety T; and
** is a site for linking the covalent warhead C;
on the condition that in L₁ to L₁₇, the heteroatoms are not directly connected via covalent bonds, where the heteroatoms are selected from N, O, and S.

In some embodiments of the present invention, the divalent linker units contained in L₁ to L₁₇ may further be selected as 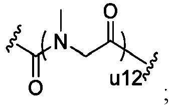 and/or L₄ may further be selected as 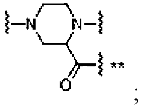 and/or u, u1, u2, u3, u4, u5, u6, u7, u8, u9, u10, u11, and u12; and v and o may each independently be 0, 1, 2, 3, 4, 5, 6, 7, 8, 9, or 10.

In a preferred embodiment of the present invention, the trifunctional compound has a structure of General Formula (Ia), and L₄ is: 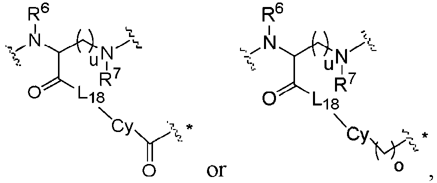
L₄ preferably has the following structure: 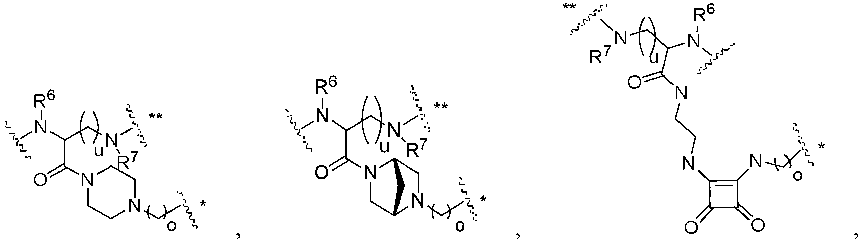 or 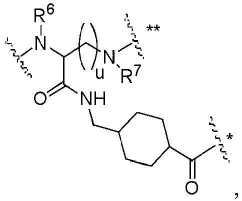
where R⁶, R⁷, L₁₈, Cy, o, and u have the same definitions as above.

In a preferred embodiment of the present invention, the trifunctional compound has a structure of General Formula (Ia), or General Formula (Ib): 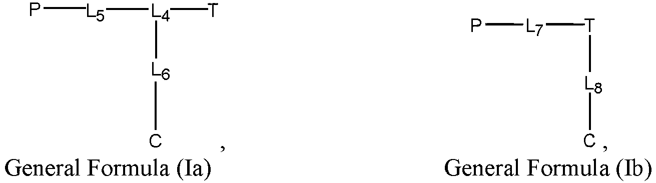 where:
L₄ is a trivalent linker moiety;
L₅, L₆, L₇, and L₈ are each independently a divalent linker moiety, and each independently contains 0 to 6 linker units, preferably 0 to 3 linker units;
C is a covalent warhead selected from the group consisting of: 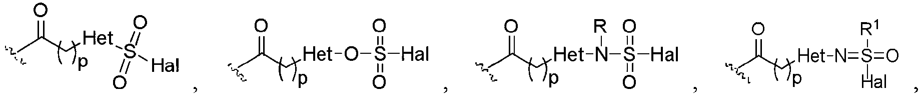 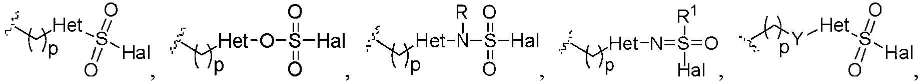 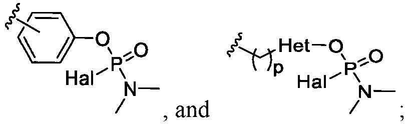
where Het is an optionally substituted C₄-C₁₂ heterocyclic group or an optionally substituted C₃-C₁₂ heteroaryl group, where when the heteroaryl group contains an N atom, the N atom may be present in a free form or an onium salt form;
Hal is For Cl;
Y is selected from the group consisting of O, S, and NR¹;
each p is independently an integer from 0 to 6;
each R¹ is independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
each R is independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
T is a targeting moiety that targets fibroblast activation protein-α (FAP-α); and
P is a chelating moiety capable of chelating a radionuclide.

In a preferred embodiment of the present invention, the trifunctional compound has a structure of General Formula (Ia) or General Formula (Ib),
where:
L₄ is a trivalent linker selected from: 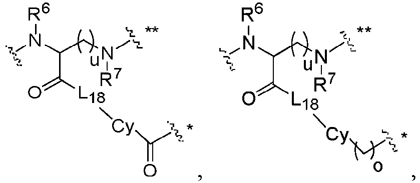 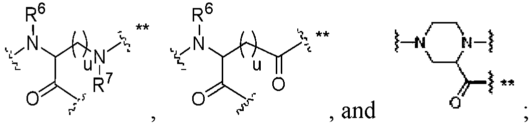
L₅, L₆, L₇, and L₈ are each independently a divalent linker moiety, and each independently contains 0 to 3 linker units, preferably 0 or 1 linker unit, and the linker unit is each independently selected from: a single bond, 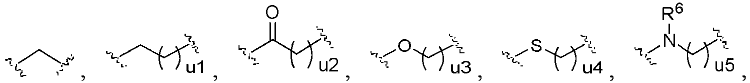 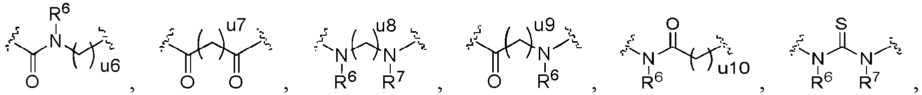 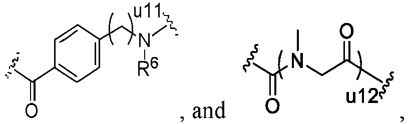
where the methylene group in the listed divalent linker units may be substituted with one or more -COR^{L2} or -COOR^{L2} groups, where each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 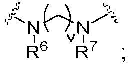
Cy is selected from 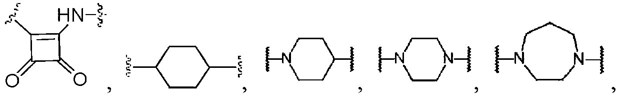 and 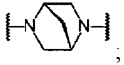
u, u1, u2, u3, u4, u5, u6, u7, u8, u9, u10, u11, u12, v, and o are each independently 0, 1, 2, 3, 4, 5, 6, 7, 8, 9, or 10; preferably, 1, 2, 3, 4, or 5;
R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
*is a site for linking T;
** is a site for linking C;
on the condition that in L₅, L₆, L₇, and L₈, the heteroatoms are not directly connected via covalent bonds, where the heteroatoms are selected from N, O, and S;
C is selected from the group consisting of: 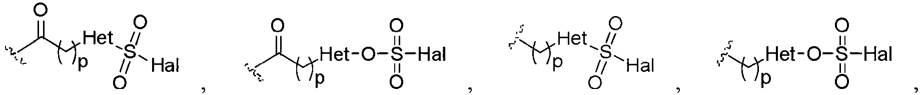 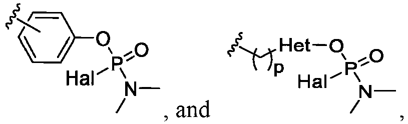
where:
   Het is a C_{4,} C₅, or C₆ heterocyclic group or a C₅ or C₆ heteroaryl group, and the heterocyclic group or heteroaryl group contains one or two heteroatoms selected from N, O, or S, where when the heteroaryl group contains an N atom, the N atom may be present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
   Hal is F;
   each p is independently an integer from 0 to 4;
   T is a targeting moiety that targets fibroblast activation protein-α (FAP-α); and
   P is a chelating moiety capable of chelating a radionuclide, selected from the following:

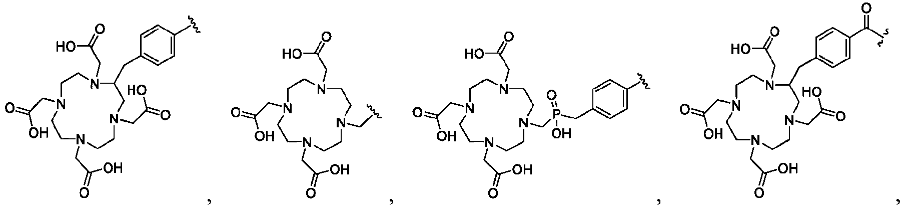

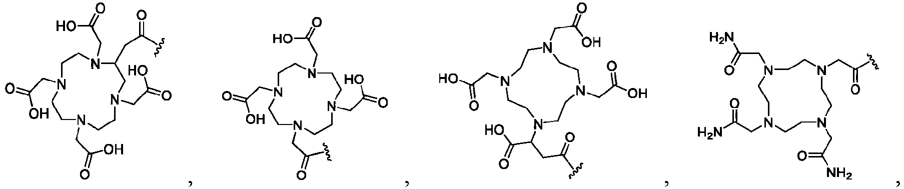

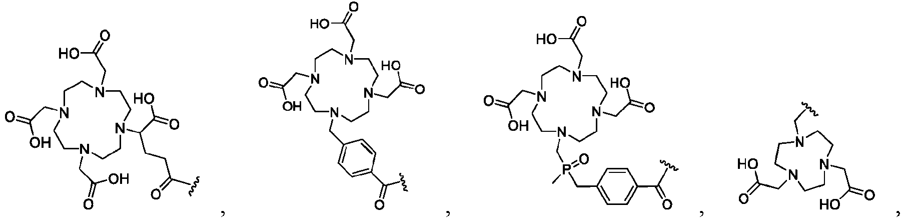

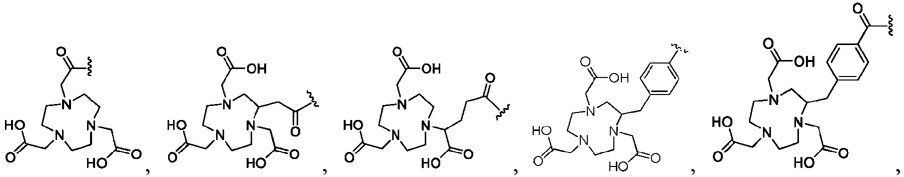

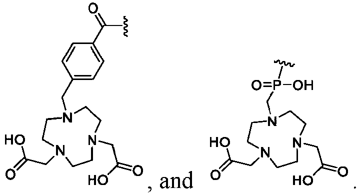

In a preferred embodiment of the present invention, the trifunctional compound has a structure of General Formula (Ia):
where:
C is selected from the group consisting of: 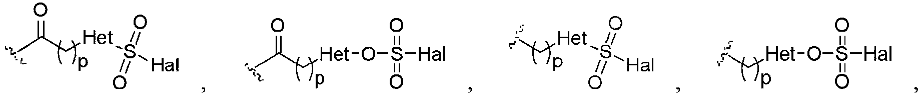 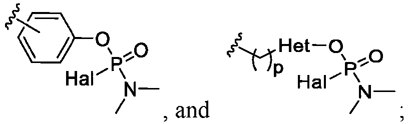
where:
   Het is a C₄, C₅, or C₆ heterocyclic group or a C₅ or C₆ heteroaryl group, and the heterocyclic group or heteroaryl group contains one or two heteroatoms selected from N, O, or S, where when the heteroaryl group contains an N atom, the N atom may be present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
   Hal is F;
   each p is independently an integer from 0 to 4; and
   P is a chelating moiety capable of chelating a radionuclide, selected from: 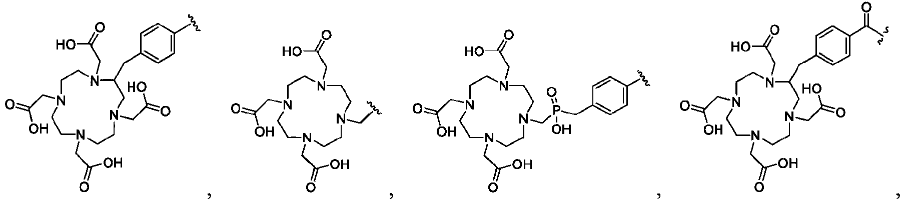 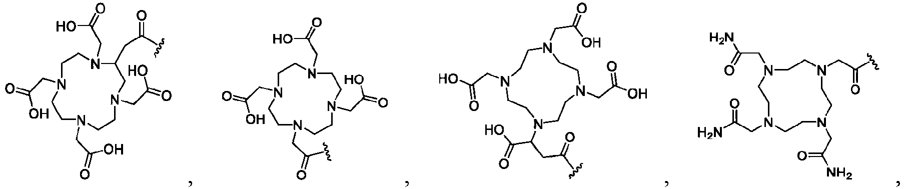 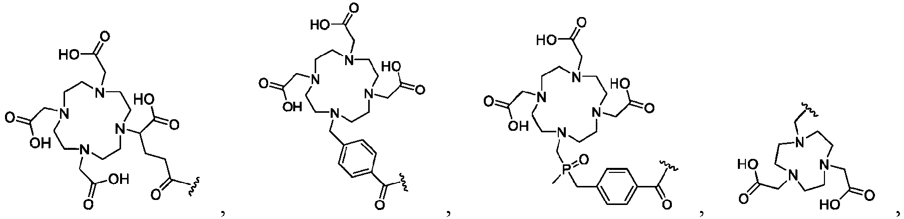 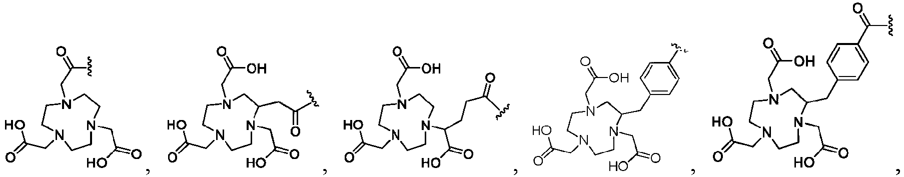 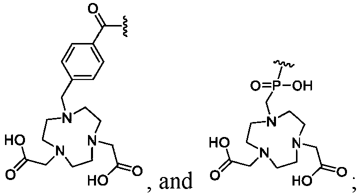
   L₄ has the following structure: 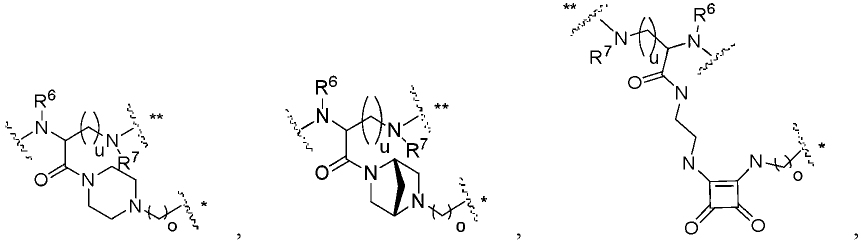 or 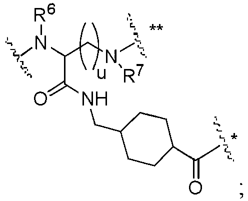
   L₅ and L₆ are each independently: a single bond, 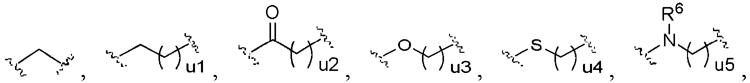 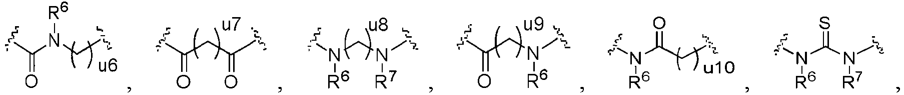 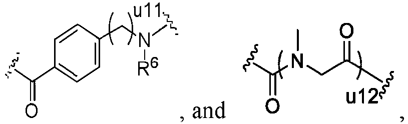
   where u, u1, u2, u3, u4, u5, u6, u7, u8, u9, u10, u11, u12, v, and o are each independently 0, 1, 2, 3, 4, 5, 6, 7, 8, 9, or 10; preferably, 1, 2, 3, 4, or 5;
   R⁶ and R⁷ are each independently selected from hydrogen and an optionally substituted C₁-C₄ group;
   * is a site for linking T;
   ** is a site for linking C;
   on the condition that in L₅ and L₆, the heteroatoms are not directly connected via covalent bonds, where the heteroatoms are selected from N, O, and S;
   T is represented by General Formula (IV), preferably General Formula (IVa), more preferably, General Formula (IVc), General Formula (IVd), General Formula (IVe), or General Formula (IVf), 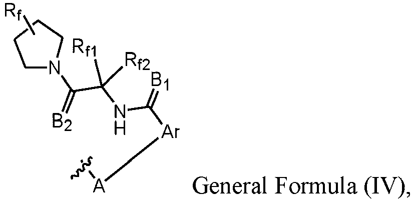
   where in the General Formula (IV), A is selected from O, S, and NR_{A}, and R_{A} is selected from H and a C₁-C₆ alkyl group;
   there are multiple R_{f} groups on the pyrrolidine ring, each R_{f} group is independently selected from H, F, Cl, -CN, -B(OH)₂, and a C₁-C₆ alkyl group, and optionally, two R_{f} groups on adjacent carbon atoms may be connected to form a cycloalkyl group, preferably a C₃-C₇ cycloalkyl group;
   B₁ and B₂ are each independently selected from O and S;
   R_{f1} and R_{f2} are each independently selected from H, D, and a C₁-C₄ alkyl group;
   Ar is a C₆-C₁₀ heteroaryl group containing one N atom; 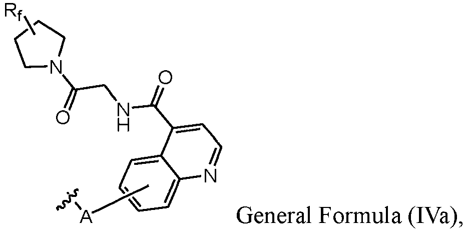
   where in the General Formula (IVa), A is selected from O, S, and NR_{A}, and R_{A} is selected from H and a C₁-C₆ alkyl group; and
   there are multiple R_{f} groups on the pyrrolidine ring, each R_{f} group is independently selected from H, F, Cl, -CN, and a C₁-C₆ alkyl group, and optionally, two R_{f} groups on adjacent carbon atoms may be connected to form a cycloalkyl group, preferably a C₃-C₇ cycloalkyl group; 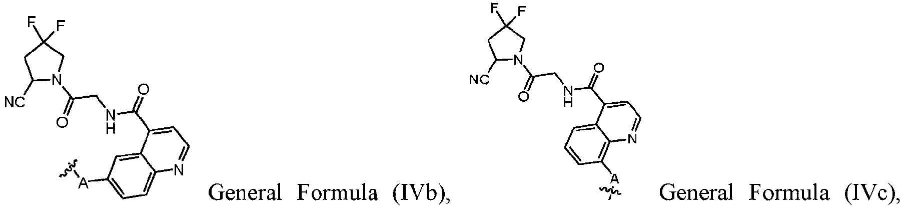 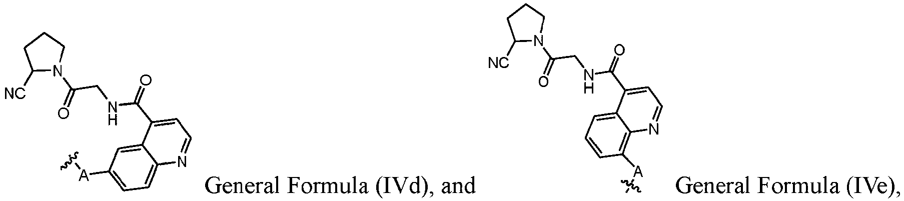
   where in the General Formulas (IVb), (IVc), (IVd) or (IVe), A is selected from O, S, and NR_{A}, and R_{A} is selected from H, a methyl group, an ethyl group, an n-propyl group, and an isopropyl group.
   In a preferred embodiment of the present invention, the trifunctional compound has a structure of General Formula (Ib),
   where:
      C in General Formula (Ib) is selected from the group consisting of: 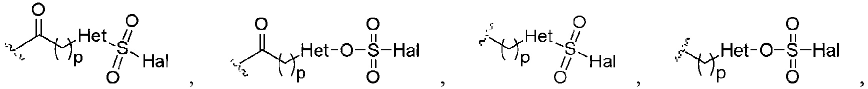 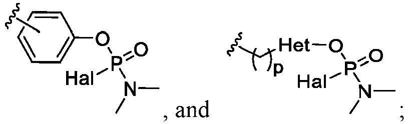
      where:
         Het is a C₄, C₅, or C₆ heterocyclic group or a C₅ or C₆ heteroaryl group, and the heterocyclic group or heteroaryl group contains one or two heteroatoms selected from N, O, or S, where when the heteroaryl group contains an N atom, the N atom may be present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
         Hal is F;
         each p is independently an integer from 0 to 4; and
         P is a chelating moiety capable of chelating a radionuclide, selected from: 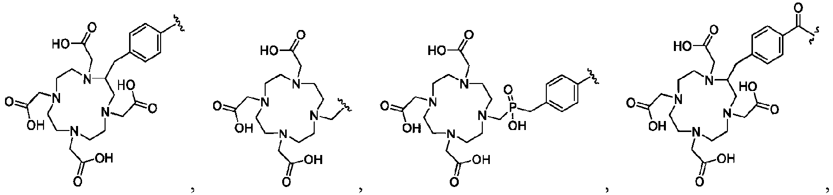 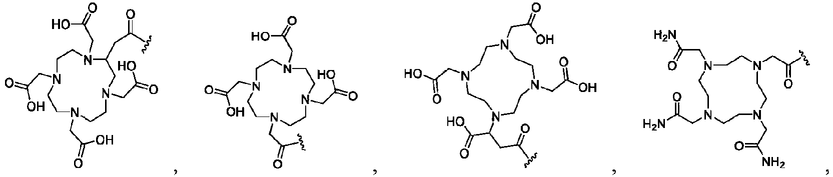 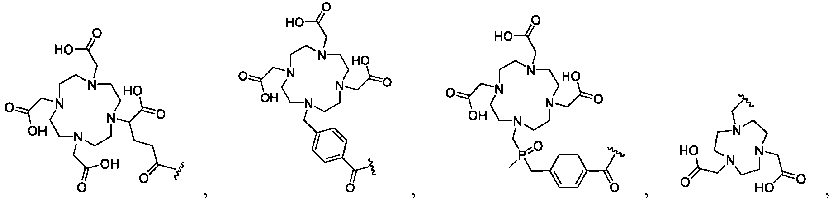 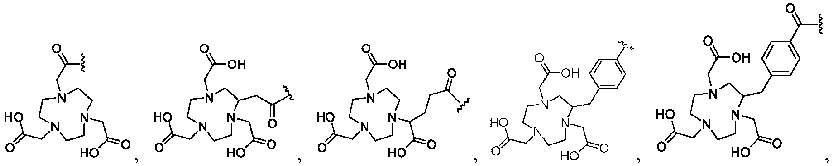 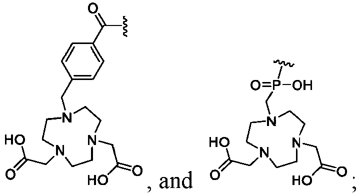
         L₇ and L₈ are each independently: a single bond, 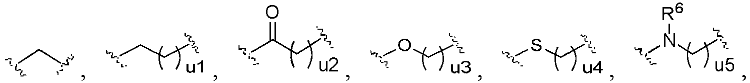 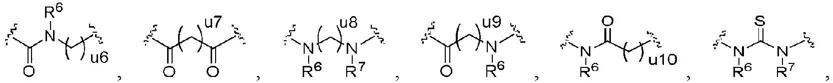 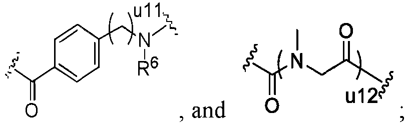
         where u, u1, u2, u3, u4, u5, u6, u7, u8, u9, u10, u11, u12, v, and o are each independently 0, 1, 2, 3, 4, 5, 6, 7, 8, 9, or 10; preferably, 1, 2, 3, 4, or 5;
         R⁶ and R⁷ are each independently selected from hydrogen and an optionally substituted C₁-C₄ group;
         on the condition that in L₇ and L₈, the heteroatoms are not directly connected via covalent bonds, where the heteroatoms are selected from N, O, and S;
         T has General Formula (V), 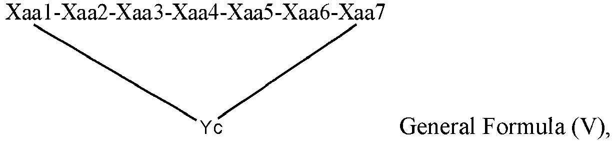
         where the peptide sequence is depicted from left to right in a direction from the N-terminus to the C-terminus; and
         Xaa1 is a residue of an amino acid of General Formula (VI), 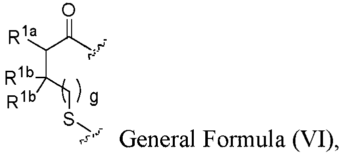
         where:
            R^{1a} is -NH-R^{1a'}, R^{1a'} is a protecting group or an amino acid, and preferably, the protecting group is R^{1a}"-C(O)- or R^{1a}"-S(O₂)-, and R^{1a}" is a C₁-C₆ alkyl group, and optionally, one -CH₂- group is replaced with -S- or -O-;
            R^{1b} is H or a methyl group;
            g is 0 or 1;
            the carbonyl group of Xaa1 is covalently linked to a nitrogen atom of Xaa2;
            the sulfur atom of Xaa1 is covalently linked to Yc as a thioether;
            Xaa2 and Xaa3 are each independently selected from a residue of an amino acid of General Formula (VII) or General Formula (VIII), 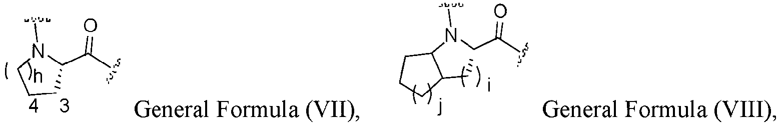
            where:
               h is 0, 1, or 2;
               i is 1 or 2;
               j is 1, 2, or 3;
               the amino acid of the General Formula (VII) may be substituted with one or two substituents selected from a methyl group, OH, NH₂, and F, at the 3- and 4-positions of the ring; and
               Xaa4 is a residue of an amino acid of General Formula (IX), 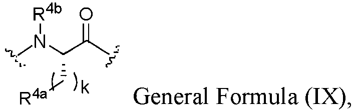
               where:
                  R^{4a} is selected from H, OH, COOH, and CONH₂;
                  q is 1, 2, or 3, and optioanlly, one or two hydrogen atoms of the 1, 2, or 3 CH₂ groups are each independently substituted by a methyl group or an ethyl group;
                  R^{4b} is H or a methyl group; and
                  Xaa5 is a residue of an amino acid of General Formula (X), 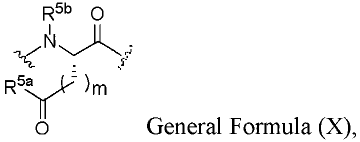
                  where:
                     R^{5a} is OH or NH₂;
                     m is 1, 2, or 3;
                     R^{5b} is H or a methyl group; and
                     Xaa6 is an amino acid selected from aromatic L-α-amino acids, and is preferably a residue of an amino acid of General Formula (XI), 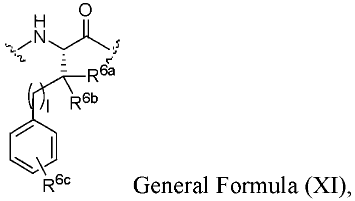
                     where:
                        R^{6a} and R^{6b} are each independently selected from H, a methyl group, an ethyl group, a propyl group, and an isopropyl group, preferably H;
                        R^{6c} represents 0 to 3 substituents, each independently selected from F, Cl, Br, NO₂, NH₂, CN, CHF₃, OH, OR^{6d} and a C₁-C₄ alkyl group;
                        R^{6d} is selected from a methyl group, an ethyl group, a propyl group, and an isopropyl group;
                        l is 0 or 1, preferably 0;
                        the CR^{6a}R^{6b}(CH₂)₁PhR^{6c} moiety may form a portion of C in the General Formula (Ib); and
                        Xaa7 is a residue of an aminothiol or an amino acid of General Formula (XII), 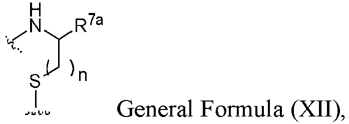
                        where:
                           R^{7a} is H, -COOH, -CONH₂, or CH₂OH;
                           n is 1 or 2; and
                           Yc has a structure of General Formula (XIII), 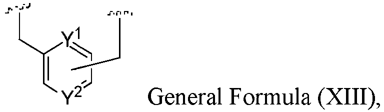
                           Yc connects the S atom of Xaa1 and the S atom of Xaa7 by forming two thioether bonds, to form a cyclic structure of General Formula (XIV), 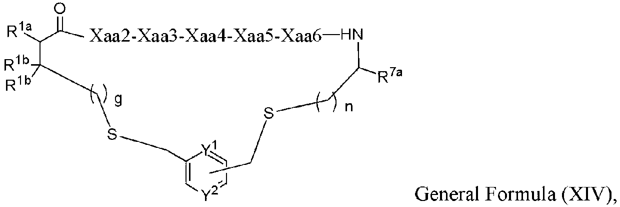
                           where a substitution pattern of the aromatic group in General Formula (XIII) is ortho, para, or meta;
                           g is 0 or 1;
                           n is 1 or 2;
                           Y¹ is CH or N;
                           Y² is CR^{L1};
                           R^{L1} is a linker unit connected to another portion of General Formula (Ib).

In a preferred embodiment of the present invention, the trifunctional compound has a structure of General Formula (Ib):
where:
L₇ and L₈ are each independently: a single bond, 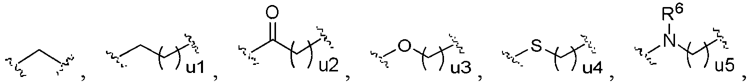 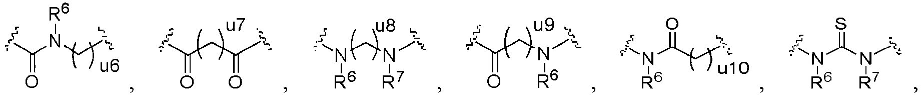 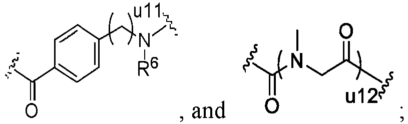
where u, u1, u2, u3, u4, u5, u6, u7, u8, u9, u10, u11, u12, v, and o are each independently 0, 1, 2, 3, 4, 5, 6, 7, 8, 9, or 10; preferably, 1, 2, 3, 4, or 5;
R⁶ and R⁷ are each independently selected from hydrogen and an optionally substituted C₁-C₄ group;
on the condition that in L₇ and L₈, the heteroatoms are not directly connected via covalent bonds, where the heteroatoms are selected from N, O, and S;
C is selected from the group consisting of: 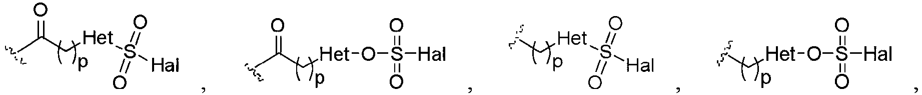 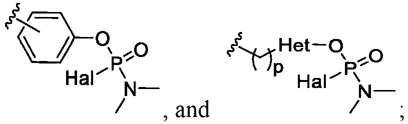
where:
   Het is a C₄, C₅, or C₆ heterocyclic group or a C₅ or C₆ heteroaryl group, and the heterocyclic group or heteroaryl group contains one or two heteroatoms selected from N, O, or S, where when the heteroaryl group contains an N atom, the N atom may be present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
   Hal is F;
   each p is independently an integer from 0 to 4; and
   P is a chelating moiety capable of chelating a radionuclide, selected from: 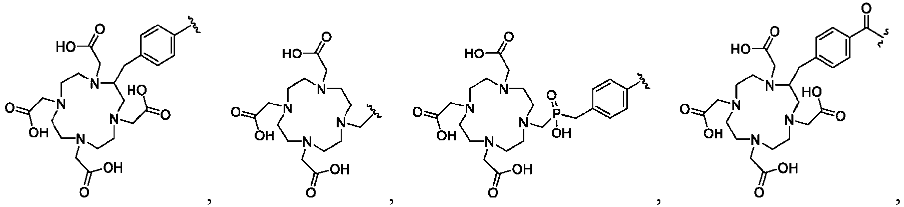 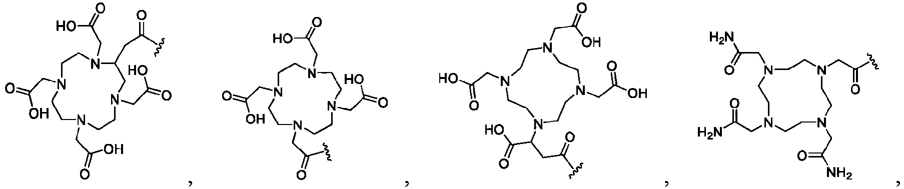 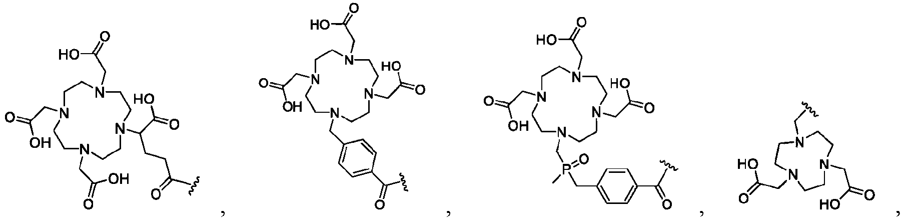 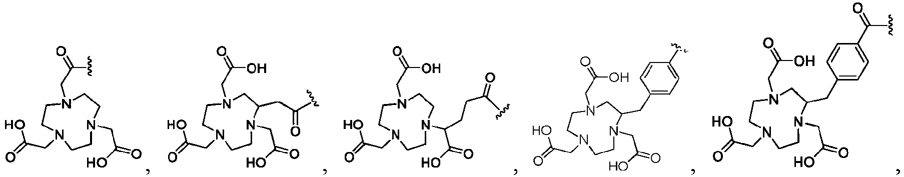 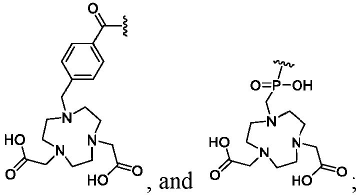
   T has General Formula (V), 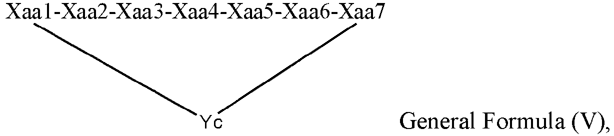
   where the peptide sequence is depicted from left to right in a direction from the N-terminus to the C-terminus; and
   Xaa1 is a residue of an amino acid of General Formula (VI), 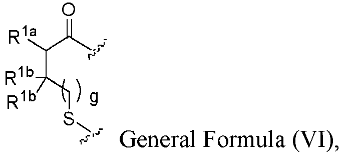
   where:
      R^{1a} is -NH-R^{1a'}, R^{1a'} is a protecting group or an amino acid, and preferably, the protecting group is R^{1a}"-C(O)- or R^{1a}"-S(O₂)-, and R^{1a}" is a C₁-C₆ alkyl group, and optioanlly, one -CH₂- group is replaced with -S- or -O-;
      R^{1b} is H or a methyl group;
      g is 0 or 1;
      the carbonyl group of Xaa1 is covalently linked to a nitrogen atom of Xaa2;
      the sulfur atom of Xaa1 is covalently linked to Yc as a thioether; and
      Xaa2 and Xaa3 are each independently selected from residues of amino acids of General Formula (VII) or General Formula (VIII), 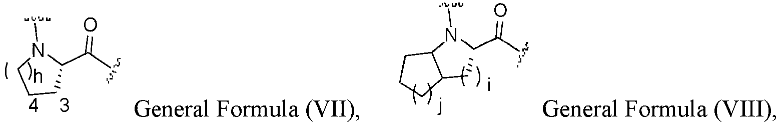
      where:
         h is 0, 1, or 2;
         i is 1 or 2;
         j is 1, 2, or 3;
         the amino acid of the General Formula (VII) may be substituted with one or two substituents selected from a methyl group, OH, NH₂, and F at the 3- and 4-positions of the ring; and
         Xaa4 is a residue of an amino acid of General Formula (IX), 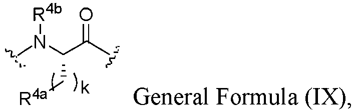
         where:
            R^{4a} is selected from H, OH, COOH, and CONH₂;
            q is 1, 2, or 3, and optioanlly, one or two hydrogen atoms of the 1, 2, or 3 CH₂ groups are each independently substituted by a methyl group or an ethyl group;
            R^{4b} is H or a methyl group; and
            Xaa5 is a residue of an amino acid of General Formula (X), 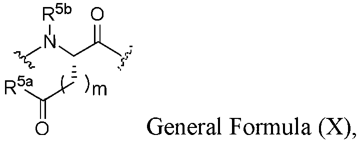
            where:
               R^{5a} is OH or NH₂;
               m is 1, 2, or 3;
               R^{5b} is H or a methyl group; and
               Xaa6 is a residue of an amino acid of General Formula (XI), 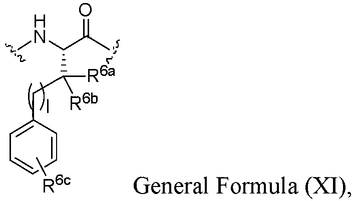
               where:
                  R^{6a} and R^{6b} are each independently selected from H, a methyl group, an ethyl group, a propyl group, and an isopropyl group, preferably H;
                  R^{6c} represents 0 or 1 substituent, each independently selected from F, Cl, NO₂, NH₂, CN, CHF₃, OH, and a C₁-C₄ alkyl group;
                  R^{6d} is selected from a methyl group, an ethyl group, a propyl group, and an isopropyl group;
                  1 is 0 or 1, preferably 0;
                  CR^{6a}R^{6b}(CH₂)₁PhR^{6c} moiety may form a portion of C in the General Formula (Ib); and
                  Xaa7 is a residue of an aminothiol or an amino acid of General Formula (XII), 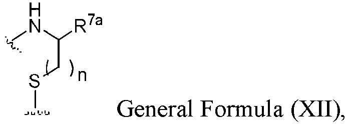
                  where:
                     R^{7a} is H or -COOH;
                     n is 1 or 2; and
                     Yc has a structure of General Formula (XIII), 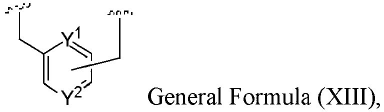
                     Yc connects the S atom of Xaa1 and the S atom of Xaa7 by forming two thioether bonds, to form a cyclic structure of General Formula (XIV), 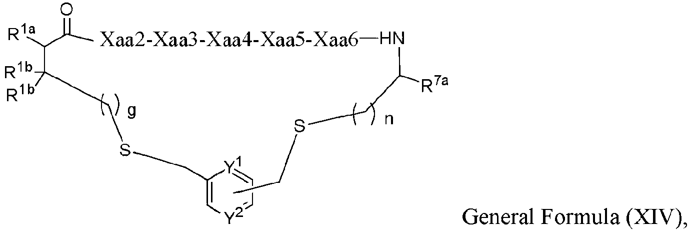
                     where a substitution pattern of the aromatic group in General Formula (XIII) is ortho, para, or meta;
                     g is 0 or 1;
                     n is 1 or 2;
                     Y¹ is CH or N;
                     Y² is CR^{L1};
                     R^{L1} is a linker unit connected to another portion of General Formula (Ib); and
                     T preferably has Formula (Va):

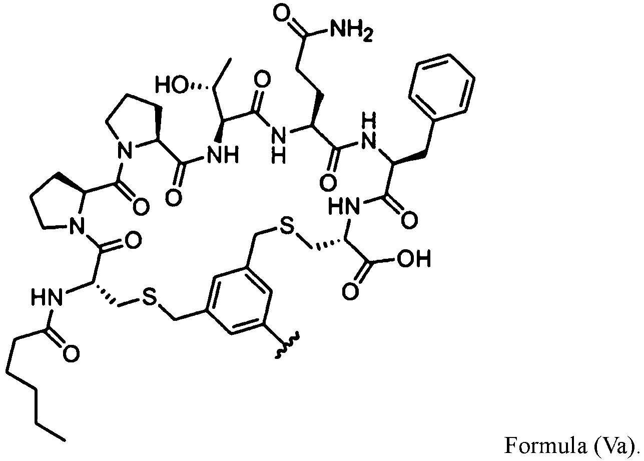

In a preferred embodiment of the present invention, the trifunctional compound has one of the following structures: 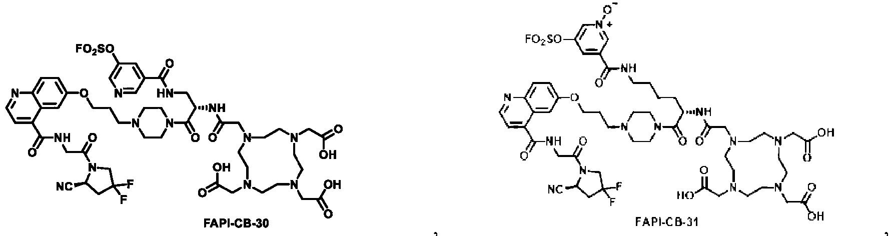 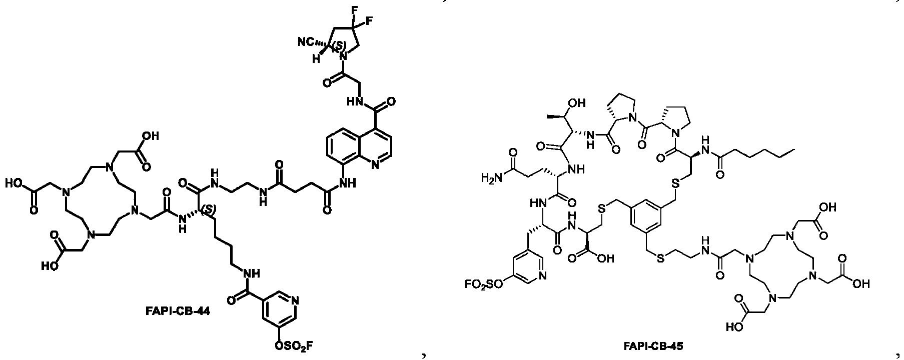 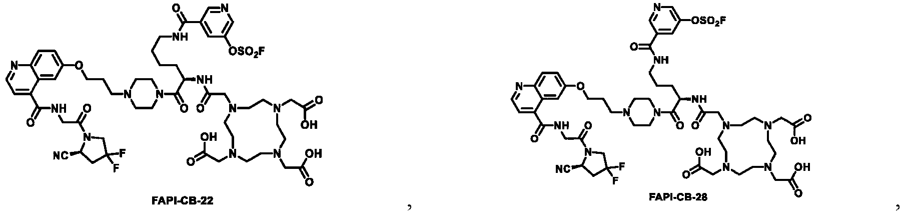 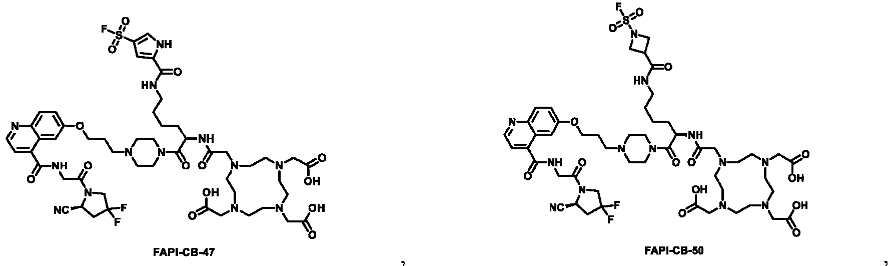 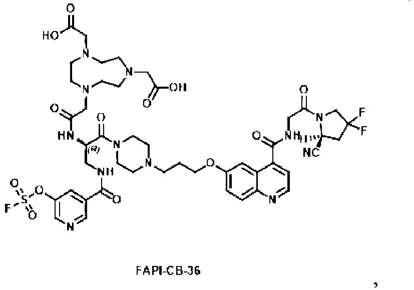 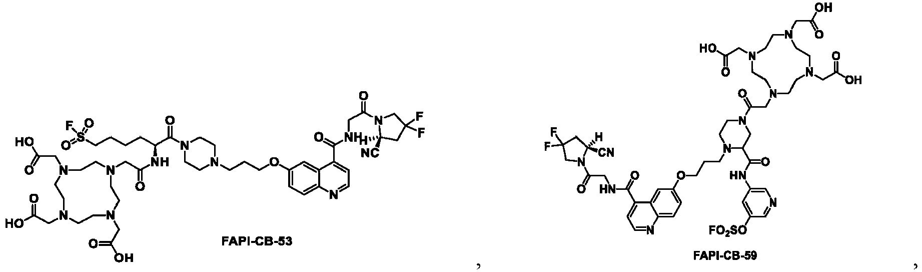 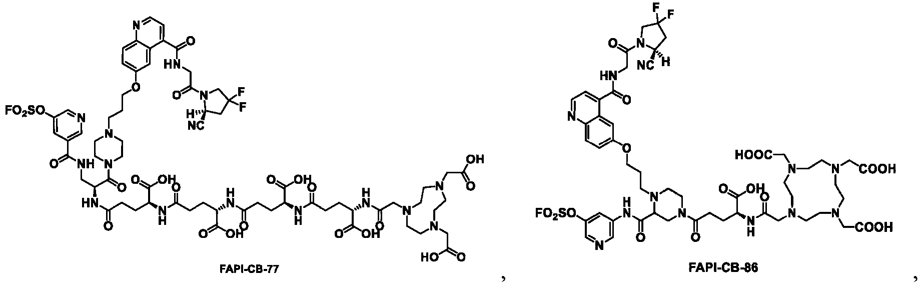 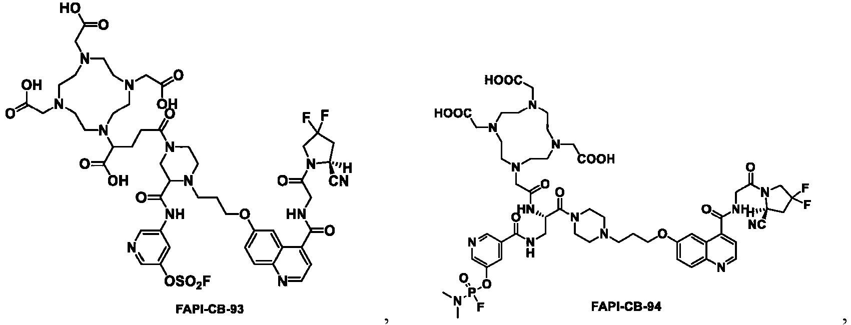 and

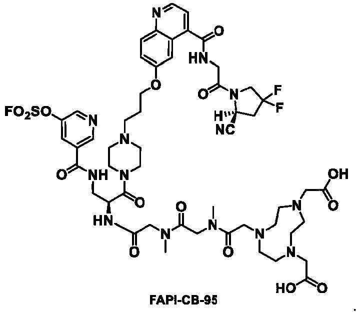

Another aspect of the present disclosure relates to the foregoing trifunctional compound, or a pharmaceutically acceptable salt, stereoisomer or solvate thereof, where the payload P is a chelating moiety capable of chelating a radionuclide, which chelates the radionuclides.

In a preferred embodiment of this aspect, the radionuclide carried or chelated by the trifunctional compound is a positron nuclide, β emitter, α emitter, an Auger electron-emitting isotope, an X-ray-emitting isotope, a fluorescence-emitting isotope, or a stable metal/non-metal element coordinated with a radionuclide, which is preferably selected from ¹¹C, ¹³N, ¹⁵O, ¹⁸F and its coordinating element, ⁴⁷Sc, ⁵¹Cr, ⁶⁷Ga, ⁶⁸Ga, ⁸⁶Y, ⁹⁰Y, ⁶⁴Cu, ⁶⁷Cu, ⁷²As, ⁷²Se, ⁸⁹Zr, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁴²Pr, ¹⁵¹Eu, ¹⁵³Eu, ¹⁶⁹Eu, ¹⁵⁹Gd, ¹⁶¹Tb, ¹⁷⁷Lu, ¹⁹⁸Au, ¹⁹⁹Ag, ²⁰¹Tl, ²¹¹At, ²⁰³Pb, ²¹²Pb, ²¹²Bi, ²¹³Bi, ²²⁵Ac, ^{99m}Tc, ¹¹¹In, ¹⁴⁹Pm, ¹⁵³Sm, ¹⁶⁵Dy, ¹⁶⁹Er, ¹⁸⁶Re, ¹⁸⁸Re, ¹⁹⁷Hg, ²²⁷Th, ⁶⁷Ga, ⁶⁸Ga, ⁸⁶Y, ⁹⁰Y, ⁵⁵Co, ¹³⁹La, ¹⁴⁰La, ¹⁴⁹Tb, ¹⁵²Tb, ¹⁵⁵Tb, ¹⁶⁶Ho, ¹⁷⁵Yb, ²²⁶Th, ²²³Ra, and ²³⁰U.

A still another aspect of the present disclosure relates to a pharmaceutical composition, including the foregoing trifunctional compound, or the pharmaceutically acceptable salt, stereoisomer or solvate thereof, and a pharmaceutically acceptable carrier.

A yet another aspect of the present disclosure relates to a kit, including or consisting of the trifunctional compound or the pharmaceutically acceptable salt, stereoisomer or solvate thereof, or the foregoing pharmaceutical composition, and an instruction for diagnosing a disease.

A still yet another aspect of the present disclosure relates to a method for diagnosing or treating a disease, which is preferably a disease involving FAP, where the method includes administering, to a subject, a therapeutically effective dose of the foregoing trifunctional compound, or the pharmaceutically acceptable salt, stereoisomer or solvate thereof, where the disease is preferably a central nervous system disease, a metabolic disease, preferably a cardiometabolic disease, or cancer.

In a preferred embodiment of this aspect, in the method for diagnosing or treating a disease, which is preferably a disease involving FAP, the cancer is selected from prostate cancer, breast cancer, pancreatic cancer, liver cancer, lung cancer, gastric cancer, renal cancer, ovarian cancer, bladder cancer, esophageal cancer, head and neck cancer, thymic cancer, cervical cancer, endometrial cancer, neuroendocrine tumor, thyroid cancer, intestinal cancer, glioma, and bone metastatic cancer.

### BRIEF DESCRIPTION OF THE DRAWINGS

FIG. 1 shows the common mechanism of action of covalent inhibitors.
FIG. 2 shows the radio-purity chromatogram of ⁶⁸Ga-FAPI-CB-30, in which the left panel represents the quality control chromatogram after labeling, the middle panel shows the stability monitoring after 1 hour of co-incubation with serum, and the right panel shows the stability monitoring after 2 hours of co-incubation with serum.
FIG. 3 shows the radioactive HPLC chromatograms of ⁶⁸Ga-FAPI-CB-31, ⁶⁸Ga-FAPI-CB-50, and Al¹⁸F-FAPI-CB-36, respectively, in the left, middle, and right panels.
FIG. 4 shows the covalent binding efficiency between ¹⁷⁷Lu-FAPI-CB-30 and the FAP target protein obtained after protein co-incubation, gel electrophoresis, autoradiography, and Coomassie blue staining. The left panel shows the control group incubated at pH 7.4 for 1 hour, and the right panel shows the sample treated with strong acid after 1 hour of incubation.
FIG. 5 shows the covalent binding ratio between ¹⁷⁷Lu-FAPI-CB-30 molecules and human recombinant FAP protein obtained after protein co-incubation, gel electrophoresis, autoradiography, and Coomassie blue staining. The left panel is the autoradiogram, and the right panel shows the quantified band gray value obtained using image analysis tools such as ImageJ, corresponding to the proportion of covalently bound molecules.
FIG. 6 shows the covalent binding ratios between molecules of ¹⁷⁷Lu-FAPI-CB series and human recombinant FAP protein obtained after protein co-incubation, gel electrophoresis, autoradiography, and Coomassie blue staining, where the lanes in the figure from left to right are ¹⁷⁷Lu-labeled FAPI-CB-22, FAPI-CB-28, FAPI-CB-31, FAPI-CB-44, FAPI-CB-45, FAPI-CB-47, and FAPI-CB-50, respectively.
FIG.7 shows comparative PET/CT imaging at 90 minutes post-injection of ⁶⁸Ga-FAPI-CB-30, ⁶⁸Ga-FAPI-CB-02, and ⁶⁸Ga-FAPI-04 into the same HT-1080-FAP mouse model. The left panel shows the structure of FAPI-CB-02; the middle panel shows PET-CT images of ⁶⁸Ga-FAPI-CB-30 and ⁶⁸Ga-FAPI-CB-02, where SUV is short for standard uptake value; and the right panel shows PET-CT images of ⁶⁸Ga-FAPI-CB-30 and ⁶⁸Ga-FAPI-04.
FIG. 8 shows a comparison of tumor SUV values between ⁶⁸Ga-FAPI-CB-31 and ⁶⁸Ga-FAPI-04 in the HT-1080-FAP tumor-bearing mouse model.
FIG. 9 shows comparative PET/CT imaging of Al¹⁸F-FAPI-CB-36 and ⁶⁸Ga-FAPI-04 in the HT-1080-FAP tumor-bearing mouse model.
FIG. 10 shows comparative PET/CT imaging of ⁶⁸Ga-FAPI-CB-50 and ⁶⁸Ga-FAPI-04 in the HT-1080-FAP tumor-bearing mouse model.
FIG. 11 shows comparative PET/CT imaging of ⁶⁸Ga-FAP-2286 and ⁶⁸Ga-FAPI-CB-45 in the HT-1080-FAP tumor-bearing mouse model.
FIG. 12 shows comparative PET/CT imaging of ⁶⁸Ga-FAPI-CB-53 and ⁶⁸Ga-FAPI-04 in the HT-1080-FAP tumor-bearing mouse model.
FIG. 13 shows comparative PET/CT imaging of ⁶⁸Ga-FAPI-CB-59 in the HT-1080-FAP tumor-bearing mouse model at different time points.
FIG. 14 shows comparative PET/CT imaging of Al¹⁸F-FAPI-CB-77 and Al¹⁸F-FAPI-74 in the HT-1080-FAP tumor-bearing mouse model.
FIG. 15 shows comparative PET/CT imaging of ⁶⁸Ga-FAPI-CB-86 and ⁶⁸Ga-FAPI-04 in the HT-1080-FAP tumor-bearing mouse model.
FIG. 16 shows comparative PET/CT imaging of ⁶⁸Ga-FAPI-CB-93 and ⁶⁸Ga-FAPI-04 in the HT-1080-FAP tumor-bearing mouse model.
FIG. 17 shows comparative PET/CT imaging of ⁶⁸Ga-FAPI-CB-94 and ⁶⁸Ga-FAPI-04 in the HT-1080-FAP tumor-bearing mouse model.
FIG. 18 shows comparative PET/CT imaging of Al¹⁸F-FAPI-CB-95 and Al¹⁸F-FAPI-74 in the HT-1080-FAP tumor-bearing mouse model.
FIG. 19 shows a comparison of tumor SUV values between ⁶⁸Ga-FAPI-CB-30 and ⁶⁸Ga-FAPI-04 in the HT-1080-FAP mouse model at 60 minutes after injection.
FIG. 20 shows that in the HT-1080-FAP tumor mouse model, ratios of tumor to blood pool standard uptake values (SUV) of the compounds FAPI-CB-28 and FAPI-CB-30 according to the present disclosure are significantly higher than those of the control group (FAPI-04).
FIG. 21 shows that compared with FAPI-04, the FAPI-CB-31 molecule shows no significant difference in blood pool uptake at 1 hour, but has higher tumor uptake.
FIG. 22 shows that the blood pool uptake of the FAPI-CB-47 molecules decreases rapidly at 0.5 hours and 2 hours, while the tumor uptake increases continuously and is statistically significantly improved than that of FAPI-04.
FIG. 23 shows PET/CT imaging of ⁶⁸Ga-FAPI-CB-50 in a cancer patient.
FIG. 24 shows SPECT/CT imaging of ¹⁷⁷Lu-FAPI-CB-30 in a cancer patient.
FIG. 25 shows SPECT/CT imaging of ¹⁷⁷Lu-FAPI-CB-86 in a tumor-bearing mouse model.
FIG. 26 shows the therapeutic effect of ¹⁷⁷Lu-FAPI-CB-30 in a tumor-bearing mouse model.
FIG. 27 shows comparative PET/CT imaging of ⁶⁸Ga-FAPI-CB-30 and ⁶⁸Ga-FAPI-04 in a mouse model of renal fibrosis.

### DETAILED DESCRIPTION

The present disclosure provides a trifunctional compound by introducing a covalent warhead into a drug. The trifunctional compound exhibits significantly enhanced uptake and retention at target sites, such as tumors, while demonstrating low uptake in non-target organs, thereby resulting in significantly reduced toxicity. Furthermore, the trifunctional compound according to the invention has excellent pharmacokinetic properties.

Before the present disclosure is further described, some terms used in the specification, examples, and appended claims are illustrated in the following sections. The definitions listed herein shall be read and understood by persons skilled in the art with reference to the remainder of the present disclosure. Unless otherwise defined, all technical and scientific terms used herein have the same meanings as those commonly understood by persons of ordinary skill in the art to which this application pertains.

### Definitions

Unless otherwise specified, when any type of range is disclosed or claimed, it is intended that each possible value that could be reasonably encompassed in the range is individually disclosed or claimed, including any sub-range encompassed therein. For example, the number of groups is 1 to 6 indicates an integer within the range. It should be understood that the range of 1 to 6 includes 1, 2, 3, 4, 5, and 6, and also includes subranges of 1 to 5, 1 to 4, and 1 to 3.

The description of the present disclosure should be construed as being in accordance with a law and principle of a chemical bond. In some cases, a hydrogen atom may be removed in order to adapt a substituent at a given position.

As used in the present disclosure, the terms "comprise," "contain," "include," and similar expressions indicate that the elements preceding such terms encompass the elements listed thereafter and their equivalents, without excluding unlisted elements. The terms "contain" or "include" as used herein may be open-ended, semi-closed, or closed in nature. In other words, these terms also encompass the meanings of "consisting essentially of" or "consisting of".

The term "pharmaceutically acceptable" as used herein means that a compound or composition is chemically and/or toxicologically compatible with other ingredients constituting a preparation and/or with the human or mammal administered with the compound or composition for prevention or treatment of a disease or a condition.

The term "subject" or "patient" as used herein includes humans and mammals. In some embodiments, a "patient" is an individual who has been diagnosed with, is suspected of having, or is at risk of developing or forming a disease, where the disease is a disease described herein, preferably a disease involving FAP.

In the context of this application, unless specifically stated to the contrary, the term "treatment" may also include prevention.

The term "alkyl group" refers to a saturated straight or branched carbon chain. Preferably, the chain contains 1 to 10 carbon atoms, i.e., 1, 2, 3, 4, 5, 6, 7, 8, 9, or 10 carbon atoms, preferably 1 to 6 carbon atoms, most preferably 1 to 3 carbon atoms. The alkyl group is, for example, a methyl group, an ethyl group, an n-propyl group, an isopropyl group, a butyl group, an isobutyl group, a tert-butyl group, a pentyl group, a hexyl group, a heptyl group, or an octyl group. The alkyl group is optionally substituted.

The term "heteroalkyl group" refers to a saturated straight or branched carbon chain. Preferably, the chain contains 1 to 9 carbon atoms, i.e., 1, 2, 3, 4, 5, 6, 7, 8, or 9 carbon atoms, preferably 1 to 6 carbon atoms, most preferably 1 to 3 carbon atoms, for example, a methyl group, an ethyl group, a propyl group, an isopropyl group, a butyl group, an isobutyl group, a sec-butyl group, a tert-butyl group, a pentyl group, a hexyl group, a heptyl group, or an octyl group, which is interrupted by the same or different heteroatoms once or more than once, for example once, twice, three times, four times, or five times. Preferably, the heteroatoms are selected from O, S, and N, for example -O-CH₃, -S-CH₃, -CH₂-O-CH₃, -CH₂-O-C₂H₅, -CH₂-S-CH₃, -CH₂-S-C₂H₅, -C₂H₄-O-CH₃, -C₂H₄-O-C₂H₅, -C₂H₄-S-CH₃, and -C₂H₄-S-C₂H₅. The heteroalkyl group is optionally substituted.

Unless otherwise specified, the terms "cycloalkyl group" and "heterocycloalkyl group" or combinations of the terms and other terms respectively refer to cyclic forms of "alkyl group" and "heteroalkyl group", preferably, with 3, 4, 5, 6, 7, 8, 9, or 10 atoms forming the ring, for example a cyclopropyl group, a cyclobutyl group, a cyclopentyl group, a cyclohexyl group, a cycloheptyl group, or a cyclooctyl group. The terms "cycloalkyl group" and "heterocycloalkyl group" are also intended to include their bicyclic, tricyclic, and polycyclic forms. The term "heterocycloalkyl group" preferably refers to a 4-membered saturated ring in which at least one ring member is an N atom; a 5-membered saturated ring in which at least one ring member is an N, O, or S atom and which optionally contains one additional O atom or one additional N atom; a 6-membered saturated ring in which at least one ring member is an N, O, or S atom and which optionally contains one additional O atom or one or two additional N atoms; or a 9-membered or 10-membered saturated bicyclic ring in which at least one ring member is an N, O, or S atom and which optionally contains one, two, or three additional N atoms. The "cycloalkyl group" and "heterocycloalkyl group" are optionally substituted, where "optionally substituted" also means that a carbon atom on the ring is substituted with a carbonyl group (C=O). Examples of cycloalkyl groups include a cyclopropyl group, a cyclobutyl group, a cyclopentyl group, a cyclohexyl group, a 1-cyclohexenyl group, a 3-cyclohexenyl group, a cycloheptyl group, a spiro[3,3]heptyl group, a spiro[3,4]octyl group, a spiro[4,3]octyl group, a spiro[3,5]nonyl group, a spiro[5,3]nonyl group, a spiro[3,6]decyl group, a spiro[6,3]decyl group, a spiro[4,5]decyl group, a spiro[5,4]decyl group, a bicyclo[2.2.1]heptyl group, a bicyclo[2.2.2]octyl group, an adamantyl group, and the like. Examples of heterocycloalkyl groups include an azetidinyl group, a 1-(1,2,5,6-tetrahydropyridyl) group, a 1-piperidinyl group, a 2-piperidinyl group, a 3-piperidinyl group, a 4-morpholinyl group, a 3-morpholinyl group, a 1,4-diazabicyclo[2.2.2]oct-2-yl group, a tetrahydrofuran-2-yl group, a tetrahydrofuran-3-yl group, a tetrahydrothiophen-2-yl group, a tetrahydrothiophen-3-yl group, a 1-piperazinyl group, a 2-piperazinyl group, and the like.

The term "heterocyclyl group" or "heterocycle" used herein refers to a non-aromatic 5-, 6-, or 7-membered ring or a polycyclic group, including but not limited to bicyclic or tricyclic groups containing fused, spiro, or bridged 3-membered, 4-membered, 5-membered, 6-membered, or 7-membered rings, where at least one carbon atom in one of the rings is substituted with a heteroatom. Each heteroatom is independently selected from atoms of oxygen, sulfur (including sulfoxides and sulfones), and/or nitrogen (which may be oxidized or quaternized). The term "heterocyclyl group" or "heterocycle" is intended to include groups with rings in which the -CH₂- is substituted by -C(=O)-, for example cyclic ureido groups (for example, 2-imidazolidinone), cyclic amido groups (for example, β-lactam, γ-lactam, δ-lactam, and ε-lactam), and piperazin-2-one; where (i) each 5-membered ring has 0 or 1 double bond, and each 6-membered ring has 0 to 2 double bonds; (ii) nitrogen and sulfur heteroatoms may optionally be oxidized; (iii) nitrogen heteroatoms may optionally be quaternized; and (iv) any of the preceding heterocycles may be fused to an aryl or heteroaryl ring, for example a benzene ring. Representative heterocycles include, but are not limited to, an azetidinyl group, a pyrrolidinyl group, a pyrazolinyl group, a pyrazolidinyl group, an imidazolidinyl group, a piperidinyl group, a piperazinyl group, an oxazolidinyl group, an isoxazolidinyl group, a morpholinyl group, a thiazolidinyl group, an isothiazolidinyl group, and a tetrahydrofuranyl group.

The term "aryl group" preferably refers to an aromatic monocyclic ring containing 6 carbon atoms, an aromatic bicyclic system containing 10 carbon atoms, or an aromatic tricyclic system containing 14 carbon atoms. An example is a phenyl group, a naphthyl group, or an anthracenyl group. The aryl group is optionally substituted.

The term "heteroaryl group" preferably refers to a 5-membered or 6-membered aromatic monocyclic ring in which at least one carbon atom is substituted by 1, 2, 3, or 4 (for 5-membered rings) or 1, 2, 3, 4, or 5 (for 6-membered rings) identical or different heteroatoms, and the heteroatoms are preferably selected from O, N, and S; an aromatic bicyclic system in which 1, 2, 3, 4, 5, or 6 of 8, 9, 10, 11, or 12 carbon atoms are substituted by identical or different heteroatoms, and the heteroatoms are preferably selected from O, N, and S; or an aromatic tricyclic system in which 1, 2, 3, 4, 5, or 6 of 13, 14, 15, or 16 carbon atoms are substituted by identical or different heteroatoms, and the heteroatoms are preferably selected from O, N, and S. The N atom in the heteroaryl group may be present in the form of an onium group. For example, an alkyl group or oxygen is attached to the trivalent aromatic N to form an onium group, that is, "heteroaryl group" in the context of this application includes a heteroaryl group in a free form (the N atom is not salified) and a heteroaryl group in the form of an onium salt. An example is a pyridyl group, an N-oxidopyridyl group, an N-alkylpyridyl group, an oxazolyl group, an isoxazolyl group, a 1,2,5-oxadiazolyl group, a 1,2,3-oxadiazolyl group, a pyrrolyl group, an imidazolyl group, a pyrazolyl group, a 1,2,3-triazolyl group, a thiazolyl group, an isothiazolyl group, a 1,2,3-thiadiazolyl group, a 1,2,5-thiadiazolyl group, a pyridyl group, a pyrimidinyl group, a pyrazinyl group, a 1,2,3-triazinyl group, a 1,2,4-triazinyl group, a 1,3,5-triazinyl group, a 1-benzofuranyl group, a 2-benzofuranyl group, an indolyl group, an isoindolyl group, a benzothienyl group, a 2-benzothienyl group, a 1H-indazolyl group, a benzimidazolyl group, a benzoxazolyl group, an indolizinyl group, a 2,1-benzoxazolyl group, a benzothiazolyl group, a 1,2-benzisothiazolyl group, a 2,1-benzisothiazolyl group, a benzotriazolyl group, a quinolyl group, an isoquinolyl group, a quinoxalinyl group, a quinazolinyl group, a quinolyl group, a 1,2,3-benzotriazinyl group, or a 1,2,4-benzotriazinyl group.

As used herein, the term "linker" refers to any chemically suitable linker. Preferably, the linker does not break or breaks only slowly under physiological conditions. When multiple substituents or linkers are described using their conventional chemical formulas written from left to right, such substituents or linkers also encompass chemically equivalent structures resulting from right-to-left notation. For example, -CH₂O- is equivalent to -OCH₂-, -C(=O)O-is equivalent to -OC(=O)-; and -OC(=O)NR- is equivalent to -NRC(=O)O-.

When the phrase "independently selected from" is used, multiple substituents mentioned (for example, multiple R groups such as groups R₁ and R₂; or variables such as "m" and "n") may be the same or different.

The term "protecting group" refers to multiple chemical moieties that block some or all reactive moieties of a compound and prevent the multiple moieties from participating in multiple chemical reactions until the protecting group is removed (for example, multiple moieties listed and described by T.W. Greene and P.G.M. Wuts, Protective Groups in Organic Synthesis, 3rd Edition, John Wiley & Sons, Inc. (1999)). When different protecting groups are used, it may be beneficial to remove each (different) protecting group in a different manner. Cleavage of the multiple protecting groups under completely different reaction conditions allow for the differential removal of these protecting groups. For example, the multiple protecting groups can be removed by an acid, a base, and hydrogenolysis. For example, multiple groups such as a trityl group, a dimethoxytrityl group, acetal, and a tertbutyldimethylsilyl group are acid-labile and can be used to protect the reactive moieties of carboxyl and hydroxyl groups in the presence of multiple amino groups protected by multiple Cbz groups (which can be removed by hydrogenolysis) and base-labile Fmoc groups. In the presence of amines blocked by multiple acid-labile groups such as tert-butyl carbamate, the reactive moieties of carboxylic acids and hydroxyl groups can be blocked by multiple base-labile groups such as a methyl group, an ethyl group, and an acetyl group, or by carbamate that is stable in both acid and base but can be removed by hydrolysis.

The expression "optionally substituted" means that one or more hydrogen atoms in a group may be substituted with substituents independently of one another. The substituents may be selected from a C₁-C₆ alkyl group, a heteroalkyl group, a cycloalkyl group, a heterocycloalkyl group, an aryl group, a heteroaryl group, halogen, a cyano group, an amino group, a nitro group, -OH, and -COOH.

As used herein, the term "radionuclide" refers to a radioactive isotope of an element that emits α particles, β particles, and/or γ rays. Types of the radionuclides include, but are not limited to, the following types: ¹⁸F, ⁵¹Cr, ⁶⁷Ga, ⁶⁸Ga, ⁸⁹Zr, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁸⁸Y, ⁹⁰Y, ¹⁴⁹Pm, ¹⁶¹Tb, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th, and ¹⁹⁹Ag.

The term "radiopharmaceutical" used in the context of the present disclosure refers to a biologically active compound modified by a radioactive isotope or radionuclide.

In the context of the present disclosure, the terms "chelating agent" and "chelate" are used interchangeably, which refer to molecules that have two or more unshared electron pairs available for donation to a metal ion, and are typically organic molecules and usually Lewis bases. Metal ions typically coordinate with the chelating agent via two or more electron pairs. The terms "bidentate chelating agent", "tridentate chelating agent", and "tetradentate chelating agent" refer to chelating agents having two, three and four electron pairs, respectively, which are also prone to provide metal ions coordinated by the chelating agent. Typically, the electron pair of the chelating agent forms a coordinate bond with a single metal ion. However, in some instances, the chelating agent can form coordinate bonds with one or more metal ions, and various binding modes are possible. The term "chelating moiety" refers to a group formed by removing one or more hydrogen atoms from the "chelating agent" or "chelate".

The term "optical dye" refers to a compound that emits visible light or infrared light after being excited by electromagnetic radiation of a shorter and appropriate wavelength. It can be understood by persons skilled in the art that each optical dye has a predetermined excitation wavelength.

The term "nuclear pharmaceutical molecule" or "nuclear pharmaceutical" refers to a molecule or compound that carries or chelates a radionuclide.

The term "structure targeting fibroblast activation protein-α" refers to a molecular fragment derived from a fibroblast activation protein inhibitor, for example a molecular fragment formed by the compounds disclosed in WO2019154886A1.

The term "pharmaceutically acceptable salt" refers to a relatively non-toxic addition salt of the compound in the present disclosure (for example, S. M. Berge et al. "Pharmaceutical Salts", J. Pharm. Sci. 1977, 66, 1-19).

Suitable pharmaceutically acceptable salts of the compounds disclosed herein may include acid addition salts of the disclosed compounds that possess sufficient basicity and carry nitrogen atoms in a chain or ring structure, for example, acid addition salts formed with the following inorganic acids: for example, hydrochloric acid, hydrobromic acid, hydroiodic acid, sulfuric acid, phosphoric acid or nitric acid, or acid addition salts formed with the following organic acids: formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, 3-hydroxy-2-naphthoic acid, niacin, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, laurylsulfuric acid, ethanesulfonic acid, benzenesulfonic acid, p-toluenesulfonic acid, methanesulfonic acid, 2-naphthalenesulfonic acid, naphthalene disulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptanoic acid, glycerophosphoric acid, aspartic acid, sulfosalicylic acid or thiocyanic acid.

In addition, another suitable pharmaceutically acceptable salt of the compounds of the present disclosure with sufficient acidity includes alkali metal salts (such as sodium or potassium salts), alkaline earth metal salts (such as calcium or magnesium salts), ammonium salts, or salts formed with organic bases that provide physiologically acceptable cations, for example, salts formed with the following substances: N-methylglucamine, dimethylglucamine, ethylglucamine, lysine, dicyclohexylamine, 1,6-hexamethylenediamine, ethanolamine, glucosamine, sarcosine, serinol, tris(hydroxymethyl)aminomethane, aminopropylene glycol, or 1-amino-2,3,4-butanetriol. In addition, a basic nitrogen-containing group can be quaternized with the following reagents: lower alkyl halides such as chlorides, bromides and iodides of methyl, ethyl, propyl and butyl; dialkyl sulfates such as dimethyl sulfate, diethyl sulfate, dibutyl sulfate and diamyl sulfate; long-chain halides such as chlorides, bromides and iodides of decyl, lauryl, myristyl and stearyl; and aralkyl halides such as benzyl and phenethyl bromides.

Persons skilled in the art should also understand that an acid addition salt of the claimed compound may be prepared via reaction of the compound and a suitable inorganic or organic acid in any one of various known methods. Alternatively, the alkali metal and alkaline earth metal salts of the acidic compounds in the present disclosure are prepared via reaction of the acidic compound and an appropriate base in various known methods.

The present disclosure includes all possible salts of the disclosed compounds, which may be a single salt or any mixture of the salts in any ratio.

The term "solvate" refers to a substance formed by combining, physically associating with, and/or solvating a compound in the present disclosure with solvent molecules, for example a disolvate, monosolvate, or hemisolvate, and a ratio of solvent molecules to the compound in the present disclosure is about 2:1, about 1:1, or about 1:2, respectively. This physical association involves ionization and covalent bonding (including hydrogen bonding) to various degrees. In some cases (for example, when one or more solvent molecules are incorporated into crystal lattice of a crystalline solid), the solvate can be isolated. Therefore, the solvates include solution-phase solvates and separable solvates. The compound in the present disclosure can exist in a solvated form together with pharmaceutically acceptable solvents (for example, water, methanol, and ethanol), and this application is intended to encompass both the solvated and non-solvated forms of the compound in the present disclosure. A solvate may be a hydrate.

The compound in the present disclosure may contain one or more asymmetric centers, which depends on desired locations and features of various substituents. Asymmetric carbon atoms can exist in an (R) or (S) configuration, a racemic mixture is obtained in the case of one asymmetric center, and a diastereoisomer mixture is obtained in the case of multiple asymmetric centers. In some cases, asymmetry may also exist due to hindered rotation around a particular bond. For example, a central bond is connected to two substituted aromatic rings of a particular compound.

Preferred compounds are compounds that can produce more desirable biological activity. Isolated, purified or partially purified isomers and stereoisomers, or racemic or diastereoisomer mixtures of the disclosed compound all fall within the scope of the present invention. Purification and isolation of such a substance can be achieved via a standard technology known in the art.

The term "pharmaceutical composition" as used herein refers to a substance and/or a combination of substances used for identifying, preventing, or treating a tissue condition or disease. The pharmaceutical composition is prepared to be suitable for administration to patients for diagnosis, prevention, and/or treatment of diseases. In addition, the pharmaceutical composition refers to a combination of an active agent and an inert or active carrier, and in this way, the composition is suitable for therapeutic use.

"Pharmaceutically acceptable" means being approved by a regulatory agency of the federal or state government, or listed in the United States Pharmacopeia or other universally acknowledged pharmacopeias for use in animals, particularly in humans.

As used herein, the term "carrier" refers to a diluent, an adjuvant, an excipient, or a vehicle administered together with a therapeutic agent. This pharmaceutical carrier can be a sterile liquid, for example saline solutions in water and oil including those of petroleum, animals, plants, or synthetic origin, for example peanut oil, soybean oil, mineral oil, and sesame oil. When the pharmaceutical composition is administered intravenously, a saline solution is the preferred carrier. Saline solutions, aqueous glucose solutions and glycerol solutions can also be used as liquid carriers for injectable solutions in particular. Suitable pharmaceutical excipients include starch, glucose, lactose, sucrose, gelatin, malt, rice, flour, chalk, silica gel, sodium stearate, glyceryl monostearate, talc, sodium chloride, skim milk powder, glycerol, propylene, ethylene glycol, water, ethanol, and the like. If needed, the composition may also contain small amounts of wetting agents, emulsifiers, or pH buffering agents. Examples of suitable pharmaceutical carriers are described in "Remington's Pharmaceutical Sciences" by E. W. Martin.

The term "halogen" refers to fluorine, chlorine, bromine, and iodine.

The term "optional" means that the situation may or may not occur.

The phrase "not directly connected via a covalent bond" means that there is at least one carbon atom therebetween, and the carbon atom may be present in the form of C, CH, CH₂, or C=O.

As used herein, diseases involving FAP refer to diseases that the cells (including, but not limited to, fibroblasts) that express FAP (preferably, in an upregulated manner), as well as tissues that express FAP or contain cells (for example, fibroblasts) which preferably express FAP in an upregulated manner, are respectively causes of the diseases and/or symptoms thereof, or are part of the underlying pathology of the diseases. The preferred cells that express FAP are cancer-associated fibroblasts (CAFs). In the implementation of the disease, preferably, when used in conjunction with the treatment, management, and/or therapy of the disease, the pharmaceutical composition separately affects the cells, tissues, and pathology, to cure, treat, or improve the disease and/or the symptom thereof. In the implementation of the disease, preferably, when used in conjunction with the diagnosis and/or performance of diagnosis of the disease, the FAP-expressing cells and/or FAP-expressing tissues are labeled, to distinguish or differentiate such cells and/or tissues from healthy or non-FAP-expressing cells, and/or healthy or non-FAP-expressing tissues. More preferably, such distinction or differentiation forms the basis for the diagnosis and the performance of diagnosis, respectively. In the implementation, labeling refers to the direct or indirect interaction between a detectable label and FAP-expressing cells and/or FAP-expressing tissues, or tissues containing such FAP-expressing cells; more preferably, such interaction involves or is based on the interaction between the label or a compound carrying such a label and FAP.

As used herein, a neoplasm is generated due to abnormal new growth of cells. The cells in the neoplasm grow faster than normal cells and will continue to grow if left untreated. The neoplasm can be benign or malignant.

As used herein, the tumor is a mass lesion and can be benign or malignant.

As used herein, cancer refers to a malignant neoplasm.

The amino acid sequences of the peptides provided herein are described in a typical peptide sequence format. For example, the three-letter codes for conventional amino acids, codes for unconventional amino acids, or abbreviations of other building blocks indicate the amino acids or building blocks exist at specific positions in the peptide sequence. The code for each amino acid or building block is connected to the code for the next and/or previous amino acid or building block in the sequence through a hyphen (which usually represents an amide bond).

When an amino acid contains more than one amino group and/or carboxyl group, all orientations of this amino acid are theoretically feasible. However, for α-amino acids, α-amino group and α-carboxyl group are preferably used, while other preferred orientations are clearly specified.

For amino acids, in abbreviations thereof, the first letter indicates stereochemistry of C-α atom (if applicable). For example, an uppercase initial letter indicates that L-amino acid is present in a peptide sequence, while a lowercase initial letter indicates that corresponding D-amino acid is present in the peptide sequence.

As used herein, aromatic L-α-amino acid is any type of L-α-amino acid that contains an aryl group.

As used herein, a heteroaromatic L-α-amino acid is any type of L-α-amino acid that contains a heteroaryl group.

Unless specified otherwise, the amino acid sequence is shown in a direction from the N-terminus to the C-terminus herein.

The amino acid in the present disclosure may be a conventional amino acid (also referred to as a natural amino acid) or an unnatural amino acid (any type of non-oligomeric compound that contains an amino group and a carboxyl group and is not a conventional amino acid).

The radionuclide in the present disclosure has a half-life that allows for diagnostic and/or therapeutic medical use. Specifically, the half-life ranges from 1 minute to 100 days.

In a preferred embodiment of the present invention, the radionuclide has decay energy that allows for diagnostic and/or therapeutic medical use. Specifically, for γ-ray emitting isotopes, the decay energy for diagnostic use ranges from 0.004 MeV to 10 MeV, preferably from 0.05 MeV to 4 MeV. For positron emitting isotopes, the decay energy for diagnostic use ranges from 0.6 MeV to 13.2 MeV, preferably from 1 MeV to 6 MeV. For particle emitting isotopes, the decay energy for diagnostic use ranges from 0.039 MeV to 10 MeV, preferably from 0.4 MeV to 6.5 MeV.

In a preferred embodiment of the present invention, the radionuclide is industrially manufactured for medical use. Specifically, the radionuclide can meet GMP quality standards.

In a preferred embodiment of the present invention, the daughter nuclide generated after radioactive decay of the radionuclide is suitable for diagnostic and/or therapeutic medical use. In addition, the daughter nuclide is stable or further decays in a manner in which diagnostic and/or therapeutic medical use is not interfered with or is even supported.

### Examples

### Reagents and Models

A starting material in the example is commercially available and/or can be prepared in various methods well known to persons skilled in the art of organic synthesis. Persons skilled in the art of organic synthesis appropriately select a reaction condition (including a solvent, reaction atmosphere, reaction temperature, duration of a test, and a post-treatment) in the following synthesis method. Persons skilled in the art of organic synthesis understand that a functional group on each part of a molecule should be compatible with a proposed reagent and reaction.

All synthesized reagents and compounds can be purchased through general commercial channels in China (excluding Hong Kong, Macao and Taiwan), where suppliers include Bide Pharmatech Co., Ltd., J&K (Beijing, China), Inno-chem (Beijing, China) and Energy Chemical (Shanghai, China). Unless otherwise specified, no synthesized reagents or compounds are further purified. SO₂F₂ gas was purchased from Shang Fluoro (Shanghai, China). All solvents used for synthesis were purchased from Beijing Tong Guang Fine Chemicals Company (Beijing, China), and HPLC-grade solvents were purchased from Fisher Scientific (Loughborough, UK).

Nuclide: ⁶⁸GaCl₃ was prepared by eluting a ⁶⁸Ge-⁶⁸Ga generator (iThemba LABS, South Africa) with 0.6 M hydrochloric acid. A solution of ¹⁷⁷LuCl₃ in 0.1 M hydrochloric acid was purchased from ITG (Germany).

Recombinant protein: The human-derived recombinant protein FAP-His tag was purchased from Novoprotein Scientific Inc. (China).

Cell model: The human fibrosarcoma cell line (HT-1080-FAP) with high FAP expression was constructed by transfecting the FAP plasmid into the human fibrosarcoma cell line (HT-1080) by WuXi Biologics (China).

Mouse model: Nu/Nu mice (SPF grade) were purchased from Beijing Vital River Laboratory Animal Technology Co., Ltd. (China), and were subcutaneously injected with HT-1080-FAP cells to construct a tumor model with high FAP expression.

### Instruments

Preparation and identification of compounds: High-Performance Liquid Chromatography (Waters), Radioactivity Detector for High-Performance Liquid Chromatography (Eckert & Ziegler Group), Ultra-High Performance Liquid Chromatography-Mass Spectrometry (Waters), High-Resolution Mass Spectrometer (Orbitrap Fusion Lumos or Bruker Solarix XR), Nuclear Magnetic Resonance Spectrometer (Bruker 400/500/600 Mhz), and Amersham Typhoon Imaging System (Amersham Typhoon RGB).

Animal experiments: Small-animal PET/CT or SPECT/CT (Mediso nanoScan^{®} PET122S or Novel Medical InliView-3000B).

### Example 1: Synthesis of Compounds

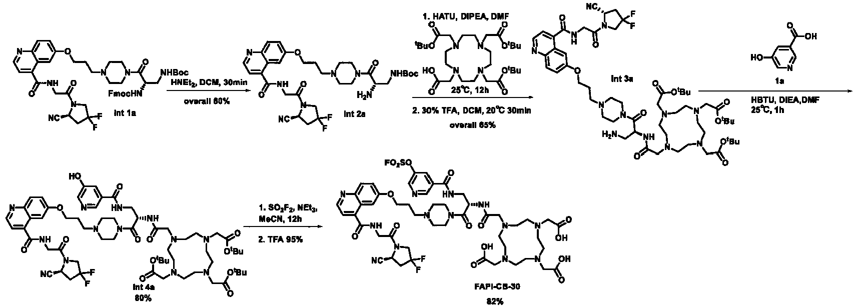

Compound FAPI-CB-30 according to the present disclosure can be synthesized via the foregoing synthetic route. In addition, some FAP-targeting compounds in the invention can be synthesized via similar synthetic routes.

### Example 1a: Synthesis of FAPI-CB-30

The initial intermediate int 1a was prepared via steps described in "Albumin Binder-Conjugated Fibroblast Activation Protein Inhibitor Radiopharmaceuticals for Cancer Therapy" (Xu, M. et al., J. Nucl. Med. 2021, jnumed.121.262533.), except that the protective lysine used therein was replaced with protective (S)-2,3-diaminopropionic acid. NHEt₂ (973.0 mg, 13.34 mmol, 1.05 mL, 26.7 eq.) was added to a solution of int 1a (450 mg, 0.5 mmol, 1.0 eq.) in acetonitrile (10.0 mL), and the reaction solution was stirred at 25°C for 30 minutes. The reaction solution was concentrated under reduced pressure to obtain a crude product, and the crude product was purified by HPLC [eluent A: H₂O (0.1% trifluoroacetic acid); and eluent B: acetonitrile, 10% B (at 2 min) to 95% B (at 20 min)], to obtain int 2 (268.8 mg, 80%) as a white solid.

DOTA-Tris (tBu) (0.5g, 0.9 mmol, 1.8 eq.), HATU (365 mg, 1.0 mmol, 2.0 eq.), and DIEA (78 mg, 0.6 mmol, 105 µL, 1.2 eq.) were added to a solution of int 2a (336 mg, 0.5 mmol, 1.0 eq.) in DMF (5.0 mL), and the reaction solution was stirred at 25°C for 12 hours. The reaction solution was added into methyl tert-butyl ether (200 mL), and the resulting mixture was stirred for 30 minutes to obtain a precipitate.

The precipitate was dissolved in dichloromethane (10 mL), trifluoroacetic acid (3 mL) was added to the solution, and the resulting mixture was allowed to react at 20°C for 30 minutes. The crude product was purified by HPLC [eluent A: H₂O (0.1% trifluoroacetic acid); and eluent B: ACN, 20% B (at 2 min) to 95% B (at 20 min)], to obtain int 3a (0.366g, yield: 65%) as a white solid.

Int 3a (11.3 mg, 10 µmol, 1.0 eq.) was dissolved in DMF (200 µL). Commercially available 1a (1.7 mg, 12.2 µmol, 1.22 eq.), DIPEA (3.9 mg, 30 µmol, 5.3 µL, 3.0 eq.), and HBTU (9.9 mg, 12.0 µmol, 1.2 eq.) were added, and the reaction mixture was stirred at 25°C for 30 minutes. Then intermediate int 4a (10.32 mg, 80%) was isolated by HPLC. After the reaction mixture was dissolved in acetonitrile (200 µL), the gas in the container was replaced with SO₂F₂ gas, 4.0 equivalents of triethylamine were added, and the reaction solution was allowed to react for 12 hours. The reaction solution was concentrated under reduced pressure to remove the solvent, then 1.0 mL of TFA was added, and the reaction mixture was allowed to react at 25°C for 3 hours. The crude product was purified by HPLC [eluent A: H₂O (0.1% TFA); and eluent B: ACN, 10% B (at 2 min) to 60% B (at 20 min)], to obtain FAPI-CB-30 (7.87 mg, 82%) as a white solid. UPLC-MS characterization conditions: Column: ACQUITY UPLC BEH C₁₈ 1.7 µm, 2.1×50 mm. Method: Flow rate: 0.5 mL/min; eluent A: H₂O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1162, and retention time: 1.56 minutes.

### Example 1b: Synthesis of FAPI-CB-31

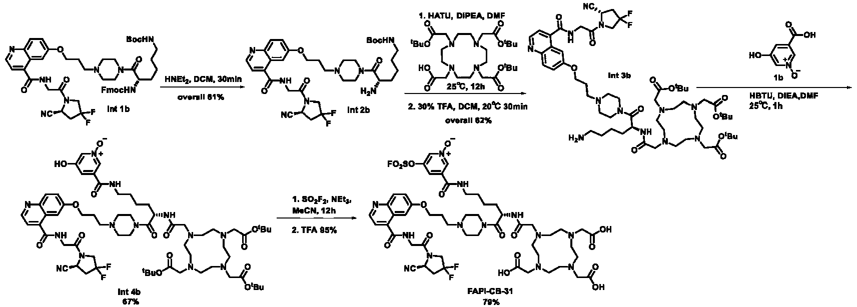

The compound int 1b was prepared via steps described in "Albumin Binder-Conjugated Fibroblast Activation Protein Inhibitor Radiopharmaceuticals for Cancer Therapy" (Xu, M. et al., J. Nucl. Med. 2021, jnumed.121.262533.). NHEt₂ (1249.0 mg, 17.1 mmol, 1.343 mL, 26.7 eq.) was added to a solution of int 1b (600 mg, 641.0 mmol, 1.0 eq.) in acetonitrile (5.0 mL), and the reaction solution was stirred at 25°C for 30 minutes. The reaction solution was concentrated under reduced pressure to obtain a crude product, and the crude product was purified by HPLC [eluent A: H₂O (0.1% trifluoroacetic acid); and eluent B: acetonitrile, 10% B (at 2 min) to 95% B (at 20 min)], to obtain int 2b (370.7 mg, yield: 81%) as a white solid.

DOTA-Tris (tBu) (0.5g, 0.9 mmol, 1.8 eq.), HATU (365 mg, 1.0 mmol, 2.0 eq.), and DIEA (78 mg, 0.6 mmol, 105 µL, 1.2 eq.) were added to a solution of int 2b (357 mg, 0.5 mmol, 1.0 eq.) in DMF (5.0 mL), and the reaction solution was stirred at 25°C for 12 hours. The reaction solution was added into methyl tert-butyl ether (200 mL), and the resulting mixture was stirred for 30 minutes to obtain a precipitate.

The precipitate was dissolved in dichloromethane (10 mL), trifluoroacetic acid (3 mL) was added to the solution, and the resulting mixture was allowed to react at 20°C for 30 minutes. The crude product was purified by HPLC [eluent A: H₂O (0.1% trifluoroacetic acid); and eluent B: ACN, 20% B (at 2 min) to 95% B (at 20 min)], to obtain int 3b (0.362g, yield: 62%) as a white solid.

Int 3b (11.7 mg, 10.0 µmol, 1.0 eq.) was dissolved in DMF (200 µL). Readily available 1b (3.9 mg, 12.0 µmol, 1.2 eq.) obtained by oxidizing commercially available 1a with 35% hydrogen peroxide, DIPEA (3.9 mg, 30.0 µmol, 5.6 µL, 3.0 eq.), and HBTU (4.6 mg, 12.0 µmol, 1.2 eq.) were added to the reaction mixture, and the resulting mixture was stirred at 25°C for 30 minutes. Then intermediate int 4b (8.8 mg, 67%) was isolated by HPLC. After the reaction mixture was dissolved in acetonitrile (200 µL), the gas in the container was replaced with SO₂F₂ gas, 4.0 equivalents of triethylamine were added, and the reaction solution was allowed to react for 12 hours. The reaction solution was concentrated under reduced pressure to remove the solvent, then 1.0 mL of TFA was added, and the reaction mixture was allowed to react at 25°C for 3 hours. The crude product was purified by HPLC [eluent A: H₂O (0.1% TFA); and eluent B: ACN, 10% B (at 2 min) to 60% B (at 20 min)], to obtain FAPI-CB-31 (6.5 mg) as a white solid. UPLC-MS characterization conditions: Column: ACQUITY UPLC BEH C18 1.7 µm, 2.1×50 mm. Method: Flow rate: 0.5 mL/min; eluent A: H₂O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1220, and retention time: 1.32 minutes.

### Example 1c: Synthesis of FAPI-CB-44

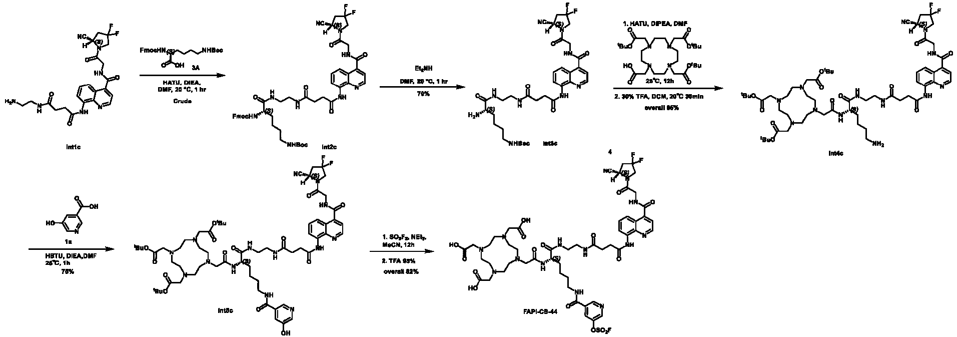

The compound int 1c was prepared via steps described in "An ultra-high-affinity small organic ligand of fibroblast activation protein for tumor-targeting applications" (Millul, J., PNAS 2021, 118 (16) e2101852118).

Commercially available 3A (468 mg, 1.0 mmol, 1.0 eq.), DIPEA (390 mg, 3.0 mmol, 527 µL, 3.0 eq.), and HATU (456.8 mg, 1.2 mmol, 1.2 eq.) were added to a solution of int 1c (501 mg, 1.0 mmol, 1.0 eq.) in DMF (10.0 mL), and after the reaction mixture was stirred at 20°C for 60 minutes, UPLC-MS analysis showed a main peak with the desired mass ([M+H]⁺ = 952). The reaction mixture was diluted with H₂O until the volume reached 20.0 mL, and extracted with ethyl acetate (20.0 mL per round, 3 rounds). The combined organic layers were washed with 20.0 mL of brine, dried over Na₂SO₄, filtered, and concentrated under reduced pressure to obtain a residue. The crude product was used for the next step without further purification. The crude product int 2c (813 mg) was obtained as a yellow solid.

NHEt₂ (1950.2 mg, 26.7 mmol, 2.097 mL, 26.7 eq.) was added to a solution of int 2c (1.0 mmol, 1.0 eq.) in DMF (10.0 mL), and the reaction solution was stirred at 20°C for 60 minutes. The reaction solution was concentrated under reduced pressure to obtain a crude product, and the crude product was purified by preparative HPLC [eluent A: H₂O (0.1% trifluoroacetic acid); and eluent B: acetonitrile, 10% B (at 2 min) to 95% B (at 20 min)], to obtain int 3c (511.7 mg, yield: 70.2%) as a white solid.

DOTA-Tris (tBu) (0.5g, 0.9 mmol, 1.8 eq.), HATU (365 mg, 1.0 mmol, 2.0 eq.), and DIEA (78 mg, 0.6 mmol, 105 µL, 1.2 eq.) were added to a solution of int 3c (364 mg, 0.5 mmol, 1.0 eq.) in DMF (5.0 mL), and the reaction solution was stirred at 25°C for 12 hours. The reaction solution was added into methyl tert-butyl ether (200 mL), and the resulting mixture was stirred for 30 minutes to obtain a precipitate.

The precipitate was dissolved in dichloromethane (10 mL), trifluoroacetic acid (3 mL) was added to the solution, and the resulting mixture was allowed to react at 20°C for 30 minutes. The crude product was purified by HPLC [eluent A: H₂O (0.1% trifluoroacetic acid); and eluent B: ACN, 20% B (at 2 min) to 95% B (at 20 min)], to obtain int 4c (0.385g, 65%) as a white solid.

Int 4c (11.8 mg, 10.0 µmol, 1.0 eq.) was dissolved in DMF (200 µL). Commercially available 1a (3.9 mg, 12.0 µmol, 1.2 eq.), DIPEA (3.9 mg, 30.0 µmol, 5.6 µL, 3.0 eq.), and HBTU (4.6 mg, 12.0 µmol, 1.2 eq.) were added to the reaction mixture, and the resulting mixture was stirred at 25°C for 30 minutes. Then intermediate int 5c (9.7 mg, 75%) was isolated by HPLC. After the reaction mixture was dissolved in acetonitrile (500 µL), the gas in the container was replaced with SOF₂ gas, 4.0 equivalents of triethylamine were added, and the reaction solution was allowed to react for 12 hours. The reaction solution was concentrated under reduced pressure to remove the solvent, then 1.0 mL of TFA was added, and the reaction mixture was allowed to react at 25°C for 3 hours. The crude product was purified by HPLC [eluent A: H₂O (0.1% TFA); and eluent B: ACN, 10% B (at 2 min) to 60% B (at 20 min)], to obtain FAPI-CB-44 (7.4 mg, 81%) as a white solid. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1219, and retention time: 1.43 minutes.

### Example 1d: Synthesis of FAPI-CB-45

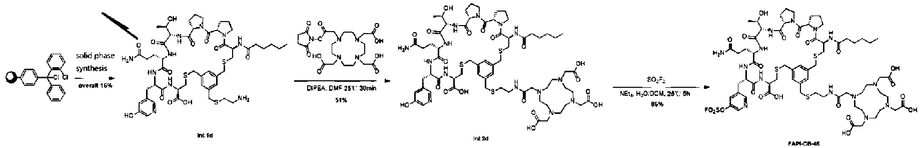

Compound int 1d was prepared via solid-phase synthesis with reference to the steps described in Patent WO 2021/005125 A1 filed by 3B PHARMACEUTICALS GMBH.

DIEA (112.8 mg, 872.7 mmol, 152.0 µL, 5.0 eq.) in one portion was added to a mixture of compound int 1d (200 mg, 174.53 mmol, 1.0 eq.) and DOTA-NHS (131.3 mg, 261.80 mmol, 1.5 eq.) in DMF (5 mL) at 25°C, and then the mixture was stirred at 25°C for 0.5 hours. The main peak was confirmed by LCMS, where [M+H]⁺ = 1486.6. The mixture was purified by preparative HPLC (TFA conditions: A: 0.075% TFA in H₂O; and B: ACN), to obtain int 2d (143.3 mg, 119.22 µmol, yield: 51%).

The int 2d (14.9 mg, 10.0 µmol) was dissolved in water (500 µL), DCM (500 µL) was added, then the gas in the container was replaced with SO₂F₂ gas, 4.0 equivalents of triethylamine were added, and the reaction solution was allowed to react for 6 hours. The reaction solution was concentrated under reduced pressure to remove the solvent. The crude product was purified by HPLC [eluent A: H₂O (0.1% TFA); and eluent B: ACN, 10% B (at 2 min) to 60% B (at 20 min)], to obtain FAPI-CB-45 (13.5 mg, 86%) as a white solid. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1569, and retention time: 1.71 minutes.

### Example 1e: Synthesis of FAPI-CB-22

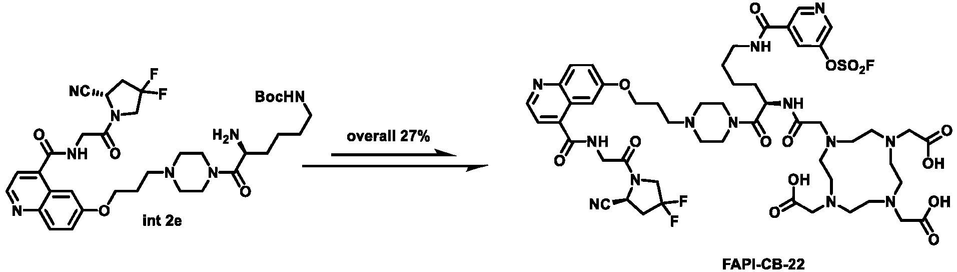

Compound FAPI-CB-22 (10.5 mg, multi-step yield: 27%) was prepared from the readily available intermediate int 2e via the method and route in Example 1a, and purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H₂O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1204, and retention time: 1.65 minutes.

### Example 1f: Synthesis of FAPI-CB-28

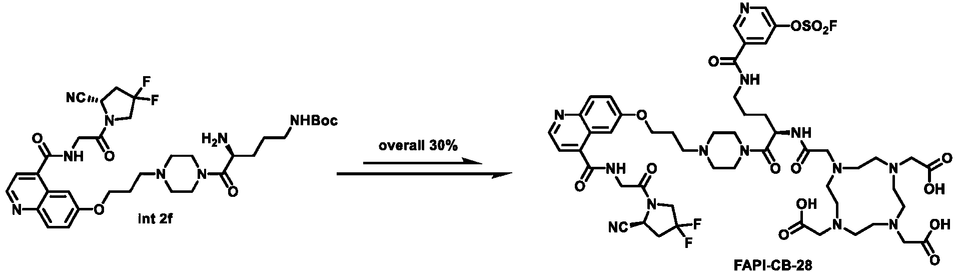

Compound FAPI-CB-28 (9.7 mg, multi-step yield: 30%) was prepared from the readily available intermediate int 2f via the method and route in Example 1a, and purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H₂O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1191, and retention time: 1.62 minutes.

### Example 1g: Synthesis of FAPI-CB-47

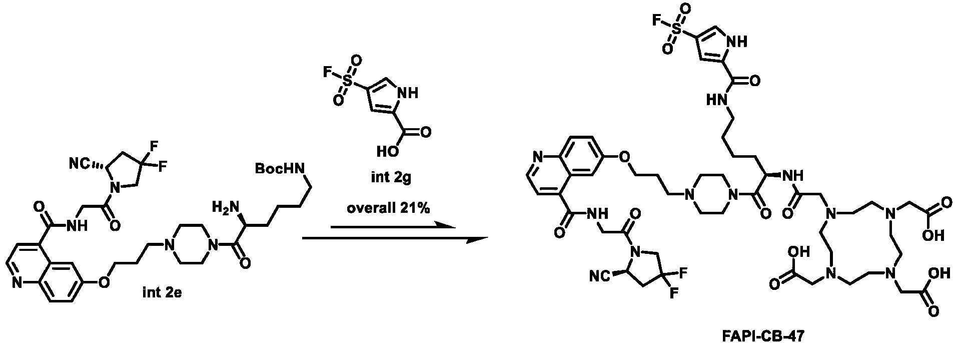

Compound FAPI-CB-47 was prepared from the readily available intermediates int 2e and int 2g via the method and route in Example 1a. The int 2g (8.0 mg, multi-step yield: 21%) was easily obtained with reference to the method in "A New Portal to SuFEx Click Chemistry: A Stable Fluorosulfuryl Imidazolium Salt Emerging as an 'F-SO2+' Donor of Unprecedented Reactivity, Selectivity, and Scope" (Taijie Guo et al.), and "Profiling Sulfur(VI) Fluorides as Reactive Functionalities for Chemical Biology Tools and Expansion of the Ligandable Proteome" (Katharine E. Gilbert et al., ACS Chem. Biol. 2023, 18, 2, 285).

The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1177, and retention time: 1.54 minutes.

### Example 1h: Synthesis of FAPI-CB-50

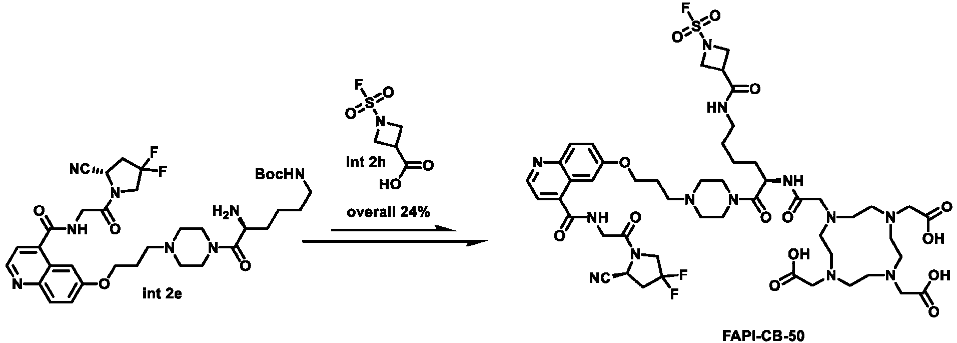

Compound FAPI-CB-50 was prepared from the readily available intermediates int 2e and int 2h via the method and route in Example 1a. The int 2h (8.5 mg, multi-step yield: 24%) was easily obtained with reference to the method in "A New Portal to SuFEx Click Chemistry: A Stable Fluorosulfuryl Imidazolium Salt Emerging as an 'F-SO2+' Donor of Unprecedented Reactivity, Selectivity, and Scope" (Taijie Guo et al.).

The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1167, and retention time: 1.43 minutes.

### Example 1i: Synthesis of FAPI-CB-36

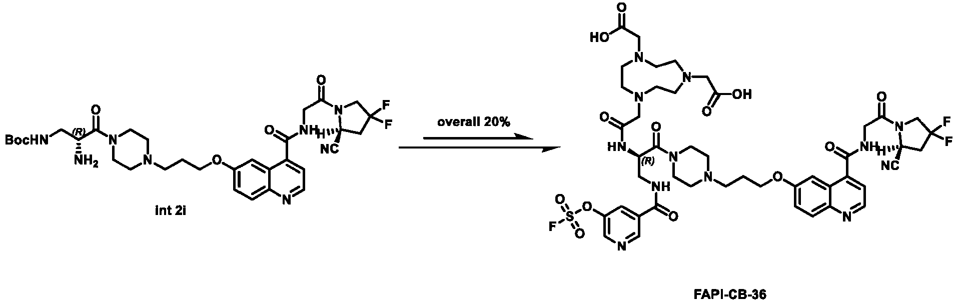

Compound FAPI-CB-36 (6.2 mg, multi-step yield: 20%) was easily prepared from the readily available intermediates int 2i via the method and route similar to those in Example 1a.

The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1061, and retention time: 1.67 minutes.

### Example 1j: Synthesis of FAPI-CB-53

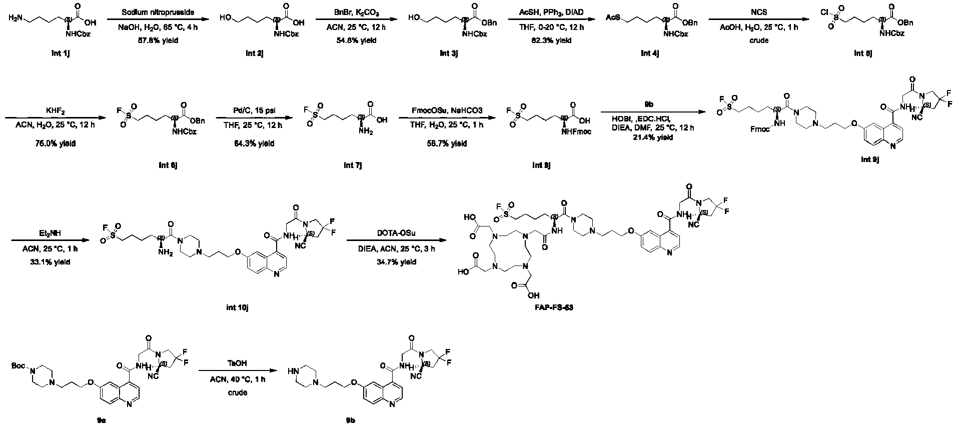

Compound int 1j (5.00g, 17.8 mmol, 1.00 eq.) was dissolved in H₂O (65.0 mL), and pH was adjusted to 9-10 with NaOH (4 mol/L, 50 mL, 11.2 eq.). The mixture was heated to 65°C in an oil bath. Sodium nitroprusside (9.57g, 32.1 mmol, 5.56 mL, 1.80 eq.) was added portionwise within 1 hour, while the pH value of the reaction mixture was maintained between 9-10 by using NaOH (4.00 mol/L). The resulting mixture was further heated for 3 hours, and an aqueous solution of NaOH (4.00 mol/L) was added from time to time to maintain the pH between 9 and 10. The mixture was filtered and extracted with ethyl acetate (250 mL) three times. The combined organic layers were rinsed with brine (100 mL), dried over Na₂SO₄, filtered, and concentrated in vacuo. The crude product was purified by preparative HPLC (0.1% TFA conditions) to obtain compound int 2j (2.90g, 10.3 mmol, yield: 57.8%) as a yellow oil.

Compound int 2j (10.3 mmol, 1.00 eq) was dissolved in ACN. K₂CO₃ (30.9 mmol, 3.00 eq) and BnBr (15.4 mmol, 1.50 eq) were added, and the reaction mixture was stirred at 25°C for 12 hours. The reaction mixture was diluted with water and extracted with ethyl acetate. The combined organic layers were washed with brine, dried over Na₂SO₄, filtered, and concentrated under reduced pressure to obtain a residue. The residue was purified by silica gel column chromatography. Compound int 3j (2.10g, 5.65 mmol, yield: 54.8%) was obtained.

DIAD (1.43g, 7.07 mmol, 1.37 mL, 1.25 eq) was dissolved in THF (25.0 mL). Then PPh₃ (1.85g, 7.07 mmol, 1.25 eq) was added at 0°C, and the mixture was stirred for 1 hour. Then compound 3j (2.10g, 5.65 mmol, 1.00 eq) and AcSH (537 mg, 7.07 mmol, 505 µL, 1.25 eq) were added at 0°C. The mixture was stirred for 11 hours at 20°C. The mixture was concentrated under vacuum. The crude product was purified by silica gel column chromatography (petroleum ether: ethyl acetate = 5: 1-0: 1, petroleum ether: ethyl acetate = 5: 1, Rf = 0.15). Compound int 4j (2.00g, 4.66 mmol, yield: 82.3%) was obtained as a colorless oil.

Compound int 4j (1.50g, 3.49 mmol, 1.00 eq) was dissolved in glacial acetic acid (50.0 mL) and water (5.0 mL), and NCS (1.40g, 10.4 mmol, 3.00 eq) was added. The reaction mixture was stirred for 1 hour at 25°C. The reaction mixture was added into water (50 mL) and extracted with dichloromethane (100 mL). The organic layer was washed three times with water (100 mL) and brine (100 mL), dried over Na₂SO₄, and concentrated to obtain compound int 5j (1.50g, crude product) as a colorless oil.

Compound int 5j (1.50g, 3.30 mmol, 1.00 eq) was dissolved in acetonitrile (42.0 mL) and water (21.0 mL), and KHF₂ (1.03g, 13.2 mmol, 435 µL, 4.00 eq) was added. The mixture was stirred for 12 hours at 25°C. The mixture was extracted with ethyl acetate (150 mL) three times. The combined organic layers were concentrated to obtain a residue. The crude product was purified by preparative HPLC. Compound int 6j (1.10g, 2.51 mmol, yield: 76.0%) was obtained as a colorless oil.

Compound int 6j (1.10g, 2.51 mmol, 1.00 eq) was dissolved in THF (20.0 mL), and Pd/C (220 mg, 206 µmol, 10% purity, 0.2 eq) was added under a nitrogen atmosphere. The suspension was degassed and purged with H₂ three times. The mixture was stirred at 25°C for 12 hours under H₂ (15 psi). The reaction mixture was filtered and concentrated to obtain compound int 7j (1.10g, 2.51 mmol, yield: 76.0%) as a white solid.

Compound int 7j (100 mg, 468 µmol, 1.00 eq) was dissolved in THF (2.0 mL) and water (2.0 mL). NaHCO₃ (39.4 mg, 468 µmol, 18.2 µL, 1.00 eq) and FmocOSu (158 mg, 468 µmol, 1.00 eq) were added, and the mixture was stirred at 25°C for 1 hour. The pH of the mixture was adjusted to 5, the mixture was extracted with ethyl acetate (100 mL), and the organic layer was concentrated. The crude product was purified by preparative HPLC. Compound int 8j (120 mg, 275 µmol, yield: 58.7%) was obtained as a white solid.

P-toluenesulfonic acid (133 mg, 775 µmol, 3.50 eq) was added to a solution containing compound 9a (130 mg, 221.61 µmol, 1.00 eq) and acetonitrile (10.0 mL). The mixture was stirred for 1 hour at 40°C. The mixture was concentrated under vacuum to obtain compound 9b (100 mg, crude product) as a yellow solid.

Compound int 8j (90.0 mg, 206 µmol, 1.00 eq), DIEA (53.4 mg, 413 µmol, 72.0 µL, 2.00 eq), HOBt (41.8 mg, 310 µmol, 1.50 eq), and EDCI (41.6 mg, 217 µmol, 1.05 eq) were added sequentially to a stirred solution containing compound 9b (95.5 mg, 196 µmol, 0.95 eq) and DMF (5.0 mL). Then the reaction mixture was stirred at 25°C for 12 hours. The reaction mixture was concentrated under vacuum, and the crude product was purified by Preparative High Performance Liquid Chromatography (under 0.1% TFA condition) to obtain compound int 9j (40.0 mg, 44.2 µmol, yield: 21.4%) as a white solid.

Diethylamine (2.84g, 38.8 mmol, 4.00 mL, 877 eq) was added to a solution containing compound int 9j (40.0 mg, 44.2 µmol, 1.00 eq) and acetonitrile (16.0 mL), and the mixture was stirred at 25°C for 1 hour. The pH of the mixture was adjusted to 5 with citric acid (0.5 mL), the mixture was extracted with ethyl acetate (10.0 mL), and the aqueous layer was lyophilized to obtain the crude product. The crude product was purified by Preparative High Performance Liquid Chromatography (under 0.1% TFA condition) to obtain compound int 10j (10.0 mg, 14.6 µmol, yield: 33.1%) as a colorless oil.

DIEA (4.74 mg, 36.6 µmol, 6.39 µL, 2.50 eq) was added to an acetonitrile (1.0 mL) solution containing compound int 10j (10.0 mg, 14.6 µmol, 1.00 eq) and DOTA-OSu (10.0 mg, 19.9 µmol, 1.36 eq), and the mixture was stirred at 25°C for 3 hours. The mixture was concentrated under a nitrogen atmosphere, and the crude product was purified by Preparative High Performance Liquid Chromatography (under 0.1% NH₄HCO₃ condition) to obtain FAPI-FS-53 (6.0 mg, 5.09 µmol, yield: 34.7%, and purity: 98.6%) as a white solid. [Eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1069, and retention time: 1.53 minutes.

### Example 1k: Synthesis of FAPI-CB-59

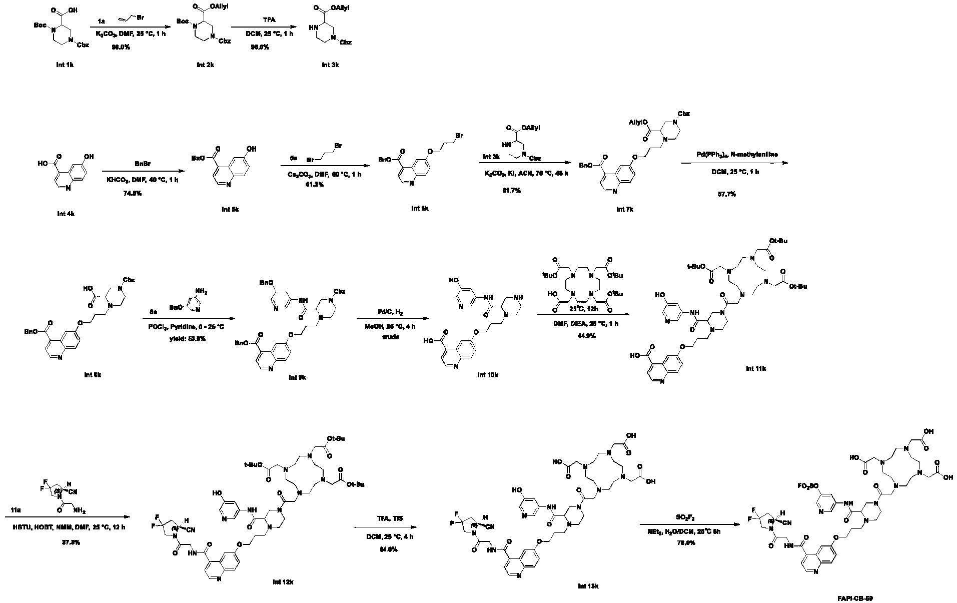

Compound int 1k (5.30g, 14.5 mmol, 1.00 eq) and compound 1a (1.94g, 16.0 mmol, 1.10 eq) were dissolved in DMF (50.0 mL), and K₂CO₃ (2.01g, 14.5 mmol, 1.00 eq) was added. The mixture was stirred for 1 hour at 25°C. The reaction mixture was diluted with ethyl acetate (200 mL). The combined organic layers were washed three times with water (100 mL), dried over Na₂SO₄, filtered, and concentrated under reduced pressure to obtain compound int 2k (5.80g, 14.3 mmol, yield: 98.0%, purity: 99.4%) as a yellow oil.

Compound int 2k (5.80g, 14.3 mmol, 1.00 eq) was dissolved in DCM (10.0 mL), and TFA (35.4g, 310 mmol, 23.1 mL, 21.8 eq) was added. The mixture was stirred for 1 hour at 25°C. The reaction mixture was concentrated under reduced pressure to obtain a residue. The residue was diluted with DCM (100 mL), and the pH of the organic layer was adjusted to 7 with a saturated aqueous solution of sodium bicarbonate. The mixture was dried over Na₂SO₄, filtered, and concentrated under reduced pressure to obtain compound int 3k (4.00g, 13.1 mmol, yield: 92.2%) as a yellow oil.

Compound int 4k (4.00g, 21.2 mmol, 1.00 eq) and BnBr (3.62g, 21.2 mmol, 2.51 mL, 1.00 eq) were dissolved in DMF (200 mL), and KHCO₃ (3.18g, 31.7 mmol, 1.50 eq) was added. The mixture was stirred for 1 hour at 40°C. The reaction mixture was filtered, and the filtrate was concentrated under reduced pressure to obtain a residue. The filtrate was diluted with ethyl acetate (500 mL), and the organic phase was washed three times with water (200 mL), then dried over Na₂SO₄, filtered, and concentrated under reduced pressure to obtain compound int 5k (4.60g, 15.8 mmol, yield: 74.8%, purity: 96.0%) as a brown solid.

Compound int 5k (4.60g, 15.8 mmol, 1.00 eq) and compound 5a (9.58g, 47.4 mmol, 4.84 mL, 3.00 eq) were dissolved in DMF (50.0 mL), and Cs₂CO₃ (10.3g, 31.6 mmol, 2.00 eq) was added. The mixture was stirred for 1 hour at 60°C. The reaction mixture was filtered, and the filtrate was diluted with ethyl acetate (200 mL). The organic layer was washed three times with water (100 mL), dried over Na₂SO₄, filtered, and concentrated under reduced pressure to obtain a residue. The residue was purified by preparative HPLC to obtain compound int 6k (4.00g, 9.68 mmol, yield: 61.2%, purity: 96.9%) as a red oil.

Compound int 6k (1.14g, 3.75 mmol, 1.50 eq) was dissolved in ACN (50.0 mL), and K₂CO₃ (691 mg, 5.00 mmol, 2.00 eq) and KI (207 mg, 1.25 mmol, 0.50 eq) were added. The mixture was stirred for 48 hours at 70°C. Two batches of the reaction mixture were processed together. The reaction mixture was filtered, and the filtrate was concentrated under reduced pressure to obtain a residue. The residue was purified by preparative HPLC to obtain compound int 7k (2.00g, 3.08 mmol, yield: 61.7%, purity: 96.1%) as a red oil.

Compound int 7k (1.80g, 2.77 mmol, 1.00 eq) was dissolved in DMF (2.00 mL), Pd(PPh₃)₄ (480 mg, 415 µmol, 0.15 eq) and N-methylaniline (594 mg, 5.54 mmol, 602 µL, 2.00 eq) were added. The mixture was stirred for 1 hour at 25°C under N₂ protection. The reaction mixture was concentrated under reduced pressure to obtain a residue. The residue was purified by preparative HPLC to obtain compound int 8k (1.00g, 1.60 mmol, yield: 57.70%, purity: 93.3%) as a yellow solid.

Compound int 8k (750 mg, 1.20 mmol, 1.00 eq) and Compound 8a (240.16 mg, 1.20 mmol, 1 eq) were dissolved in DCM (20.0 mL). Pyridine (474 mg, 6.00 mmol, 484 µL, 5.00 eq) and POCl₃ (221 mg, 1.44 mmol, 134 µL, 1.20 eq) were added at 0°C. The mixture was stirred for 1 hour at 25°C. The organic layer was washed three times with water (10.0 mL), dried over Na₂SO₄, filtered, and concentrated under reduced pressure to obtain a residue. The residue was purified by preparative HPLC to obtain compound int 9k (500 mg, 646 µmol, yield: 53.8%, purity: 98.9%) as a yellow oil.

Compound int 9k (200 mg, 258 µmol, 1.00 eq) was dissolved in methanol (2.00 mL), and Pd/C (55.0 mg, 51.7 µmol, purity: 10%, 0.20 eq) was added under N₂ protection. The suspension was degassed under vacuum and purged with H₂ several times. The mixture was stirred at 25°C under H₂ (15 psi) for 3 hours. The reaction mixture was filtered, and the filtrate was concentrated under reduced pressure to obtain compound int 10k (50.0 mg, crude product) as a colorless oil.

50.0 mg (111 µmol, 1.00 eq) of compound int 10k and DOTA-OSu (74.2 mg, 111 µmol, 1.00 eq) were dissolved in DMF (1.00 mL), and then DIEA (14.3 mg, 1115 µmol, 19.3 µL, 1.00 eq) was added. The mixture was stirred for 1 hour at 25°C. The reaction mixture was added to stirred MTBE (100 mL), and then centrifuged to obtain compound int 11k (50.0 mg, 49.69 µmol, yield: 44.9%, purity: 100%) as a yellow oil.

Compound int 11k (50.0 mg, 49.7 µmol, 1.00 eq) was dissolved in DMF (2.00 mL), and then HBTU (28.3 mg, 74.5 µmol, 1.50 eq), HOBT (10.1 mg, 74.5 µmol, 1.50 eq), TEA (25.1 mg, 248 µmol, 34.6 µL, 5.00 eq) and compound 11a (47.0 mg, 74.5 µmol, 1.50 eq) were added. The mixture was stirred for 12 hours at 25°C. The reaction mixture was purified by preparative HPLC (Column: Phenomenex Luna C18 150×25 mm×10 µm; mobile phase: [Water (FA) - Acetonitrile]; gradient: 14% - 44% B; 10 minutes), to obtain compound int 12k (25.0 mg, 18.5 µmol, yield: 37.3%, purity: 87.3%) as a white solid.

20.0 mg (14.8 µmol, 1.00 eq) of compound int 12k was dissolved in DCM (5.00 mL), and then TFA (6.14g, 53.9 mmol, 4.00 mL, 3631 eq) and TIS (9.39 mg, 59.3 µmol, 12.2 µL, 4.00 eq) were added. The mixture was stirred for 4 hours at 25°C. The reaction mixture was filtered and concentrated under reduced pressure to obtain a residue. The residue was purified by prep-HPLC (TFA conditions) to obtain compound int 13k (14.2 mg, 9.49 µmol, yield: 64.0%, purity: 99.4%, 4TFA) as a white solid.

Compound FAPI-CB-59 (11.8 mg, yield: 78%, purity: 99.5%) was prepared from 14.0 mg of compound int 13k via the method and route similar to those in Example 1d. The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1092, and retention time: 1.76 minutes.

### Example 1l: Synthesis of FAPI-CB-77

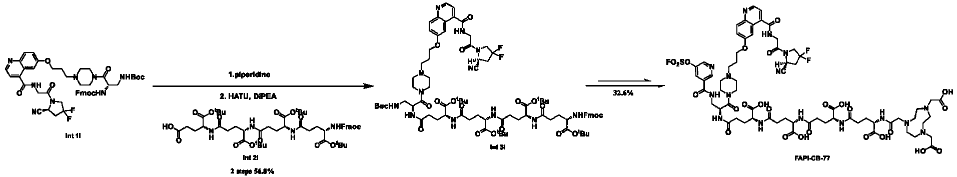

Compound int 2l is easily prepared via solid-phase peptide synthesis. Compound FAPI-CB-77 (5.5 mg, yield: 32.6%, purity: 98.2%) was prepared from compound int 3l via HPLC purification [eluent A: H₂O (0.1% TFA); eluent B: ACN, 10% B (at 2 min) to 60% B (at 20 min)] in the method and route similar to those in Example 1a. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1577, and retention time: 1.71 minutes.

### Example 1m: Synthesis of FAPI-CB-86

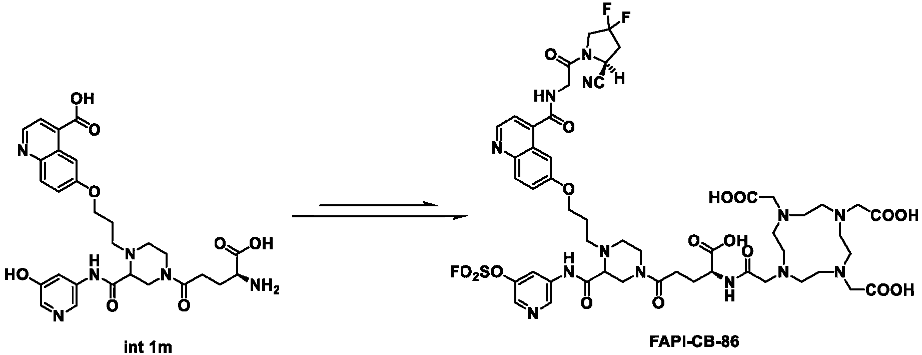

Compound FAPI-CB-86 (3.5 mg, yield: 12.0%, purity: 97.2%) was prepared from compound int 1m via the method and route similar to those in Example 1k. The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A.

The calculated m/z value is [M+H]⁺: 1221, and retention time: 1.90 minutes.

### Example 1n: Synthesis of FAPI-CB-93

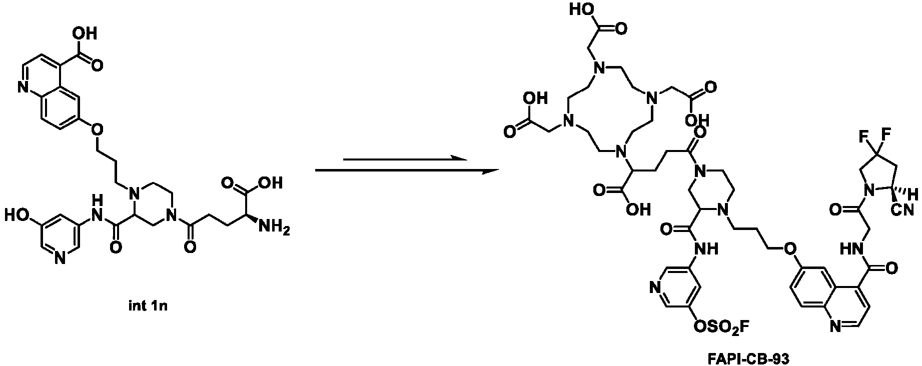

Compound FAPI-CB-93 (3.5 mg, yield: 12.0%, purity: 97.2%) was prepared from int 1n via the method and route similar to those in Example 1k. The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1164, and retention time: 1.85 minutes.

### Example 1o: Synthesis of FAPI-CB-94

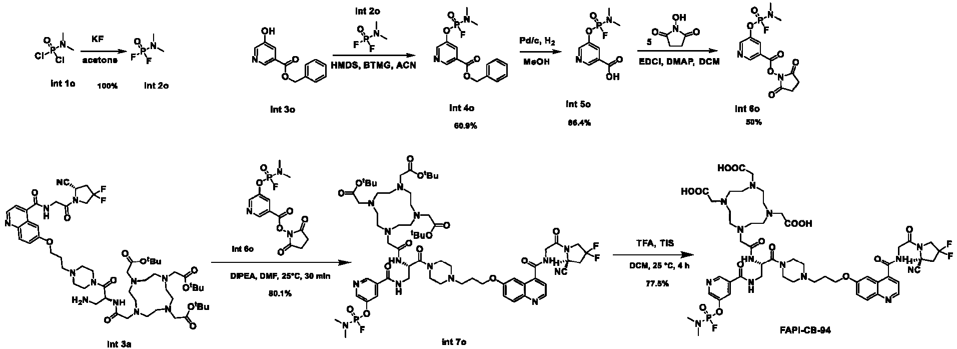

Compound int 5o was prepared with reference to the steps described in "Phosphorus Fluoride Exchange: Multidimensional Catalytic Click Chemistry from Phosphorus Connective Hubs" (Sun, S. et al., Chem 2023, 9, 2128-2143).

Compound int 1o (6.0g, 37.0 mmol) was dissolved in acetone (150 mL) and the resulting mixture was stirred. KF (17.1g, 296 mmol) was added to the solution. The mixture was stirred at room temperature for 3 hours and then filtered through Celite. The solvent was evaporated under reduced pressure to obtain a sufficient amount of phosphoramide difluoride int 2o in a single yield.

The reaction mixture of int 2o and commercially available int 3o was stirred at room temperature for 1 hour. Then 200 mL of ethyl acetate was added to dilute the reaction mixture, and the organic phase was washed with water (200 mL) and brine (200 mL) sequentially. The organic phase was dried over anhydrous sodium sulfate, and evaporated under reduced pressure to obtain a crude product, and then the crude product was purified by column chromatography (silica gel, Hexane: EA = 4:1) to obtain the colorless oily product int 3o (4.6g, yield: 60.9%).

100 mg of 10% Pd/C was added to a 50 mL round-bottom flask, and then 5 mL of methanol was added under an inert atmosphere (argon). Compound int 4o (1.0g, 3.0 mmol) was dissolved in methanol (5 mL), the resulting mixture was added to the flask, and the flask was sealed with a rubber stopper. The round-bottom flask was evacuated to dryness and then filled with hydrogen gas by using a hydrogen balloon. The mixture was vigorously stirred under hydrogen gas for 1 hour and then filtered through Celite. The solvent was removed under reduced pressure to obtain compound int 5o (627 mg, 84.6%) as a white solid.

Compound int 5o (120 mg, 0.81 mmol, 1 eq), compound 5 (115 mg, 0.97 mmol, 1.2 eq) and EDCI (191 mg, 0.97 mmol, 1.2 eq) were dissolved in DCM (5 mL) and the mixture was stirred at room temperature for 1 hour. LCMS analysis indicated the reaction was incomplete, and then DMAP (9 mg, 0.08 mmol, 0.1 eq) was added. The mixture was further stirred for 2 hours, and LCMS indicated the reaction was complete. Petroleum ether was added for recrystallization, to obtain compound int 6o (140 mg, yield: 50%) as a white solid.

Compound int 7o (yield: 80.1%) was prepared from int 3a via the method and route similar to those in Example 1d. Compound FAPI-CB-94 (3.5 mg, yield: 77.6%, purity: 98.2%) was prepared from int 7o via HPLC purification [eluent A: H₂O (0.1% TFA); eluent B: ACN, 10% B (at 2 min) to 60% B (at 20 min)] in the method and route similar to those in Example 1a. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1190, and retention time: 1.53 minutes.

### Example 1p: Synthesis of FAPI-CB-95

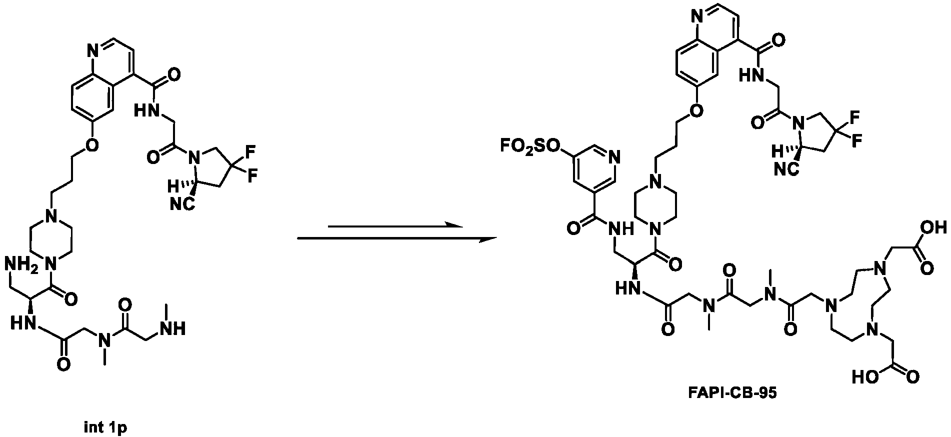

Compound FAPI-CB-95 (3.6 mg, yield: 11.9%, purity: 98.5%) was prepared from intermediate compound int 1p easily obtainable through solid-phase peptide synthesis via the method and route similar to those in Example 1a.

The crude product was purified by HPLC [eluent A: H₂O (0.1% TFA); and eluent B: ACN, 10% B (at 2 min) to 60% B (at 20 min)]. UPLC-MS characterization conditions: Column: ACQUITY UPLC BEH C18 1.7 µm, 2.1×50 mm. Method: Flow rate: 0.5 mL/min; eluent A: H2O (0.1% TFA); eluent B: MeOH; 0-0.2 min, 95% A; 0.2-3.0 min, 95% to 5% A, curve 6; 3.0-4.0 min, 5% A; 4.0-4.5 min, 5% to 95% A, and curve 6; 4.5-5.0 min, 95% A. The calculated m/z value is [M+H]⁺: 1203, and retention time: 1.61 minutes.

### Example 2: Preparation of Nuclear pharmaceutical molecules from Radionuclide-Labeled Precursor Compound

Generally, the radiolabeling of ⁶⁸Ga was performed after 10 minutes of incubation with 10 nmol of FAPI-CB series precursors at a pH of 4.0 and 90°C. The product was purified via a C₁₈ column (Waters). Radiochemical purity of the product was measured by high performance liquid chromatography with an equipped radioactivity detector (radio-HPLC). In addition, stability of the product was tested at 37°C in phosphate buffer (0.01 M, pH 7.4) and human serum (proteins needed to be removed from the human serum via acetonitrile precipitation).

Generally, the radiolabeling of ¹⁷⁷Lu was performed after 10 minutes of incubation with several FAPI-CB series precursors at a pH of 4.5 and 50°C to 90°C. The product was purified via a C₁₈ column (Waters). Radiochemical purity of the product was measured by high performance liquid chromatography with an equipped radioactivity detector (radio-HPLC). In addition, the stability of the product was tested at 37°C in phosphate buffer (0.01 M, pH 7.4) and human serum (proteins needed to be removed from the human serum via acetonitrile precipitation).

Generally, the radiolabeling of Al¹⁸F was as follows: A commercially available aqueous solution of ¹⁸F-containing anions prepared via an accelerator was concentrated to 10 µL by using a nitrogen evaporator. Then 2 µL of 0.2 M sodium acetate solution containing 6 mM Al³⁺ (pH = 4.0, 12 nmol), 30 µL of acetonitrile, and 20 µL of 0.2 M sodium acetate buffer (pH = 4.0) were added, and the resulting mixture was heated at 100°C for 5 minutes to prepare [Al¹⁸F]²⁺. Then 20 nmol of the corresponding FAPI-CB series precursor molecules were added, and the mixture was incubated at 100°C for 15 minutes. The product was purified via a C₁₈ column (Waters). Radiochemical purity of the product was measured by high performance liquid chromatography with an equipped radioactivity detector (radio-HPLC). In addition, the stability of the product was tested at 37°C in phosphate buffer (0.01 M, pH 7.4) and human serum (proteins needed to be removed from the human serum via acetonitrile precipitation).

Radio-HPLC characterization conditions: Column: XBridge^{®} C₁₈ 5 µm, 4.6×150 mm; eluent A: H₂O (0.1% trifluoroacetic acid); eluent B: acetonitrile (0.1% trifluoroacetic acid); 0-2 min, 90% A phase; 2-10 min, 90% to 40% A phase, linear gradient mode 6; 10-12 min, 40% A phase; 12-13 min, 40% to 90% A phase, linear gradient mode 6; 13-15 min, 90% A phase; retention time: 7.4 min for the product (purity ≈ 98.6%); and 6.1 min for the hydrolytic by-product (about 1.4%).

FAPI-CB-22, FAPI-CB-28, FAPI-CB-30, FAPI-CB-31, FAPI-CB-44, FAPI-CB-45, FAPI-CB-47, FAPI-CB-50, FAPI-CB-53, FAPI-CB-59, FAPI-CB-77, FAPI-CB-86, FAPI-CB-93, and FAPI-CB-94 labeled with ⁶⁸Ga, ¹⁷⁷Lu, ⁸⁶Y, and ²²⁵Ac, as well as FAPI-CB-36 and FAPI-CB-95 labeled with Al¹⁸F, can be easily obtained via methods similar to those described above and disclosed existing technology.

FIG. 2 shows the radio-purity chromatogram of ⁶⁸Ga-FAPI-CB-30, in which the left panel represents the quality control chromatogram after labeling, the middle panel shows the stability monitoring after 1 hour of co-incubation with serum, and the right panel shows the stability monitoring after 2 hours of co-incubation with serum.

FIG. 3 shows radioactive HPLC chromatograms of ⁶⁸Ga-FAPI-CB-31, ⁶⁸Ga-FAPI-CB-50, and Al¹⁸F-FAPI-CB-36, respectively, in the left, middle, and right panels.

### Example 3: Molecular Experiment: Target Protein Co-Incubation, Acid-Base Treatment, Gel Electrophoresis, Autoradiography, and Coomassie Brilliant Blue Staining Experiment

A total volume of 20 µL of ¹⁷⁷Lu-FAPI-CB-30 with a precursor molecule concentration of 200 nM was incubated with 4 µM human recombinant FAP protein at pH 7.4 (pH of serum) or pH 6.5 (pH of tumor microenvironment) and 37°C for 1 hour. Then the samples were subjected to a relatively strong acid or alkali denaturation treatment to eliminate reversible interactions. Then SDS-PAGE was performed to isolate the proteins from the nuclear pharmaceutical molecules on polyacrylamide gel. Then the gel was taken for phosphor screen autoradiography (Amersham Typhoon imaging) to obtain radioactive bands of the nuclear pharmaceutical. Finally, the gel was stained with Coomassie brilliant blue, and the target protein appeared blue. The radioactive bands were compared with staining results of Coomassie brilliant blue. If the nuclear pharmaceutical formed irreversible covalent bonds with the protein, radioactive bands could be observed in the protein chromogenic region at the upper part of the gel.

FIG. 4 shows covalent binding efficiency between ¹⁷⁷Lu-FAPI-CB-30 and human recombinant FAP protein obtained after protein co-incubation, gel electrophoresis, autoradiography, and Coomassie blue staining. The left panel shows a control group incubated at pH 7.4 for 1 hour, and the right panel shows a sample treated with a strong acid after 1 hour of incubation, indicating that there are still covalent bonds.

### Example 4: Molecular Experiment: Long-Duration Co-Incubation of Target Protein, Gel Electrophoresis, Autoradiography, and Coomassie Brilliant Blue Staining Experiment

¹⁷⁷Lu-FAPI-CB-30 with a precursor molecule concentration of 150 nM was incubated with 3 µM human recombinant FAP protein at pH 7.4 and 37°C. Then samples of the same volume were taken at different time points, mixed with a protein loading buffer, and stored in a refrigerator at -80°C. All samples were collectively subjected to denaturation (at 95°C for 5 minutes), and then SDS-PAGE was performed to isolate proteins from nuclear pharmaceutical molecules on polyacrylamide gel. Then the gel was taken for phosphor screen autoradiography (Amersham Typhoon imaging) to obtain radioactive bands of the nuclear pharmaceutical. Finally, the gel was stained with Coomassie brilliant blue, and the target protein appeared blue. The radioactive bands were compared with staining results of Coomassie brilliant blue. If the nuclear pharmaceutical formed irreversible covalent bonds with the protein, radioactive bands could be observed in the protein chromogenic region at the upper part of the gel.

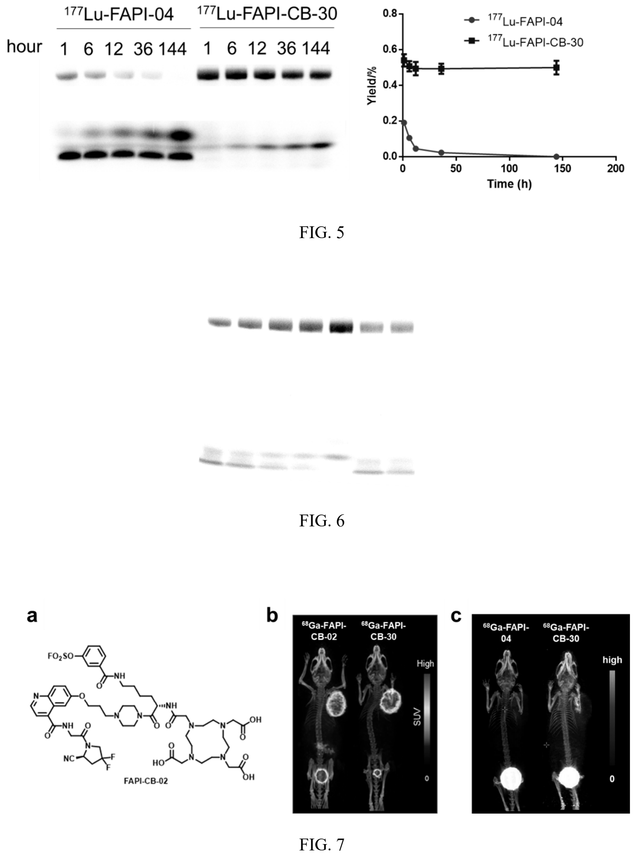

FIG. 5 shows the covalent binding ratio between ¹⁷⁷Lu-FAPI-CB-30 molecules and human recombinant FAP protein obtained after protein co-incubation, gel electrophoresis, autoradiography, and Coomassie blue staining. The left panel is the autoradiogram, and the right panel shows the quantified band gray value obtained using image analysis tools such as ImageJ, corresponding to the proportion of covalently bound molecules.

### Example 5: Molecular Experiment: Long-Duration Co-Incubation of Target Protein, Gel Electrophoresis, Autoradiography, and Coomassie Brilliant Blue Staining Experiment

¹⁷⁷Lu-FAPI-CB series molecules with a precursor molecule concentration of 150 nM was incubated with 3 µM human recombinant FAP protein at pH 7.4 and 37°C. Then samples of the same volume were taken at specific time points, mixed with a protein loading buffer, and stored in a refrigerator at -80°C. All samples were collectively subjected to denaturation (at 95°C for 5 minutes), and then SDS-PAGE was performed to isolate proteins from nuclear pharmaceutical molecules on polyacrylamide gel. Then the gel was taken for phosphor screen autoradiography (Amersham Typhoon imaging) to obtain radioactive bands of the nuclear pharmaceutical. Finally, the gel was stained with Coomassie brilliant blue, and the target protein appeared blue. The radioactive bands were compared with staining results of Coomassie brilliant blue. If the nuclear pharmaceutical formed irreversible covalent bonds with the protein, radioactive bands could be observed in the protein chromogenic region at the upper part of the gel.

FIG. 6 shows the covalent binding ratios between molecules of ¹⁷⁷Lu-FAPI-CB series and human recombinant FAP protein obtained after protein co-incubation, gel electrophoresis, autoradiography, and Coomassie blue staining, where the lanes in the figure from left to right are ¹⁷⁷Lu-labeled FAPI-CB-22, FAPI-CB-28, FAPI-CB-31, FAPI-CB-44, FAPI-CB-45, FAPI-CB-47, and FAPI-CB-50, respectively.

### Example 6: Animal Experiment: PET/CT Imaging and Biodistribution

All PET/CT scans were performed on Mediso nanoScan^{®} PET 122S for small-animal PET/CT.

A specific number of HT-1080-FAP tumor-bearing mice were first given tail vein injections of 7.4 MBq to 18.5 MBq of ⁶⁸Ga/⁸⁶Y-FAPI-04 (control group) and radionuclide-labeled FAPI-CB series molecules at specified time points. Then imaging was performed at designated time points. At a specific time point, the mice were anesthetized, then sacrificed by cervical dislocation, and dissected to take organs for radioactivity measurement. Uptake and retention of the nuclear pharmaceuticals at the tumors were comprehensively quantitatively analyzed.

### Example 7a: Comparative PET/CT Imaging of ⁶⁸Ga-FAPI-CB-30 and ⁶⁸Ga-FAPI-CB-02 or ⁶⁸Ga-FAPI-04 in HT-1080-FAP Tumor-Bearing Mouse Model

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 12 µg of FAPI-CB-02 precursor (a structure thereof is shown in FIG. 7a, and FAPI-CB-02 was synthesized by performing basically the same steps as those in Example 1a, with the corresponding reactants replaced) was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-CB-02 was obtained. The labeled ⁶⁸Ga-FAPI-CB-02 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, FAPI-CB-30, as the control, was labeled using the same procedure, and PET imaging was performed on the same mouse at intervals of 24 hours. The obtained image is shown in FIG. 7b. It can be seen that the uptake of the ⁶⁸Ga-FAPI-CB-30 probe is comparable to that of ⁶⁸Ga-FAPI-CB-02 at tumor sites, but has a lower blood pool background.

⁶⁸Ga-FAPI-CB-30 and ⁶⁸Ga-FAPI-04 were compared using the same procedure. As shown in FIG. 7c, it can be seen that tumor uptake of ⁶⁸Ga-FAPI-CB-30 was increased, with no significant elevation in the background.

### Example 7b: Comparative PET/CT Imaging of ⁶⁸Ga-FAPI-CB-31 and ⁶⁸Ga-FAPI-04 in HT-1080-FAP Tumor-Bearing Mouse Model

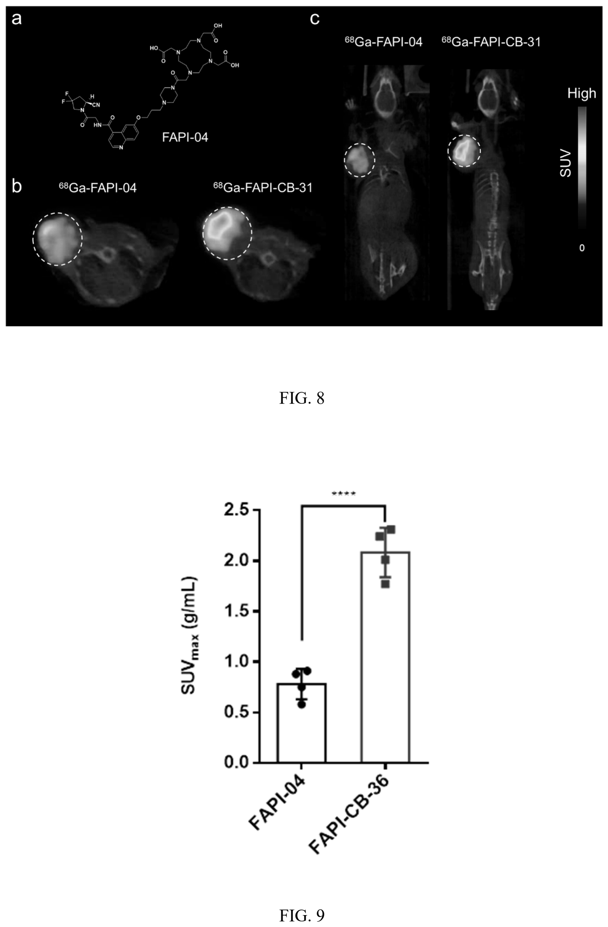

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 9 µg of FAPI-04 precursor was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-04 was obtained. The labeled ⁶⁸Ga-FAPI-04 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, FAPI-CB-31, as the control, was labeled using the same procedure, and PET imaging was performed on the same mouse at intervals of 24 hours. The obtained image is shown in FIG. 8. It can be seen that the uptake of the ⁶⁸Ga-FAPI-CB-31 probe is higher than that of ⁶⁸Ga-FAPI-04 at tumor sites, with a similar blood pool background (the structure of FAPI-04 is shown in FIG. 8a).

### Example 7c: Comparative PET/CT Imaging of Al¹⁸F-FAPI-CB-36 and⁶⁸Ga-FAPI-04 in HT-1080-FAP Tumor-Bearing Mouse Model

The ⁶⁸Ga-FAPI-04 labeled using the method in Example 2 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, Al¹⁸F-FAPI-CB-36, as the control, was labeled via the Al¹⁸F labeling procedure in Example 2, and PET imaging was performed on the same mouse at intervals of 24 hours. The obtained SUVₘₐₓ of the tumor uptake is shown in FIG. 9. It can be seen that the uptake of the Al¹⁸F-FAPI-CB-36 probe was significantly higher than that of ⁶⁸Ga-FAPI-04 at the tumor site, and the average SUVₘₐₓ of tumors in mice was up to 2.08, while the average SUVₘₐₓ of ⁶⁸Ga-FAPI-04 in the same group of mice was only 0.78.

### Example 7d: Comparative PET/CT Imaging of ⁶⁸Ga-FAPI-CB-50 and ⁶⁸Ga-FAPI-04 in HT-1080-FAP Tumor-Bearing Mouse Model

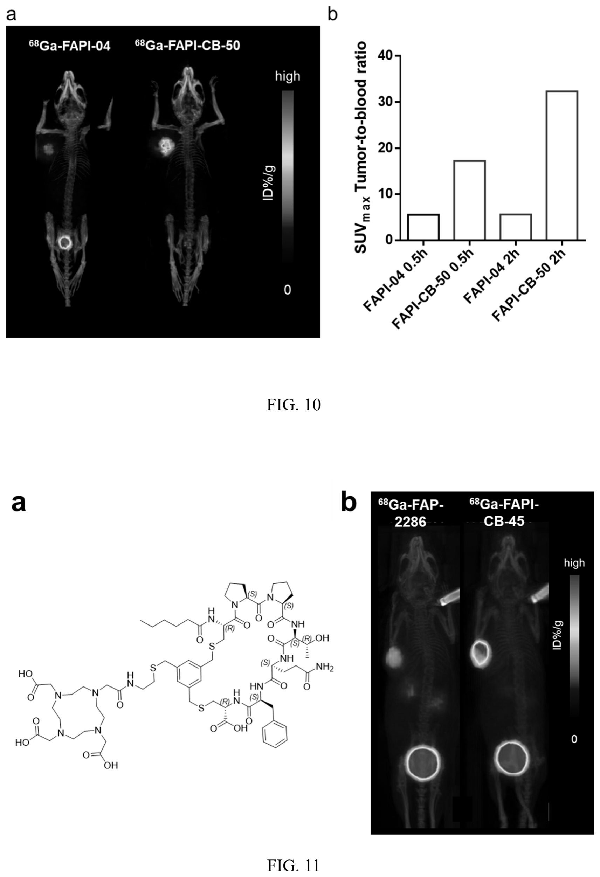

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 9 µg of FAPI-04 precursor was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-04 was obtained. The labeled ⁶⁸Ga-FAPI-04 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, FAPI-CB-50, as the control, was labeled using the same procedure, and PET imaging was performed on the same mouse at intervals of 24 hours. The obtained image at 2 hours post-injection is shown in FIG. 10a. It can be seen that the uptake of the ⁶⁸Ga-FAPI-CB-50 probe is higher than that of ⁶⁸Ga-FAPI-04 at tumor sites, with a lower blood pool background. As shown in FIG. 10b, the ratio of tumor SUVₘₐₓ to blood pool SUVₘₐₓ was examined, and ⁶⁸Ga-FAPI-CB-50 exhibited a better clearance rate.

### Example 7e: Comparative PET/CT Imaging of ⁶⁸Ga-FAP-2286 and ⁶⁸Ga-FAPI-CB-45 in HT-1080-FAP Tumor-Bearing Mouse Model

The ⁶⁸Ga-FAP-2286 (the structure of the precursor is shown in FIG. 11A, which is commercially available or can be easily obtained with reference to Patent WO 2021/005125 A1 filed by 3B PHARMACEUTICALS GMBH) labeled using the method in Example 2 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, ⁶⁸Ga-FAPI-CB-45, as the control, was labeled using the same procedure, and PET imaging was performed on the same mouse at intervals of 24 hours. The obtained image is shown in FIG. 11B. It can be seen that the uptake of the ⁶⁸Ga-FAPI-CB-45 probe is higher than that of ⁶⁸Ga-FAP-2286 at tumor sites, with no significant elevation in the background.

### Example 7f: Comparative PET/CT Imaging of ⁶⁸Ga-FAPI-CB-53 and ⁶⁸Ga-FAPI-04 in HT-1080-FAP Tumor-Bearing Mouse Model

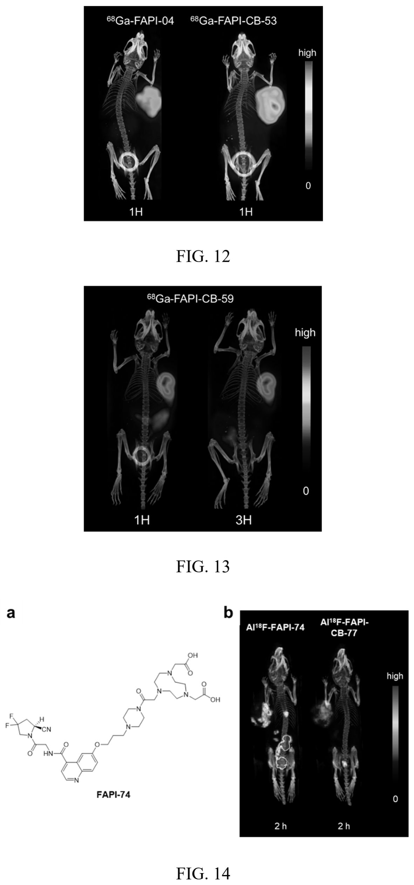

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 9 µg of FAPI-04 precursor was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-04 was obtained. The labeled ⁶⁸Ga-FAPI-04 was diluted with normal saline, and about 3.7 MBq of the diluted solution (at a volume of about 200 µL) was injected into each mouse. At 1 hour post-injection, PET imaging was performed, and PET image processing software was used for reconstruction. In addition, FAPI-CB-53, as the control, was labeled using the same procedure, and PET imaging was performed on the same mouse at intervals of 24 hours. The obtained image at 1 hour post-injection is shown in FIG. 12. It can be seen that the uptake of the ⁶⁸Ga-FAPI-CB-53 probe is higher than that of ⁶⁸Ga-FAPI-04 at tumor sites, with blood pool background maintained at a lower level.

### Example 7g: Comparative PET/CT Imaging of ⁶⁸Ga-FAPI-CB-59 and ⁶⁸Ga-FAPI-04 in HT-1080-FAP Tumor-Bearing Mouse Model

⁶⁸Ga-FAPI-CB-59 was labeled via the procedure similar to that in Example 2, and PET imaging was performed on the HT-1080-FAP mouse. The obtained images at 1 and 3 hours post-injection are shown in FIG. 13. It can be seen that ⁶⁸Ga-FAPI-CB-59 probe shows relatively high uptake in tumor sites, and there is almost no change in the tumor uptake at 3 hours compared with that at 1 hour, exhibiting good retention in the tumors.

### Example 7h: Comparative PET/CT Imaging of Al¹⁸F-FAPI-CB-77 and Al¹⁸F-FAPI-74 in HT-1080-FAP Tumor-Bearing Mouse Model

The Al¹⁸F-FAPI-74 (a structure is shown in FIG. 14a) labeled using the method in Example 2 was diluted with normal saline, and about 7.4 MBq of the diluted solution (at a volume of about 200 µL) was injected into each mouse. At 2 hours post-inj ection, PET imaging was performed, and PET image processing software was used for reconstruction. In addition, Al¹⁸F-FAPI-CB-77, as the control, was labeled via the Al¹⁸F labeling procedure in Example 2, and PET imaging was performed on the same mouse at intervals of 48 hours. The obtained image is shown in FIG. 14b. It can be seen that although the uptake of the Al¹⁸F-FAPI-CB-77 probe in tumor sites is lower than that of Al¹⁸F-FAPI-74, the uptake of the Al¹⁸F-FAPI-CB-77 probe in the biliary and intestinal backgrounds is significantly reduced, indicating that the Al¹⁸F-FAPI-CB-77 probe may still have certain advantages in tumor detection in the digestive system.

### Example 7i: Comparative PET/CT Imaging of ⁶⁸Ga-FAPI-CB-86 and ⁶⁸Ga-FAPI-04 in HT-1080-FAP Tumor-Bearing Mouse Model

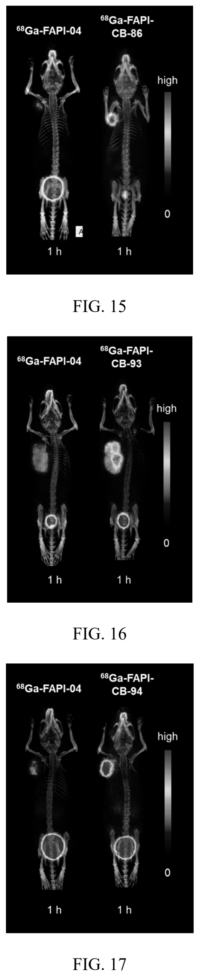

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 9 µg of FAPI-04 precursor was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-04 was obtained. The labeled ⁶⁸Ga-FAPI-04 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, FAPI-CB-50, as the control, was labeled using the same procedure, and PET imaging was performed on the same mouse at intervals of 24 hours. The obtained image at 2 hours post-injection is shown in FIG. 15a. It can be seen that the uptake of the ⁶⁸Ga-FAPI-CB-50 probe is higher than that of ⁶⁸Ga-FAPI-04 at tumor sites, with a lower blood pool background. As shown in FIG. 15b, the ratio of tumor SUVₘₐₓ to blood pool SUVₘₐₓ was examined, and ⁶⁸Ga-FAPI-CB-50 exhibited a better clearance rate.

### Example 7j: Comparative PET/CT Imaging of ⁶⁸Ga-FAPI-CB-93 and ⁶⁸Ga-FAPI-04 in HT-1080-FAP Tumor-Bearing Mouse Model

The ⁶⁸Ge-⁶⁸Ga generator (iThemba LABS, South Africa) was eluted with 5 mL of 0.6 M high-purity hydrochloric acid to obtain a Ga-68 hydrochloric acid solution. 1 mL of the eluted Ga-68 solution was taken, and 100 µL of 3M sodium hydroxide and 130 µL of 3M sodium acetate were added to adjust the acidity to a final pH of 4.0, 9 µg of FAPI-04 precursor was added, and the reaction mixture was heated to 90°C, and maintained at the temperature for 10 minutes. The reaction solution was passed through a C₁₈ column (Waters) to remove free ions, and then the C₁₈ column was eluted with an ethanol solution. Labeled ⁶⁸Ga-FAPI-04 was obtained. The labeled ⁶⁸Ga-FAPI-04 was diluted with normal saline, and about 3.7 MBq of the diluted solution (at a volume of about 200 µL) was injected into each mouse. At 90 minutes post-injection, PET imaging was performed, and PET image processing software was used for reconstruction. In addition, FAPI-CB-50, as the control, was labeled using the same procedure, and PET imaging was performed on the same mouse at intervals of 24 hours. The obtained image at 2 hours post-injection is shown in FIG. 16a. It can be seen that the uptake of the ⁶⁸Ga-FAPI-CB-50 probe is higher than that of ⁶⁸Ga-FAPI-04 at tumor sites, with a lower blood pool background. As shown in FIG. 16b, the ratio of tumor SUVₘₐₓ to blood pool SUVₘₐₓ was examined, and ⁶⁸Ga-FAPI-CB-50 exhibited a better clearance rate.

### Example 7k: Comparative PET/CT Imaging of ⁶⁸Ga-FAPI-CB-94 and ⁶⁸Ga-FAPI-04 in HT-1080-FAP Tumor-Bearing Mouse Model

The ⁶⁸Ga-FAPI-04 labeled using the method in Example 2 was diluted with normal saline, and about 3.7 MBq of the diluted solution (at a volume of about 200 µL) was injected into each mouse. At 1 hour post-injection, PET imaging was performed, and PET image processing software was used for reconstruction. In addition, FAPI-CB-94, as the control, was labeled using the same procedure, and PET imaging was performed on the same mouse at intervals of 24 hours. The obtained image at 1 hour post-injection is shown in FIG. 17. It can be seen that the uptake of the ⁶⁸Ga-FAPI-CB-94 is higher than that of ⁶⁸Ga-FAPI-04 at tumor sites, with no significant elevation in the blood pool background.

### Example 7l: Comparative PET/CT Imaging of Al¹⁸F-FAPI-CB-95 and Al¹⁸F-FAPI-74 in HT-1080-FAP Tumor-Bearing Mouse Model

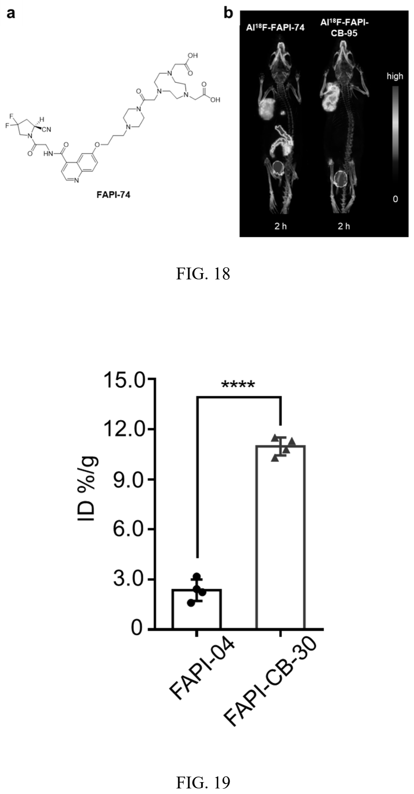

The Al¹⁸F-FAPI-74, the structure of which is shown in FIG. 18a, labeled using the method in Example 2, was diluted with normal saline, and about 7.4 MBq of the diluted solution (at a volume of about 200 µL) was injected into each mouse. At 2 hours post-injection, PET imaging was performed, and PET image processing software was used for reconstruction. In addition, Al¹⁸F-FAPI-CB-95, as the control, was labeled via the Al¹⁸F labeling procedure in Example 2, and PET imaging was performed on the same mouse at intervals of 48 hours. The obtained image is shown in FIG. 18b. It can be seen that the uptake of the Al¹⁸F-FAPI-CB-95 probe in tumor sites is higher than that of Al¹⁸F-FAPI-74, and the uptake of the Al¹⁸F-FAPI-CB-95 probe in the biliary and intestinal backgrounds is significantly reduced.

### Example 8: Tumor Uptake Value

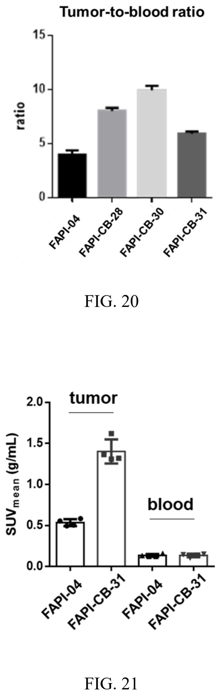

Each mouse bearing an HT-1080-FAP-labeled tumor (4 mice per group) was injected with 18.5 MBq of ⁶⁸Ga-FAPI-04 via the tail vein at time point 0, and then subjected to PET-CT scanning on Day 1. After 24 hours, ⁸Ga had basically decayed completely, 18.5 MBq of ⁶⁸Ga-FAPI-CB with the same specific activity was injected via the tail vein, and PET-CT scanning was performed again to obtain image data. The tumor uptake value can be semi-quantified by using the Standard Uptake Value (SUV) obtained by analyzing images reconstructed after PET-CT scanning via the software equipped on the equipment. SUV refers to the ratio of the radioactivity of the imaging agent taken up by local tissues to the average injection radioactivity in the whole body. That is, SUV = radioactive concentration in the lesion (kBq/mL)/(injection dose (MBq)/body weight (kg)). Compared with FAPI-04 in the most relevant prior art, in the HT-1080-FAP mouse model, the maximum standard uptake value (SUVₘₐₓ), mean standard uptake value (SUVₘₑₐₙ), or percentage of injection dose (ID%/g) of FAPI-CB molecules in some examples of the present invention all showed significant more than 3-fold increase at the time point of 1 hour (n=4, p < 0.0001, see FIG. 19). There is also a significant increase in the ratio of the standard uptake value of tumor to the standard uptake value of blood pool (see FIG. 20), and obvious advantages are also observed in the representative PET-CT images. In addition, in some embodiments, compared with FAPI-04, FAPI-CB molecules exhibit no significant difference in blood pool uptake at the time point of 1 hour, but have higher tumor uptake (see FIG. 21).

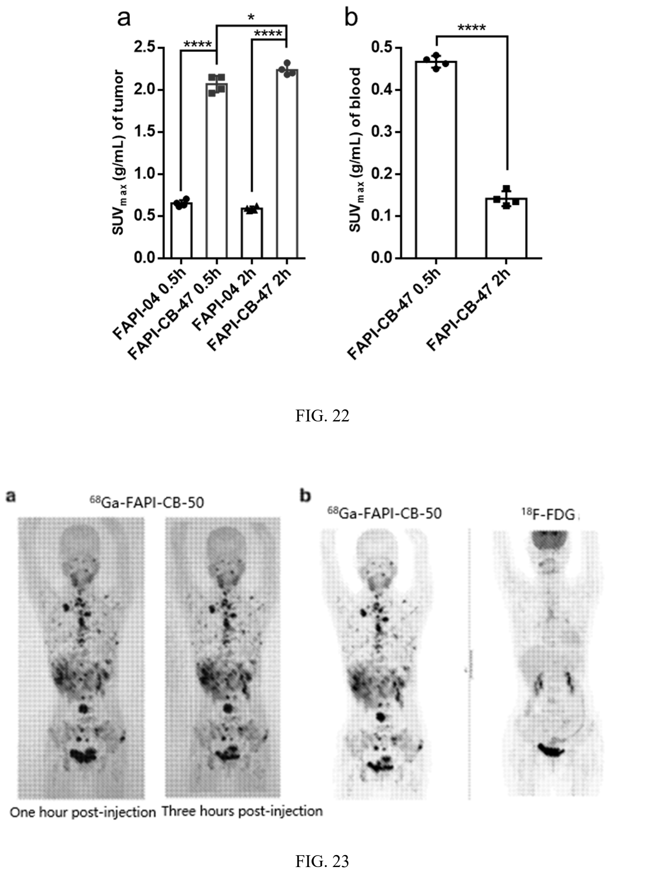

In some examples, for example, for PET-CT results of ⁶⁸Ga-FAPI-CB-47 and ⁶⁸Ga-FAPI-04, as shown in FIG. 22a, the tumor uptake value of ⁶⁸Ga-FAPI-CB-47 was significantly increased, and the tumor uptake was increased to some extent over time. FIG. 22b indicates that the clearance rate of ⁶⁸Ga-FAPI-CB-47 in the blood pool was relatively high, and compared with the blood pool uptake at the time point of 0.5 hours, the blood pool uptake at 2 hours was reduced by approximately two-thirds, showing controllable cumulative toxicity.

Therefore, the radionuclide-labeled FAPI-CB compounds according to the present disclosure exhibited significantly increased tumor uptake values and controllable or reduced background uptake, which is of great significance for improving the clinical tumor detection rate or facilitating tumor treatment.

### Example 9 PET/CT Imaging of ⁶⁸Ga-FAPI-CB-50 in Cancer Patients

The clinical-grade ⁶⁸Ge-⁶⁸Ga generator meeting GMP standards was eluted with 3 mL of 0.05 M high-purity hydrochloric acid to obtain a ⁶⁸Ga hydrochloric acid solution. An aliquot of the eluted ⁶⁸Ga solution was adjusted to pH 4.0, and 300 µg of the FAPI-CB-50 precursor was added. The reaction mixture was heated to 90°C and maintained at this temperature for 15 minutes. The reaction solution was passed through a C₁₈ column (Waters) to remove free ions, followed by elution of the C₁₈ column with an 80% ethanol solution to obtain labeled ⁶⁸Ga-FAPI-CB-50. The labeled ⁶⁸Ga-FAPI-CB-50 was diluted with normal saline filtered through a sterile membrane, and approximately 188 MBq of the drug was administered to patients with confirmed cancer via intravenous injection in the forearm. PET/CT scanning and imaging were performed at 60 minutes and 180 minutes post-injection, and reconstruction was carried out using PET-CT image processing software. The resulting images are shown in Figure 23a, respectively. It can be observed that the ⁶⁸Ga-FAPI-CB-50 probe exhibited high uptake in tumor sites, with a maximum standardized uptake value (SUVₘₐₓ) up to approximately 45. Additionally, the radioactivity in lesions showed almost no decrease over time, while the uptake in non-target organs decreased gradually.

The patient underwent ¹⁸F-FDG imaging within one week, and the resulting image is shown in Figure 23b. Compared with ¹⁸F-FDG, ⁶⁸Ga-FAPI-CB-50 clearly exhibited a superior tumor imaging effect.

### Example 10: SPECT/CT Imaging of ¹⁷⁷Lu-FAPI-CB-30 in Cancer Patients

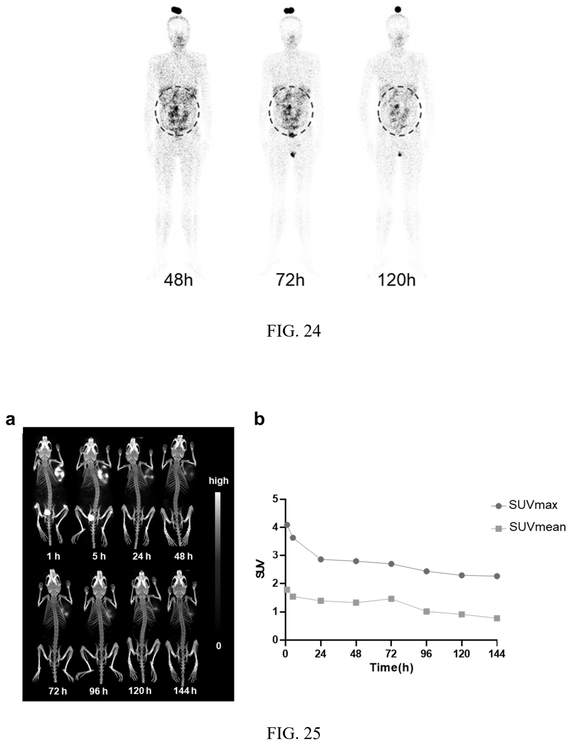

¹⁷⁷Lu-FAPI-CB-30 was obtained by labeling, in the method in Example 2, with carrier-free ¹⁷⁷Lu for clinical use that meets GMP standards (radiochemical yield > 99%, radiochemical purity > 99%, and molar activity ≈ 37 MBq/nmol). The labeled ¹⁷⁷Lu-FAPI-CB-30 was diluted with normal saline filtered through a sterile membrane, and about 1.11 GBq of the drug was administered via intravenous injection into the forearm vein of the patient diagnosed with cancer. At 24, 48, and 120 hours after injection, SPECT/CT scanning was performed. The images were reconstructed by using SPECT/CT image processing software. The obtained image is shown in FIG. 24. It can be seen that the uptake of ¹⁷⁷Lu-FAPI-CB-30 in tumor sites was relatively high (a peritoneal metastasis is outlined by a dashed circle). In addition, the uptake value in lesions decreased slowly over time, while the uptake value in non-target organs decreased more rapidly, which increased a target-to-background ratio, indicating a relatively wide therapeutic window of this candidate molecule.

### Example 11: SPECT/CT Imaging of ¹⁷⁷Lu-FAPI-CB-86 in Tumor-Bearing Mouse

The ¹⁷⁷Lu-FAPI-CB-86 labeled using the method in Example 2 was diluted with normal saline, and about 25.9 MBq of ¹⁷⁷Lu-FAPI-CB-86 (molar activity ≈ 29.6 MBq/nmol, and volume ≈ 200 µL) was injected into HT-1080-FAP tumor-bearing mice via tail veins. At 90 minutes post-injection, PET imaging was performed, and SPECT/CT image processing software was used for reconstruction. The obtained results are shown in FIG. 25, where FIG. 25a is the SPECT/CT image of the representative mouse, and FIG. 25b shows the tumor SUV-time curve. It can be seen from the results that the radionuclide molecules can be retained in tumors with high FAP expression for a long time and exhibit a significant therapeutic effect (tumor shrinkage).

### Example 12: Treatment of Tumor-Bearing Mice with ¹⁷⁷Lu-FAPI-CB-30

When an average tumor size reached approximately 100 mm³ (Day 0), HT-1080-FAP tumor-bearing mice in the same batch were randomly divided into 3 groups: a negative control group (injected with normal saline, where the number n of mice is equal to 8), a low-dose group (injected with 22.2 MBq of ¹⁷⁷Lu-FAPI-CB-30, where the number n of mice is equal to 6), and a high-dose group (injected with 44.4 MBq of ¹⁷⁷Lu-FAPI-CB-30, where the number n of mice is equal to 6). A single round of treatment was performed only on Day 0. The ¹⁷⁷Lu-labeled FAPI-CB-30 was prepared in the method described in Example 2 (radiochemical yield > 99%, radiochemical purity > 99%, and molar activity ≈ 29.6 MBq/nmol). The tumor size and body weight of the mice were monitored every 2 or 3 days.

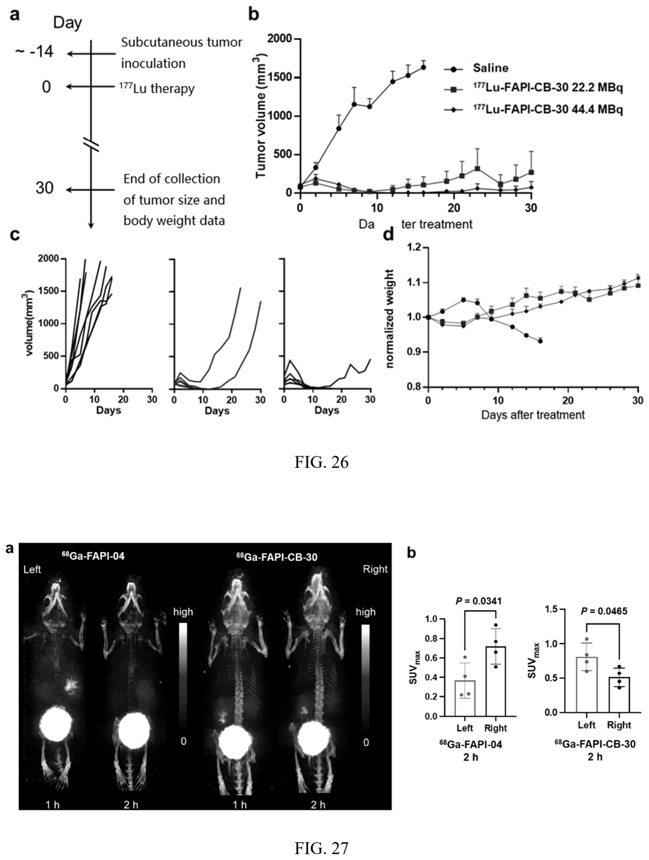

As shown in FIG. 26, FIG. 26a is a schematic diagram of a treatment protocol, FIG. 26b is an averaged tumor growth curve, FIG. 26c is a tumor growth curve of each mouse in each group, and FIG. 26d is an averaged normalized body weight curve. It can be seen that compared with the normal saline used for the control group, ¹⁷⁷Lu-FAPI-CB-30 exhibits a significant tumor inhibitory effect with a certain dose dependence, and the changes in mouse body weight indicate that toxicity of ¹⁷⁷Lu-FAPI-CB-30 is controllable.

### Example 13: Comparative PET/CT Imaging of ⁶⁸Ga-FAPI-CB-30 and ⁶⁸Ga-FAPI-04 in Mouse Model of Renal Fibrosis

A batch of mouse models with left renal fibrosis (with high FAP expression in fibrotic areas) were constructed by inducing left renal obstruction in mice via unilateral ureteral ligation, which is formally known as Unilateral Ureteral Obstruction (UUO). On Day 14 after modeling, the ⁶⁸Ga-FAPI-04 labeled using the method in Example 2 was diluted with normal saline, and 11.2 MBq of the diluted solution (at a volume of about 200 µL, where the number n of mice is equal to 4) was injected into each mouse model. At 1 hour post-injection, PET imaging was performed, and PET image processing software was used for reconstruction. In addition, the ⁶⁸Ga-FAPI-CB-30, as the control, was labeled using the same procedure, and PET imaging was performed on another 4 mouse models. The representative image (coronal plane) obtained at 1 hour post-injection is shown in FIG. 27a. ⁶⁸Ga-FAPI-CB-30 exhibits higher uptake in the left kidney with more severe renal fibrosis, while ⁶⁸Ga-FAPI-04 shows an opposite trend. FIG. 27b shows a quantitative result of a renal SUV value. This example illustrates the advantages of ⁶⁸Ga-FAPI-CB-30 in monitoring and staging of renal fibroses.

Unless specified in the context clearly otherwise, in this description and the appended claims, singular forms "a", "an", and "the" include a plural form. Unless otherwise defined, all technical and scientific terms used hereinhave the same meanings as those commonly understood by persons of ordinary skill in the art. Except for the disclosed specific order, the methods described herein may be performed in any logically possible order.

The representative examples are intended to help illustrate the present invention and are not intended for, nor should they be construed as, limiting the scope of the present invention. In fact, in addition to those shown and described herein, various modifications of the present invention and numerous other examples thereof shall become apparent to persons skilled in the art, including scientific and patent references cited in the examples and description. The examples contain important additional information, instances, and guidance that can be applied in the practice of the present invention in various examples and equivalents thereof.

## Claims

1. A trifunctional compound, or a pharmaceutically acceptable salt, stereoisomer or solvate thereof, wherein the trifunctional compound comprises at least one targeting moiety T and at least one payload P, **characterized in that**, in addition to any covalent warhead optionally present in the targeting moiety T itself, the trifunctional compound comprises at least one covalent warhead C as described below,
the payload P is any optical functional group, radiolabeled functional group, radiolabelable functional group, or functional group of a small-molecule cytotoxic drug, which is usable for optical imaging, positron emission tomography, single-photon emission computed tomography, chemotherapy, or radiotherapy;
the targeting moiety T comprises a targeting moiety capable of targeting a protein or a tissue, and the targeting moiety is a small molecule; and
the covalent warhead C is capable of forming a reversible or irreversible covalent linkage with the protein or tissue targeted by the targeting moiety T,
wherein the covalent warhead C is selected from the group consisting of: 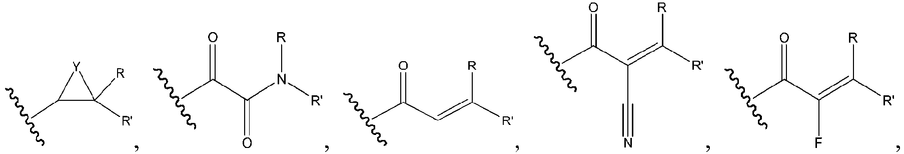 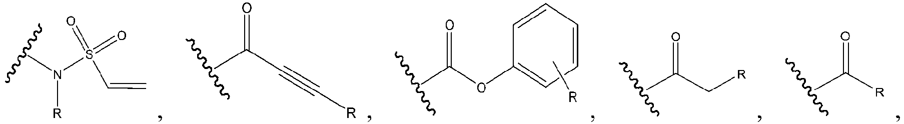 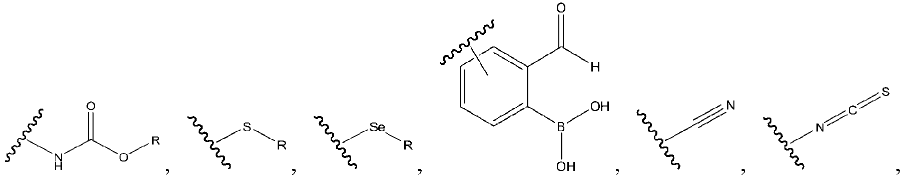 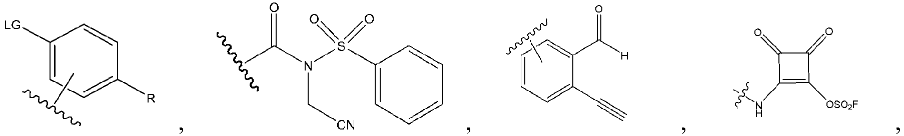 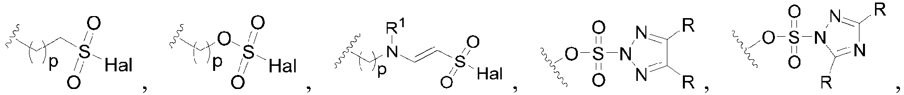 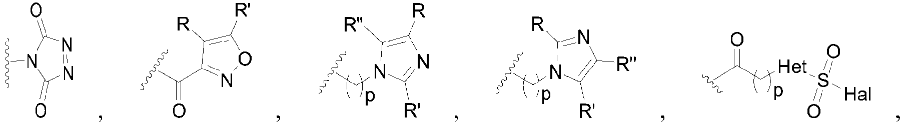 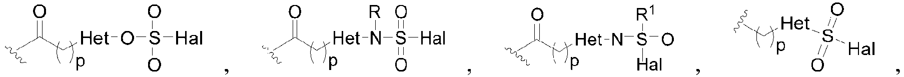 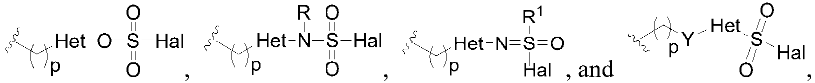
wherein:
Y is selected from the group consisting of O, S, and NR¹;
LG is a leaving group that is displaceable by a protein residue, and is preferably halogen or OTs, wherein Ts is a p-toluenesulfonyl group;
R, R' and R" are each independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
Het is an optionally substituted heterocyclic group or heteroaryl group, wherein when the heteroaryl group contains an N atom, the N atom is present in a free form or an onium salt form;
Hal is halogen, preferably F or Cl;
each p is independently an integer from 0 to 12; and
the at least one targeting moiety T, at least one payload P, and at least one covalent warhead C are connected via a linker moiety.

2. The trifunctional compound, or the pharmaceutically acceptable salt, stereoisomer or solvate thereof according to claim 1, wherein the covalent warhead C is selected from the group consisting of: 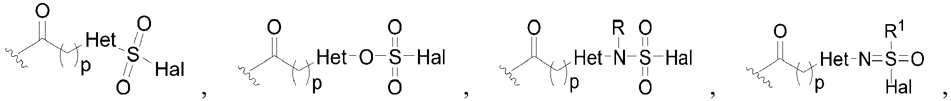 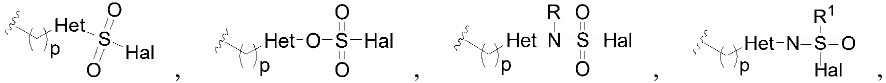 and 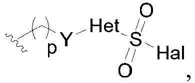
wherein Het is an optionally substituted C₄-C₁₂ heterocyclic group or an optionally substituted C₅-C₁₂ heteroaryl group, wherein when the heteroaryl group contains an N atom, the N atom is present in a free form or an onium salt form;
Hal is F;
each p is independently an integer from 0 to 6; and
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group.

3. The trifunctional compound, or the pharmaceutically acceptable salt, stereoisomer or solvate thereof according to claim 1, wherein C is selected from the group consisting of: 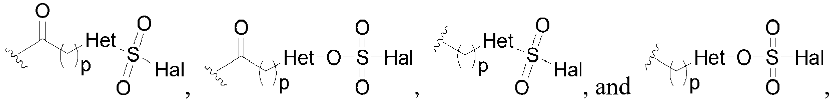 wherein:
Het is a C₄, C₅, or C₆ heterocyclic group or a C₅ or C₆ heteroaryl group, and the heterocyclic group or heteroaryl group contains one or two heteroatoms selected from N, O, or S, wherein when the heteroaryl group contains an N atom, the N atom is present in a free form or an onium salt form, preferably in the free form; more preferably, Het is an azetidinyl group, a pyridyl group, a pyrrolyl group, or an N-oxidopyridyl group;
Hal is F; and
each p is independently an integer from 0 to 4.

4. A trifunctional compound, a pharmaceutically acceptable salt, stereoisomer or solvate thereof, wherein the trifunctional compound comprises at least one targeting moiety T and at least one payload P, and in addition to any covalent warhead optionally present in the targeting moiety T itself, the trifunctional compound comprises at least one covalent warhead C as described below,
the payload P is any optical functional group, radiolabeled functional group, radiolabelable functional group, or functional group of a small-molecule cytotoxic drug, which is usable for optical imaging, positron emission tomography, single-photon emission computed tomography, chemotherapy, or radiotherapy;
the targeting moiety T comprises a targeting moiety capable of targeting a protein or a tissue, and the targeting moiety is a small molecule; and
the covalent warhead C is capable of forming a reversible or irreversible covalent linkage with the protein or tissue targeted by the targeting moiety T,
wherein the covalent warhead C is selected from the group consisting of: 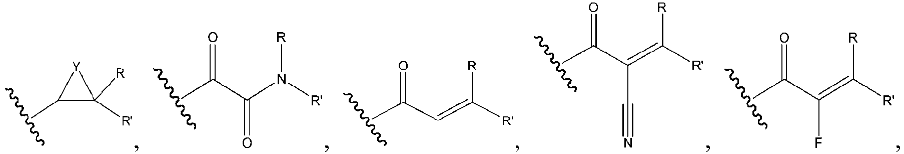 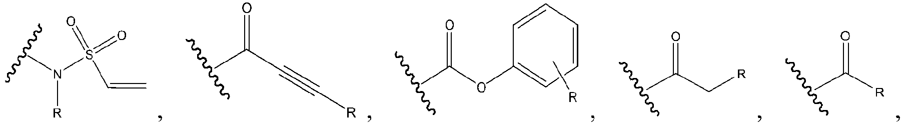 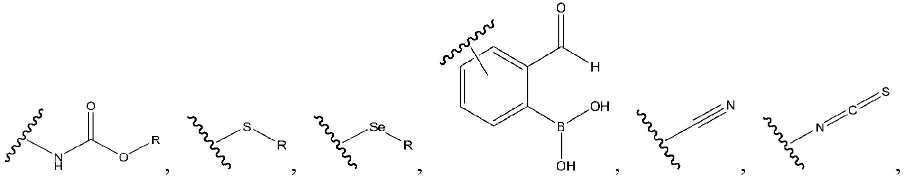 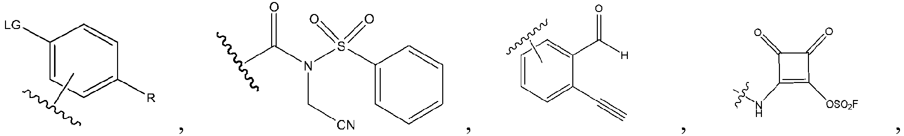 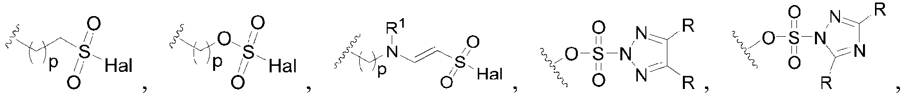 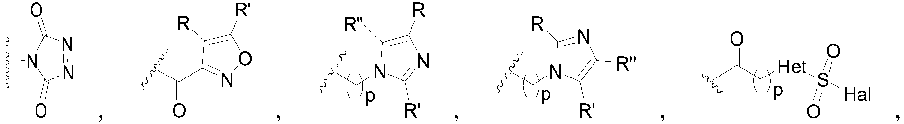 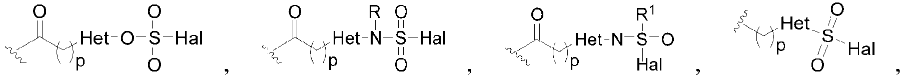 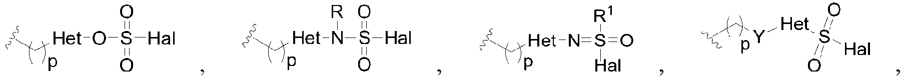 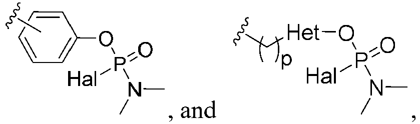 wherein:
Y is selected from the group consisting of O, S, and NR¹;
LG is a leaving group that is displaceable by a protein residue, and is preferably halogen or OTs, wherein Ts is a p-toluenesulfonyl group;
R, R' and R" are each independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
Het is an optionally substituted heterocyclic group or heteroaryl group, wherein when the heteroaryl group contains an N atom, the N atom is present in a free form or an onium salt form;
Hal is a leaving group, preferably halogen, OH, or NH₂, more preferably, F or Cl;
each p is independently an integer from 0 to 12; and
the at least one targeting moiety T, at least one payload P, and at least one covalent warhead C are connected via a linker moiety.

5. The trifunctional compound, or the pharmaceutically acceptable salt, stereoisomer or solvate thereof according to claim 4, wherein the covalent warhead C is selected from the group consisting of: 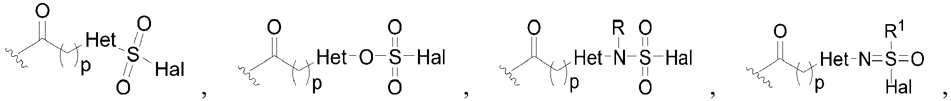 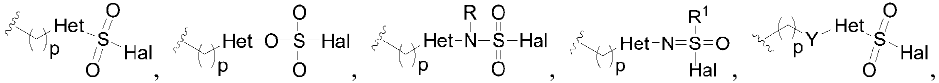 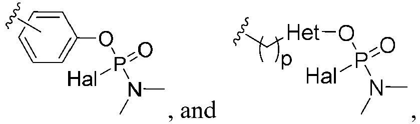
wherein Het is an optionally substituted C₄-C₁₂ heterocyclic group or an optionally substituted C₅-C₁₂ heteroaryl group, wherein when the heteroaryl group contains an N atom, the N atom is present in a free form or an onium salt form;
Hal is F;
each p is independently an integer from 0 to 6; and
R¹ is selected from the group consisting of hydrogen, an optionally substituted alkyl group, an optionally substituted cycloalkyl group, an optionally substituted heterocycloalkyl group, an optionally substituted aryl group, and an optionally substituted heteroaryl group.

6. The trifunctional compound, or the pharmaceutically acceptable salt, stereoisomer or solvate thereof according to claim 4, wherein C is selected from the group consisting of: 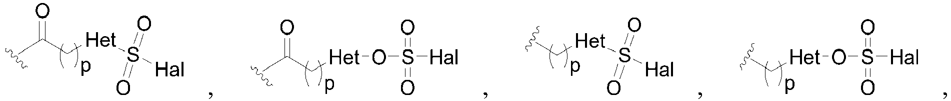 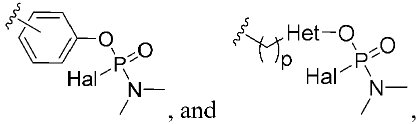 wherein:
Het is a C₄, C₅, or C₆ heterocyclic group or a C₅ or C₆ heteroaryl group, and the heterocyclic group or heteroaryl group contains one or two heteroatoms selected from N, O, or S, wherein when the heteroaryl group contains an N atom, the N atom is present in a free form or an onium salt form, preferably in the free form; more preferably, Het is an azetidinyl group, a pyridyl group, a pyrrolyl group, or an N-oxidopyridyl group;
Hal is F; and
each p is independently an integer from 0 to 4.

7. The trifunctional compound, or the pharmaceutically acceptable salt, stereoisomer or solvate thereof according to any one of claims 1 to 6, wherein the trifunctional compound comprises 1 to 3 (for example, 1 or 2) payloads P and 1 to 3 (for example, 1 or 2) targeting moieties T, and in addition to any covalent warhead optionally present in the targeting moiety T itself, the trifunctional compound comprises 1 to 3 (for example, 1 or 2) covalent warheads C, the linker moieties each independently comprises 0 to 6 linker units, and the linker unit is optionally a divalent linker (for example, a single bond) or a trivalent linker.

8. The trifunctional compound, or the pharmaceutically acceptable salt, stereoisomer or solvate thereof according to any one of claims 1 to 7, wherein the trifunctional compound has a structure of General Formula (I), General Formula (II), or General Formula (III): 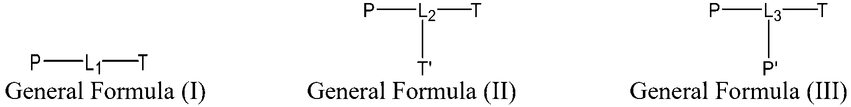 wherein:
payloads P and P' are each independently the same or different moieties selected from a radionuclide-containing moiety, a chelating moiety capable of chelating a radionuclide, an optical dye moiety, or a small-molecule cytotoxic drug moiety;
targeting moieties T and T' are each independently the same or different targeting moieties capable of targeting a protein or a tissue, and the targeting moiety is a small molecule;
in General Formula (I), General Formula (II), and General Formula (III), in addition to any covalent warhead optionally present in the targeting moiety T itself, the compound further comprises one, two, or three identical or different covalent warheads C, each independently connected to P, T, L₁, T', or P' in the general formulas via the same or different linker moieties L₄; and
each of L₁, L₂, and L₃ is a linker moiety and independently comprises 0 to 6 linker units.

9. The trifunctional compound, or the pharmaceutically acceptable salt, stereoisomer or solvate thereof according to any one of claims 1 to 8, wherein the trifunctional compound has a structure of General Formula (Ia), General Formula (Ib), General Formula (Ic), General Formula (IIa), or General Formula (IIIa): 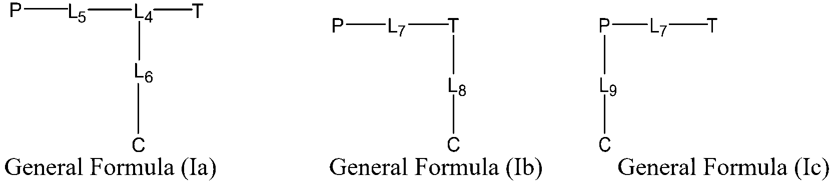 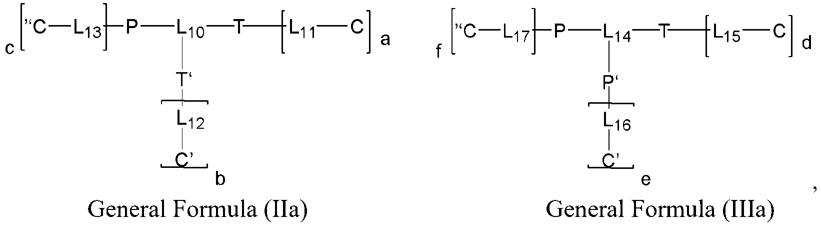 wherein:
L₄, L₅, L₆, L₇, L₈, L₉, L₁₀, L₁₁, L₁₂, L₁₃, L₁₄, L₁₅, L₁₆, and L₁₇ are each independently a linker moiety, and each independently contains 0 to 6 linker units;
a, b, c, d, e, and f are each independently 0 or 1, and 0 means that the group labeled by the number in the parenthesis does not exist, wherein the sum of a, b, and c is at least 1, and the sum of c, d, and e is at least 1; and
C, C', and C" each independently has the same meaning as defined in claims 1 to 6.

10. The trifunctional compound, or the pharmaceutically acceptable salt, stereoisomer or solvate thereof according to any one of claims 1 to 9, wherein the targeting moiety targets fibroblast activation protein- α (FAP-α).

11. The trifunctional compound, or the pharmaceutically acceptable salt, stereoisomer or solvate thereof according to any one of claims 1 to 10, wherein the targeting moiety has General Formula (IV), preferably General Formula (Iva), more preferably, General Formula (IVc), General Formula (IVd), General Formula (IVe), or General Formula (IVf): 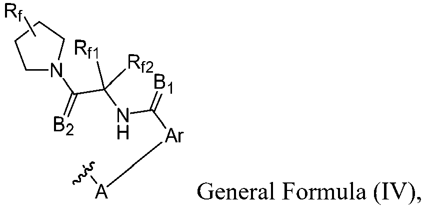
wherein in the General Formula (IV), A is selected from O, S, and NR_{A}, and R_{A} is selected from H and a C₁-C₆ alkyl group;
there are multiple R_{f} groups on the pyrrolidine ring, each R_{f} group is independently selected from H, F, Cl, -CN, -B(OH)₂, a C₁-C₆ alkyl group, and an α-chloroketone group, and optionally, two R_{f} groups on adjacent carbon atoms are connected to form a cycloalkyl group, preferably a C₃-C₇ cycloalkyl group;
B₁ and B₂ are each independently selected from O and S;
R_{f1} and R_{f2} are each independently selected from H, D, and a C₁-C₄ alkyl group; and
Ar is a C₆-C₁₀ heteroaryl group containing one N atom, 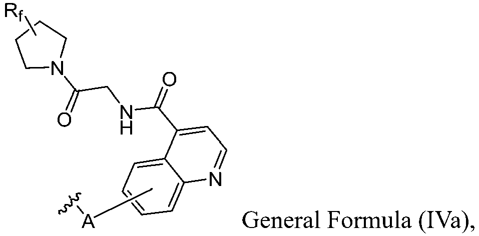
wherein in the General Formula (IVa), A is selected from O, S, and NR_{A}, and R_{A} is selected from H and a C₁-C₆ alkyl group; and
there are multiple R_{f} groups on the pyrrolidine ring, each R_{f} group is independently selected from H, F, Cl, -CN, a C₁-C₆ alkyl group, and an α-chloroketone group, and optionally, two R_{f} groups on adjacent carbon atoms are connected to form a cycloalkyl group, preferably a C₃-C₇ cycloalkyl group, 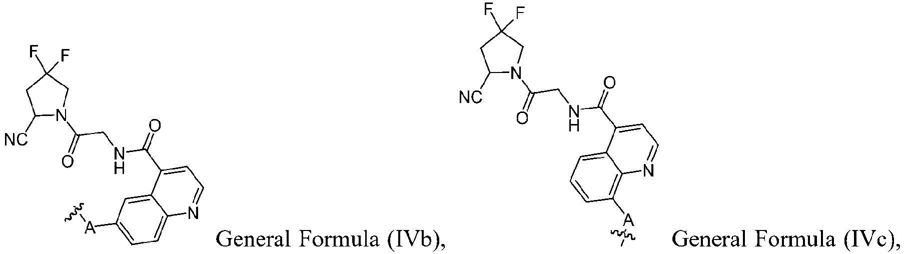 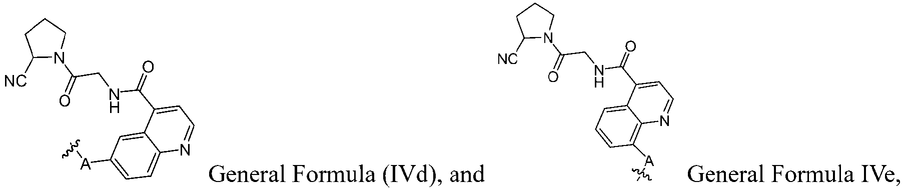
wherein in General Formula (IVb), (IVc), (IVd) or (IVe), A is selected from O, S, and NR_{A}, and R_{A} is selected from H, a methyl group, an ethyl group, an n-propyl group, and an isopropyl group.

12. The trifunctional compound, or the pharmaceutically acceptable salt, stereoisomer or solvate thereof according to any one of claims 1 to 9, wherein the targeting moiety has General Formula (V): 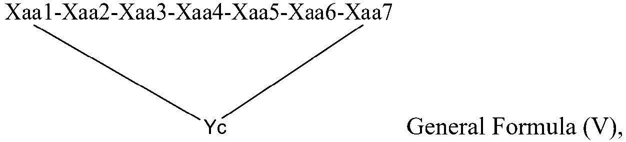
wherein the peptide sequence is depicted from left to right in a direction from the N-terminus to the C-terminus;
Xaa1 is a residue of an amino acid of General Formula (VI), 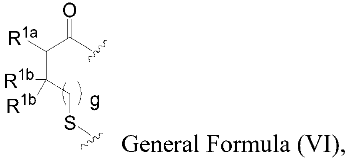
wherein:
R^{1a} is -NH-R^{1a'}, R^{1a'} is a protecting group or an amino acid, and preferably, the protecting group is R^{1a}"-C(O)- or R^{1a}"-S(O₂)-, and R^{1a}" is a C₁-C₆ alkyl group, and optionally, one -CH₂- group is replaced with -S- or -O-;
R^{1b} is H or a methyl group;
g is 0 or 1;
the carbonyl group of Xaa1 is covalently linked to a nitrogen atom of Xaa2;
the sulfur atom of Xaa1 is covalently linked to Yc as a thioether;
Xaa2 and Xaa3 are each independently selected from a residue of an amino acid of General Formula (VII) or General Formula (VIII), 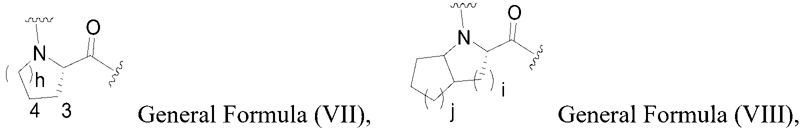
wherein:
h is 0, 1, or 2;
i is 1 or 2;
j is 1, 2, or 3;
the amino acid of the General Formula (VII) is optionally substituted with one or two substituents selected from a methyl group, OH, NH₂, and F at the 3- and 4-positions of the ring;
Xaa4 is a residue of an amino acid of General Formula (IX), 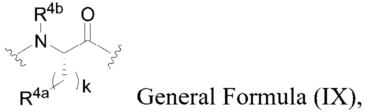
wherein:
R^{4a} is selected from H, OH, COOH, and CONH₂;
q is 1, 2, or 3, and optionally, one or two hydrogen atoms of the 1, 2, or 3 CH₂ groups are each independently substituted by a methyl group or an ethyl group;
R^{4b} is H or a methyl group;
Xaa5 is a residue of an amino acid of General Formula (X), 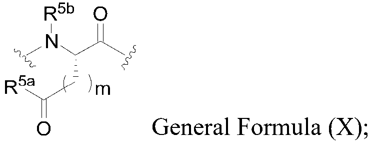
wherein:
R^{5a} is OH or NH₂;
m is 1, 2, or 3;
R^{5b} is H or a methyl group;
Xaa6 is an amino acid selected from aromatic L-α-amino acids, and is preferably a residue of an amino acid of General Formula (XI), 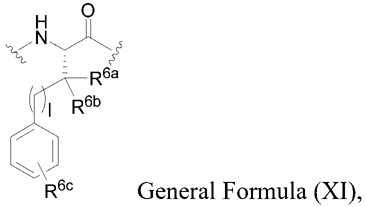
wherein:
R^{6a} and R^{6b} are each independently selected from H, a methyl group, an ethyl group, a propyl group, and an isopropyl group, preferably H;
R^{6c} represents 0 to 3 substituents, each independently selected from F, Cl, Br, NO₂, NH₂, CN, CHF₃, OH, OR^{6d}, and a C₁-C₄ alkyl group;
R^{6d} is selected from a methyl group, an ethyl group, a propyl group, and an isopropyl group; 1 is 0 or 1, preferably 0;
wherein the CR^{6a}R^{6b}(CH₂)₁PhR^{6c} moiety may form a portion of the covalent warhead C in the General Formula (I), (II), or (III); and
Xaa7 is a residue of an aminothiol or an amino acid of General Formula (XII), 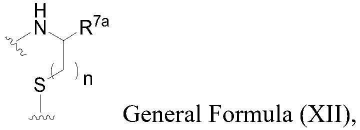
wherein:
R^{7a} is H, -COOH, -CONH₂, or CH₂OH;
n is 1 or 2; and
Yc has a structure of General Formula (XIII), 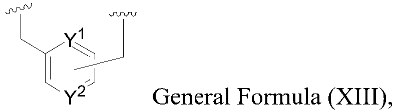
wherein Yc connects the S atom of Xaa1 and the S atom of Xaa7 by forming two thioether bonds, thereby forming a cyclic structure of General Formula (XIV), 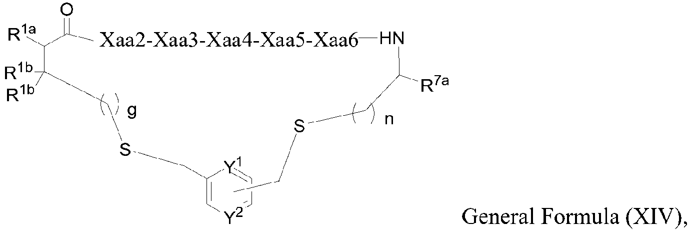
wherein a substitution pattern of the aromatic group in General Formula (XIII) is ortho, para,
or meta;
g is 0 or 1;
n is 1 or 2;
Y¹ is CH or N;
Y² is CR^{L1};
R^{L1} is a linker unit connected to another portion of General Formula (I), and
the targeting moiety preferably has Formula (Va):

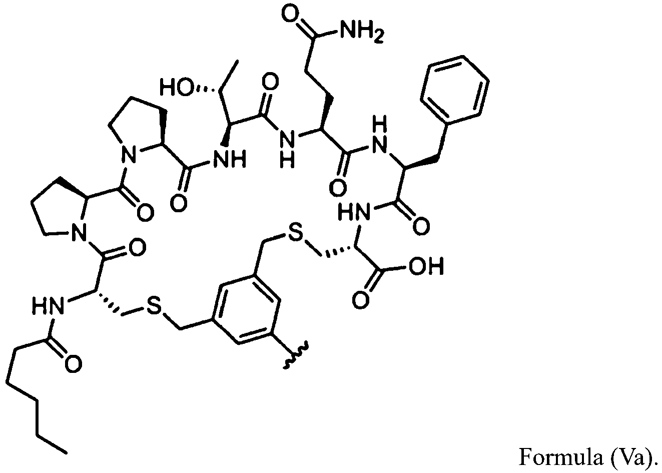

13. The trifunctional compound, or the pharmaceutically acceptable salt, stereoisomer or solvate thereof according to any one of claims 1 to 12, wherein the payload P comprises at least one moiety selected from the following groups of moieties:
Group 1: moieties with at least one radionuclide, selected from: 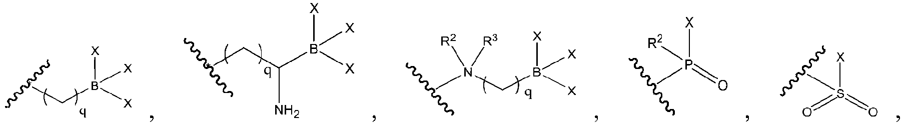 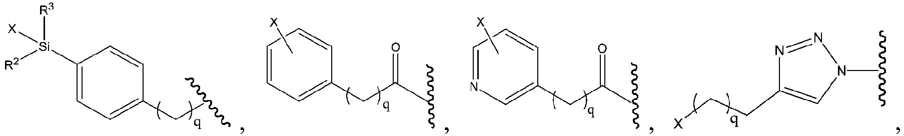 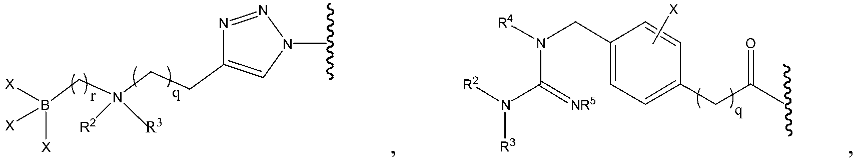 and 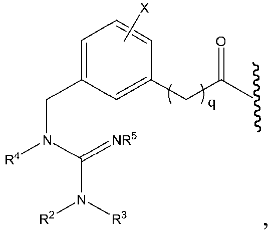
wherein R², R³, R⁴, and R⁵ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
X is selected from ¹⁸F, ¹²³I, ¹²⁴I, ¹²⁵I, ¹³¹I, and ²¹¹At, or a non-radioactive isotope thereof;
q and r are each independently an integer from 0 to 4; and
the N on the triazole ring at the linking site shown in the structure may not be present on the payload P, but on the linker unit instead;
Group 2: chelating moieties capable of chelating a radionuclide, selected from: 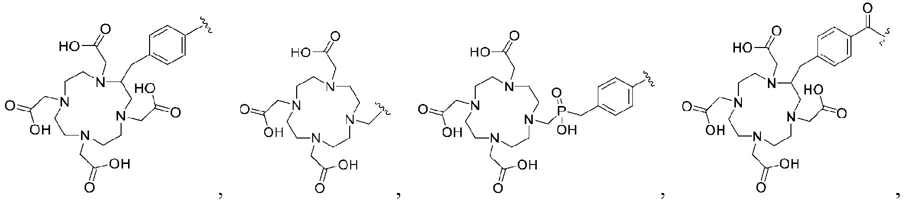 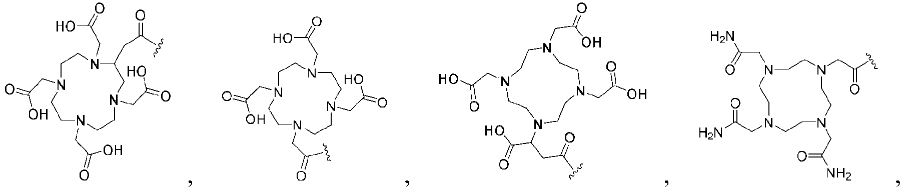 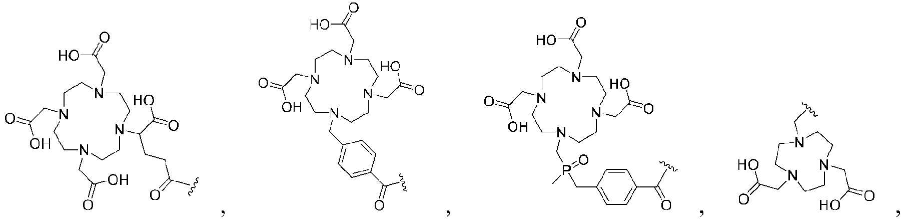 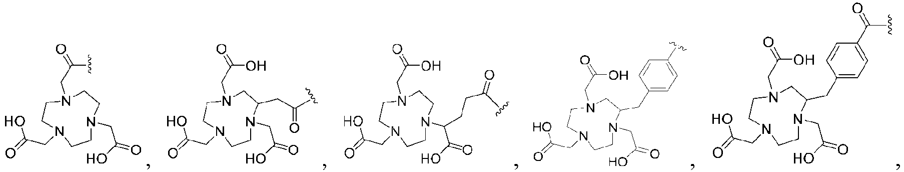 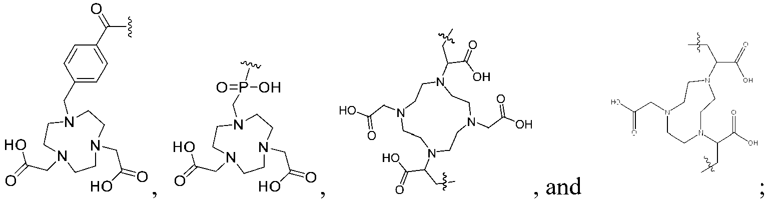
Group 3: Optical dye moieties, selected from: 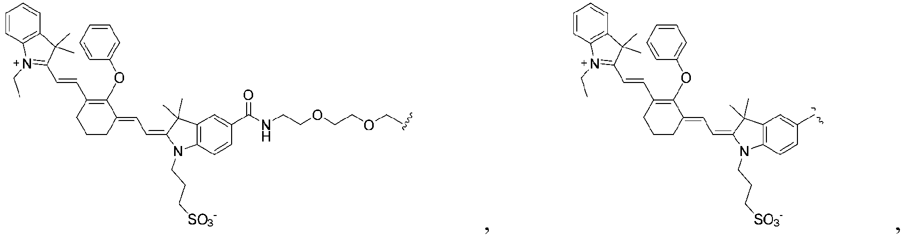 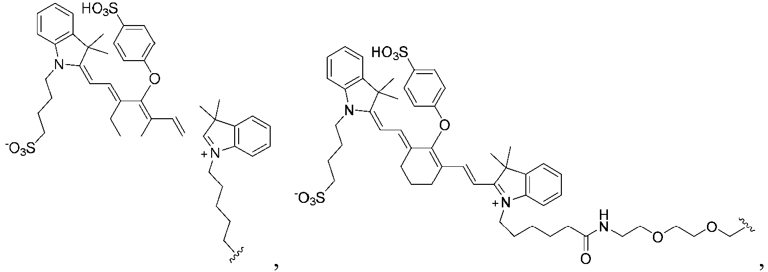 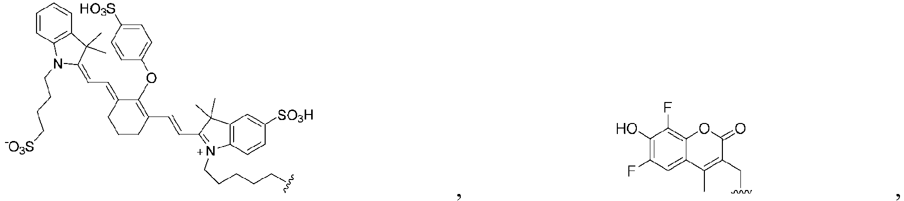 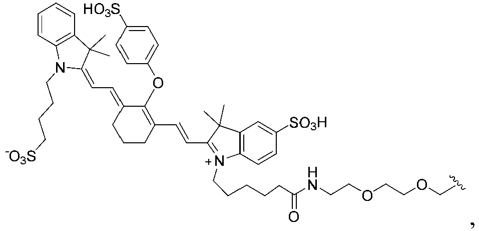 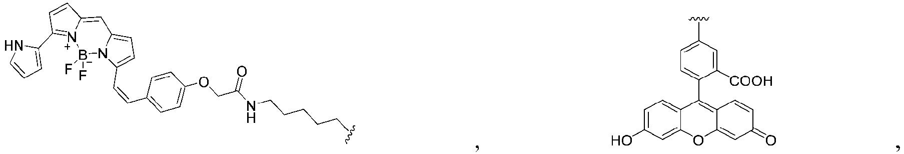 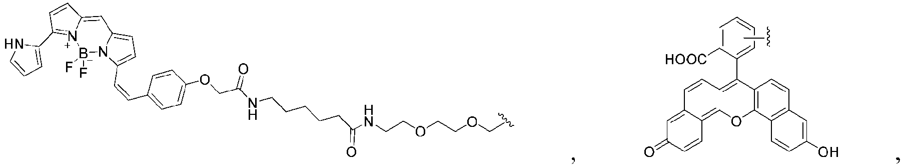 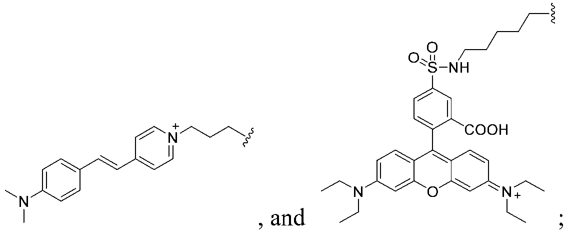 and
Group 4: Small-molecule cytotoxic drug moieties, selected from:

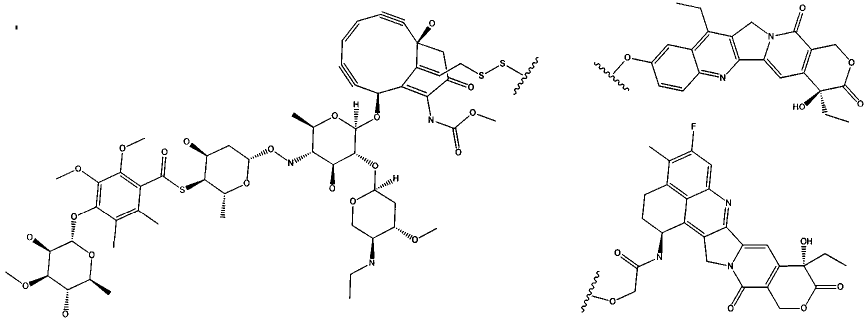

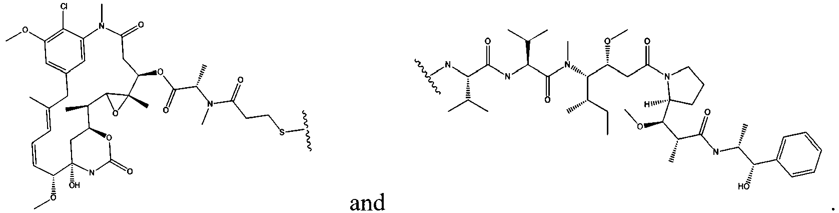

14. The trifunctional compound, or the pharmaceutically acceptable salt, stereoisomer or solvate thereof according to any one of claims 1 to 13, wherein
when L₁ is a trivalent linker, that is, when L₁ is linked to the covalent warhead C, L₄ is independently selected from: 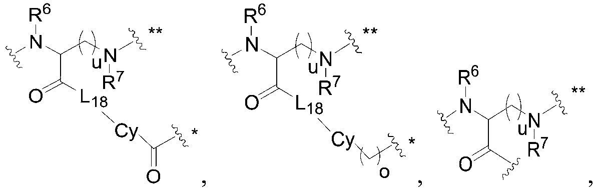 and 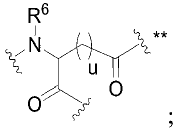
divalent linker units contained in L₁ to L₁₇ are each independently selected from: a single bond, 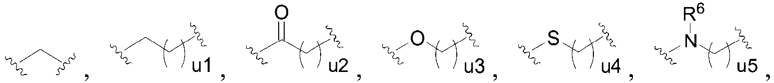 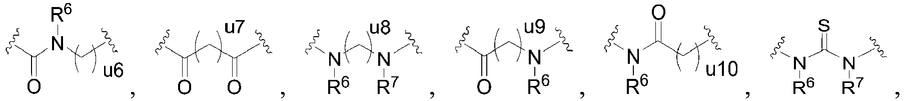 and 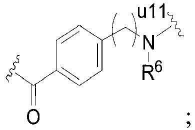
wherein the methylene group in the listed divalent linker units is optionally substituted with one or more -COR^{L2} or -COOR^{L2} groups, wherein each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 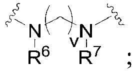
Cy is selected from 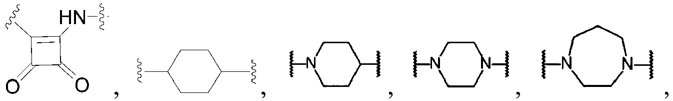 and 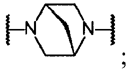
u, u1, u2, u3, u4, u5, u6, u7, u8, u9, u10, u11, v, and o are each independently 1, 2, 3, 4, or 5; R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
* is a site for linking the targeting moiety T; and
** is a site for linking the covalent warhead C,
on the condition that in L₁ to L₁₇, the heteroatoms are not directly connected via covalent bonds, wherein the heteroatoms are selected from N, O, and S.

15. The trifunctional compound, or the pharmaceutically acceptable salt, stereoisomer or solvate thereof according to any one of claims 1 to 13, wherein
when L₁ is a trivalent linker, that is, when L₁ is linked to the covalent warhead C, L₄ is independently selected from: 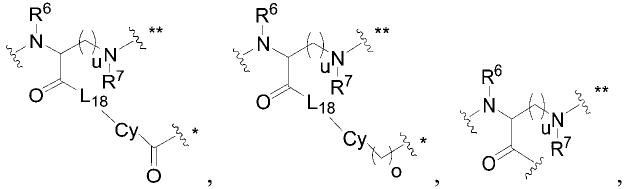 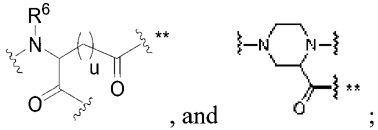
divalent linker units contained in L₁ to L₁₇ are each independently selected from: a single bond, 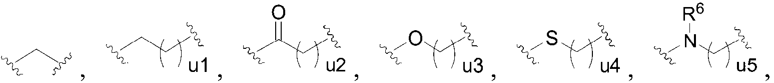 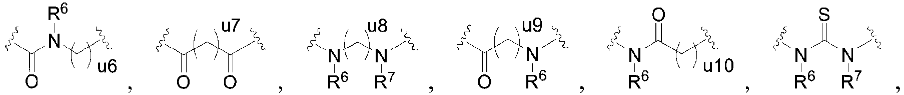 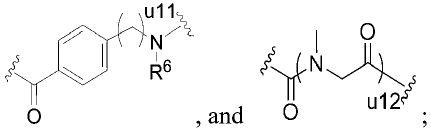
wherein the methylene group in the listed divalent linker units is optionally substituted with one or more -COR^{L2} or -COOR^{L2} groups, wherein each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 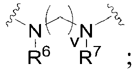
Cy is selected from 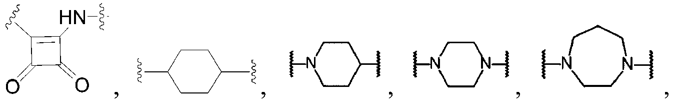 and 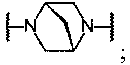
u, u1, u2, u3, u4, u5, u6, u7, u8, u9, u10, u11, u12, v, and o are each independently 0, 1, 2, 3, 4, 5, 6, 7, 8, 9, or 10;
R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
* is a site for linking the targeting moiety T; and
** is a site for linking the covalent warhead C;
on the condition that in L₁ to L₁₇, the heteroatoms are not directly connected via covalent bonds, wherein the heteroatoms are selected from N, O, and S.

16. The trifunctional compound, or the pharmaceutically acceptable salt, stereoisomer or solvate thereof according to any one of claims 1 to 11 and claims 13 to 15, wherein the trifunctional compound has a structure of General Formula (Ia), and L₄ is: 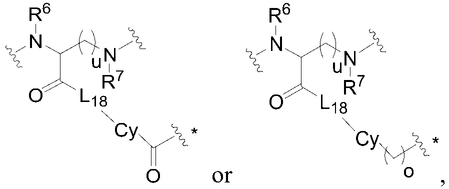
preferably, L₄ has the following structure: 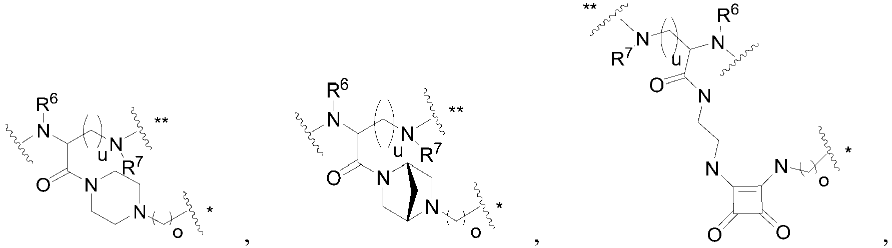 or 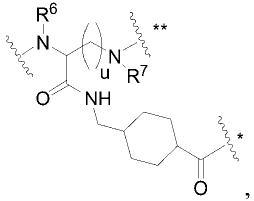
wherein R⁶, R⁷, L₁₈, Cy, o, and u have the same definitions as in claim 14 or 15.

17. The trifunctional compound, or the pharmaceutically acceptable salt, stereoisomer or solvate thereof according to claim 1, wherein the trifunctional compound has a structure of General Formula (Ia) or General Formula (Ib): 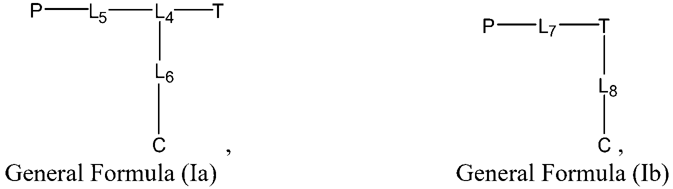 wherein:
L₄ is a trivalent linker moiety;
L₅, L₆, L₇, and L₈ are each independently a divalent linker moiety, and each independently contains 0 to 6 linker units, preferably 0 to 3 linker units;
C is a covalent warhead selected from the group consisting of: 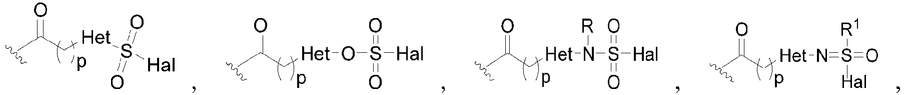 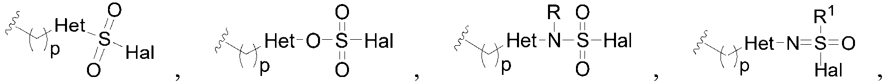 and 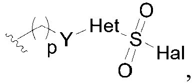
wherein Het is an optionally substituted C₄-C₁₂ heterocyclic group or an optionally substituted C₅-C₁₂ heteroaryl group, wherein when the heteroaryl group contains an N atom, the N atom is present in a free form or an onium salt form;
Hal is F or Cl;
Y is selected from the group consisting of O, S, and NR¹;
each p is independently an integer from 0 to 6;
each R¹ is independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
each R is independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
T is a targeting moiety that targets fibroblast activation protein-α (FAP-α); and
P is a chelating moiety capable of chelating a radionuclide.

18. The trifunctional compound, or the pharmaceutically acceptable salt, stereoisomer or solvate thereof according to claim 17, wherein the trifunctional compound has a structure of General Formula (Ia) or General Formula (Ib),
wherein:
L₄ is a trivalent linker selected from: 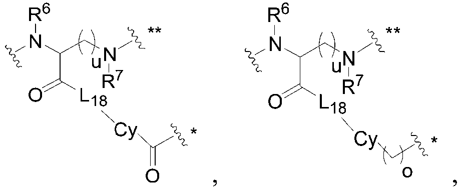 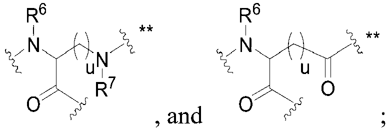
L₅, L₆, L₇, and L₈ are each independently a divalent linker moiety, and each independently contains 0 to 3 linker units, preferably 0 or 1 linker unit, and the linker unit is independently selected from:
a single bond, 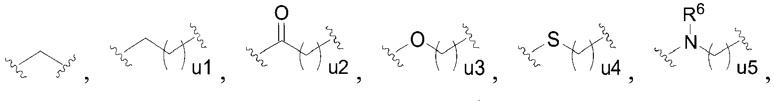 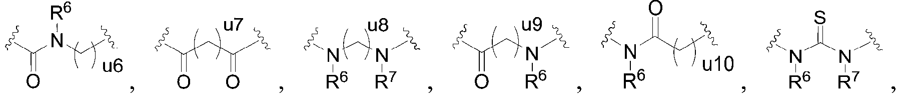 and 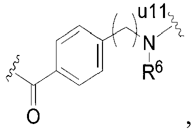
wherein the methylene group in the listed divalent linker units is optionally substituted with one or more -COR^{L2} or -COOR^{L2} groups, wherein each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 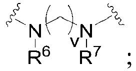
Cy is selected from 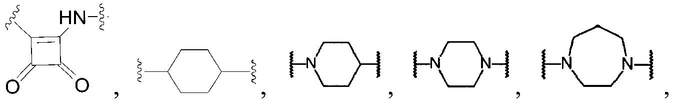 and 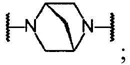
u, u1, u2, u3, u4, u5, u6, u7, u8, u9, u10, u11, v, and o are each independently 1, 2, 3, 4, or 5;
R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
* is a site for linking T; and
** is a site for linking C;
on the condition that in L₅, L₆, L₇, and L₈, the heteroatoms are not directly connected via covalent bonds, wherein the heteroatoms are selected from N, O, and S;
C is selected from the group consisting of: 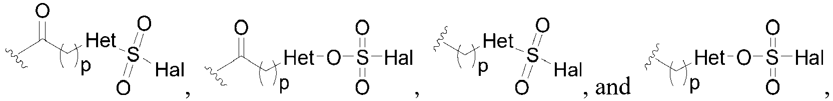
wherein:
Het is a C₄, C₅, or C₆ heterocyclic group or a C₅ or C₆ heteroaryl group, and the heterocyclic group or heteroaryl group contains one or two heteroatoms selected from N, O, or S, wherein when the heteroaryl group contains an N atom, the N atom is present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
Hal is F;
each p is independently an integer from 0 to 4;
T is a targeting moiety that targets fibroblast activation protein-α (FAP-α); and
P is a chelating moiety capable of chelating a radionuclide, selected from:

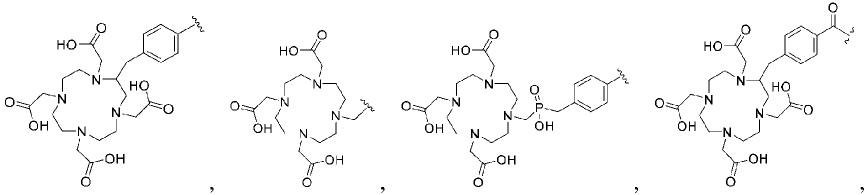

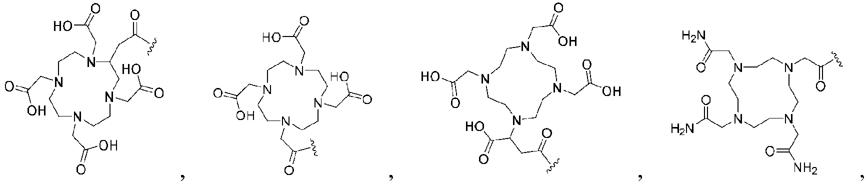

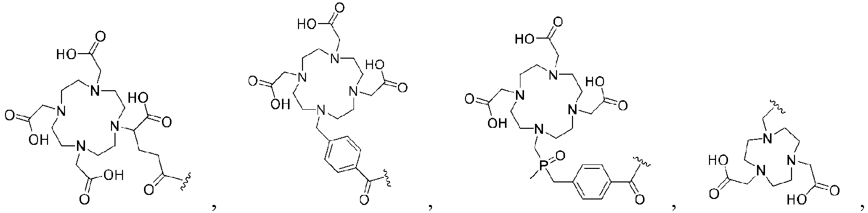

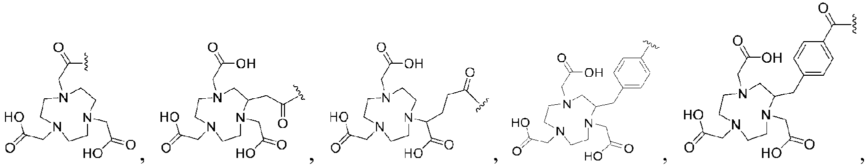

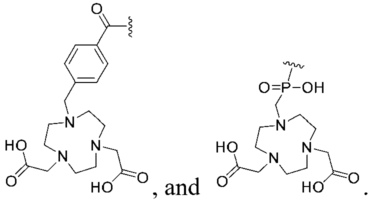

19. The trifunctional compound, or the pharmaceutically acceptable salt, stereoisomer or solvate thereof according to claim 17 or 18, wherein the trifunctional compound has a structure of General Formula (Ia):
wherein:
C and P have the same definitions as in claim 18; and
L₄ has the following structure: 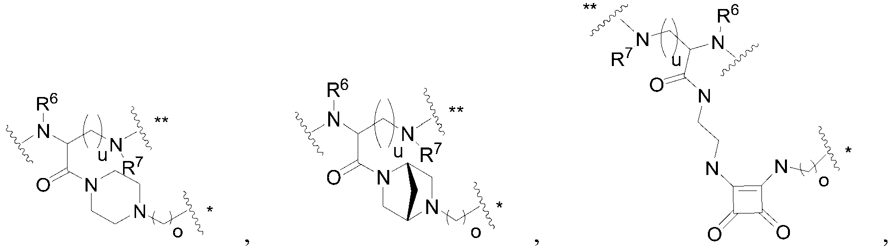 or 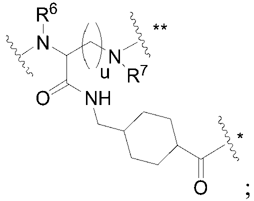
L₅ and L₆ are each independently:
a single bond, 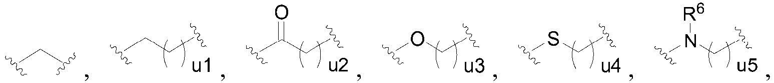 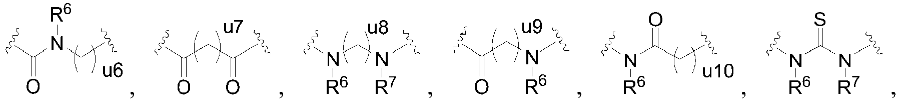 and 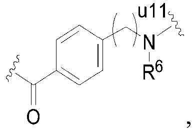
wherein u, u1, u2, u3, u4, u5, u6, u7, u8, u9, u10, u11, v, and o are each independently 1, 2, 3, 4, or 5;
R⁶ and R⁷ are each independently selected from hydrogen and an optionally substituted C₁-C₄ group;
* is a site for linking T;
** is a site for linking C;
on the condition that in L₅ and L₆, the heteroatoms are not directly connected via covalent bonds, wherein the heteroatoms are selected from N, O, and S;
T is represented by General Formula (IV), preferably General Formula (IVa), more preferably, General Formula (IVc), General Formula (IVd), General Formula (IVe), or General Formula (IVf), 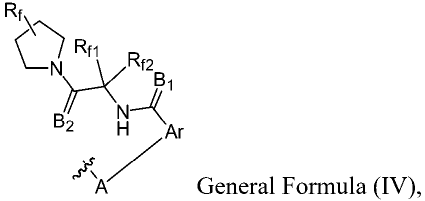
wherein in the General Formula (IV), A is selected from O, S, and NR_{A}, and R_{A} is selected from H and a C₁-C₆ alkyl group;
there are multiple R_{f} groups on the pyrrolidine ring, each R_{f} group is independently selected from H, F, Cl, -CN, -B(OH)₂, a C₁-C₆ alkyl group, and an α-chloroketone group, and optionally,
two R_{f} groups on adjacent carbon atoms are connected to form a cycloalkyl group, preferably a C₃-C₇ cycloalkyl group;
B₁ and B₂ are each independently selected from O and S;
R_{f1} and R_{f2} are each independently selected from H, D, and a C₁-C₄ alkyl group;
Ar is a C₆-C₁₀ heteroaryl group containing one N atom; 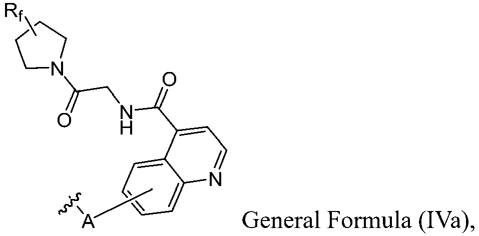
wherein in the General Formula (IVa), A is selected from O, S, and NR_{A}, and R_{A} is selected from H and a C₁-C₆ alkyl group; and
there are multiple R_{f} groups on the pyrrolidine ring, each R_{f} group is independently selected from H, F, Cl, -CN, and a C₁-C₆ alkyl group, and optionally, two R_{f} groups on adjacent carbon atoms are connected to form a cycloalkyl group, preferably a C₃-C₇ cycloalkyl group; 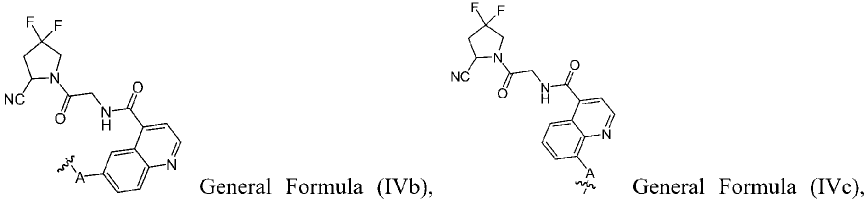 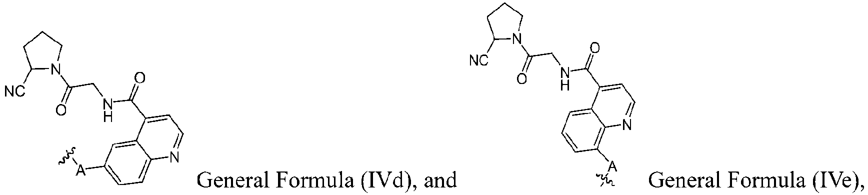
wherein in the General Formula (IVb), (IVc), (IVd) or (IVe), A is selected from O, S, and NR_{A}, and R_{A} is selected from H, a methyl group, an ethyl group, an n-propyl group, and an isopropyl group.

20. The trifunctional compound, or the pharmaceutically acceptable salt, stereoisomer or solvate thereof according to claim 17 or 18, wherein the trifunctional compound has a structure of General Formula (Ib),
wherein:
C and P in the General Formula (Ib) have the same definitions as in claim 18; and
L₇ and L₈ are each independently:
a single bond, 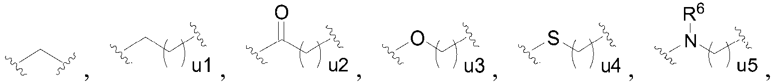 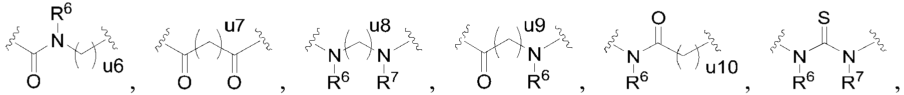 and 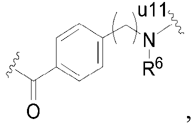
wherein u, u1, u2, u3, u4, u5, u6, u7, u8, u9, u10, u11, v, and o are each independently 1, 2, 3, 4, or 5;
R⁶ and R⁷ are each independently selected from hydrogen and an optionally substituted C₁-C₄ group;
on the condition that in L₇ and L₈, the heteroatoms are not directly connected via covalent bonds, wherein the heteroatoms are selected from N, O, and S;
T has General Formula (V), 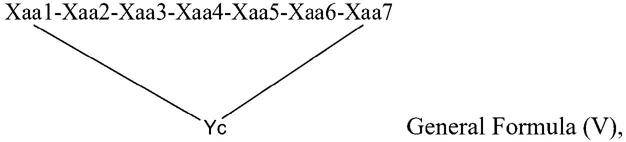
wherein the peptide sequence is depicted from left to right in a direction from the N-terminus to the C-terminus,
Xaal is a residue of an amino acid of General Formula (VI), 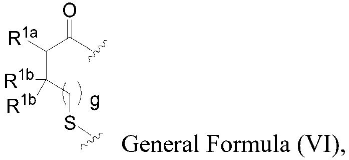
wherein:
R^{1a} is -NH-R^{1a'}, R^{1a'} is a protecting group or an amino acid, and preferably, the protecting group is R^{1a}"-C(O)- or R^{1a}"-S(O₂)-, and R^{1a}" is a C₁-C₆ alkyl group, and optionally, one -CH₂- group is replaced with -S- or -O-;
R^{1b} is H or a methyl group;
g is 0 or 1;
the carbonyl group of Xaa1 is covalently linked to a nitrogen atom of Xaa2;
the sulfur atom of Xaa1 is covalently linked to Yc as a thioether;
Xaa2 and Xaa3 are each independently selected from a residue of an amino acid of General Formula (VII) or General Formula (VIII), 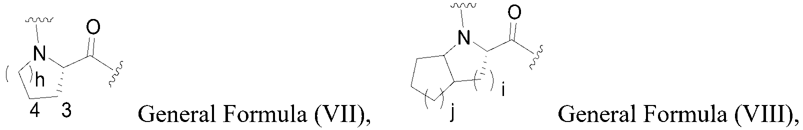
wherein:
h is 0, 1, or 2;
i is 1 or 2;
j is 1, 2, or 3;
the amino acid of the General Formula (VII) is optionally substituted with one or two substituents selected from a methyl group, OH, NH₂, and F at the 3- and 4-positions of the ring;
Xaa4 is a residue of an amino acid of General Formula (IX), 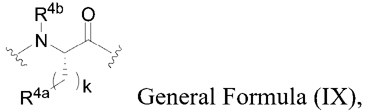
wherein:
R^{4a} is selected from H, OH, COOH, and CONH₂;
q is 1, 2, or 3, and optionally, one or two hydrogen atoms of the 1, 2, or 3 CH₂ groups are each independently substituted by a methyl group or an ethyl group;
R^{4b} is H or a methyl group;
Xaa5 is a residue of an amino acid of General Formula (X), 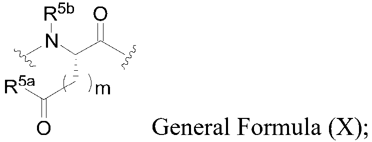
wherein:
R^{5a} is OH or NH₂;
m is 1, 2, or 3;
R^{5b} is H or a methyl group;
Xaa6 is an amino acid selected from aromatic L-α-amino acids, and is preferably a residue of an amino acid of General Formula (XI), 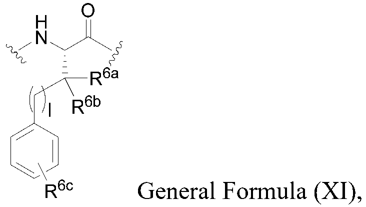
wherein:
R^{6a} and R^{6b} are each independently selected from H, a methyl group, an ethyl group, a propyl group, and an isopropyl group, preferably H;
R^{6c} represents 0 to 3 substituents, each independently selected from F, Cl, Br, NO₂, NH₂, CN, CHF₃, OH, OR^{6d}, and a C₁-C₄ alkyl group;
R^{6d} is selected from a methyl group, an ethyl group, a propyl group, and an isopropyl group; 1 is 0 or 1, preferably 0;
wherein the CR^{6a}R^{6b}(CH₂)₁PhR^{6c} moiety may form a portion of the covalent warhead C in the General Formula (Ib); and
Xaa7 is a residue of an aminothiol or an amino acid of General Formula (XII), 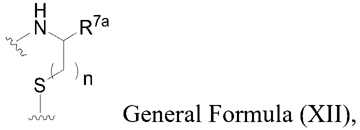
wherein:
R^{7a} is H, -COOH, -CONH₂, or CH₂OH;
n is 1 or 2; and
Yc has a structure of General Formula (XIII), 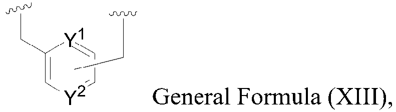
wherein Yc connects the S atom of Xaal and the S atom of Xaa7 by forming two thioether bonds, thereby forming a cyclic structure of General Formula (XIV), 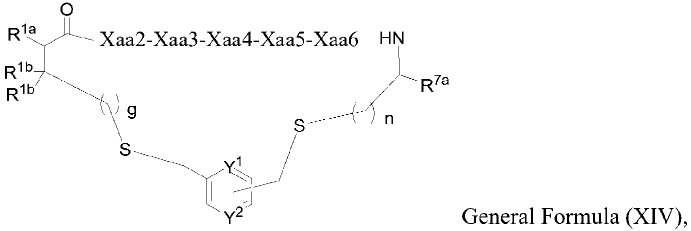
wherein a substitution pattern of the aromatic group in General Formula (XIII) is ortho, para, or meta;
g is 0 or 1;
n is 1 or 2;
Y¹ is CH or N;
Y² is CR^{L1};
R^{L1} is a linker unit connected to another portion of the General Formula (Ib).

21. The trifunctional compound, or the pharmaceutically acceptable salt, stereoisomer or solvate thereof according to claim 17 or 18, wherein the trifunctional compound has a structure of General Formula (Ib):
wherein:
L₇, L₈, C, and P have the same definitions as in claim 20; and
T has General Formula (V); 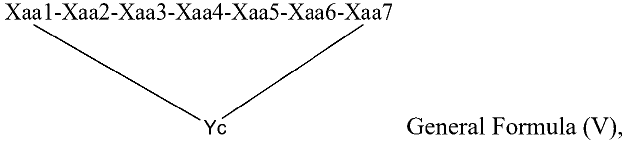
wherein the peptide sequence is depicted from left to right in a direction from the N-terminus to the C-terminus,
Xaal is a residue of an amino acid of General Formula (VI), 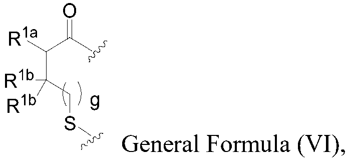
wherein:
R^{1a} is -NH-R^{1a'}, R^{1a'} is a protecting group or an amino acid, and preferably, the protecting group is R^{1a}"-C(O)- or R^{1a}"-S(O₂)-, and R^{1a}" is a C₁-C₆ alkyl group, and optionally, one -CH₂- group is replaced with -S- or -O-;
R^{1b} is H or a methyl group;
g is 0 or 1;
the carbonyl group of Xaa1 is covalently linked to a nitrogen atom of Xaa2;
the sulfur atom of Xaa1 is covalently linked to Yc as a thioether;
Xaa2 and Xaa3 are each independently selected from a residue of an amino acid of General Formula (VII) or General Formula (VIII), 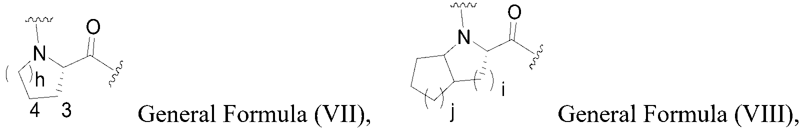
wherein:
h is 0, 1, or 2;
i is 1 or 2;
j is 1, 2, or 3;
the amino acid of the General Formula (VII) is optionally substituted with one or two substituents selected from a methyl group, OH, NH₂, and F at the 3- and 4-positions of the ring;
Xaa4 is a residue of an amino acid of General Formula (IX), 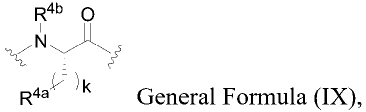
wherein:
R^{4a} is selected from H, OH, COOH, and CONH₂;
q is 1, 2, or 3, and optionally, one or two hydrogen atoms of the 1, 2, or 3 CH₂ groups are each independently substituted by a methyl group or an ethyl group;
R^{4b} is H or a methyl group;
Xaa5 is a residue of an amino acid of General Formula (X), 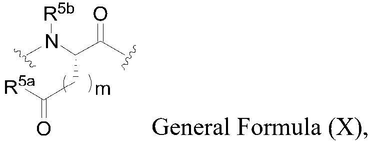
wherein:
R^{5a} is OH or NH₂;
m is 1, 2, or 3;
R^{5b} is H or a methyl group;
Xaa6 is a residue of an amino acid of General Formula (XI), 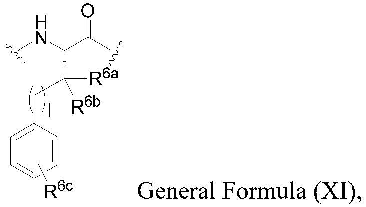
wherein:
R^{6a} and R^{6b} are each independently selected from H, a methyl group, an ethyl group, a propyl group, and an isopropyl group, preferably H;
R^{6c} represents 0 to 1 substituent, which is selected from F, Cl, NO₂, NH₂, CN, CHF₃, OH, and a C₁-C₄ alkyl group;
R^{6d} is selected from a methyl group, an ethyl group, a propyl group, and an isopropyl group; 1 is 0 or 1, preferably 0;
wherein the CR^{6a}R^{6b}(CH₂)₁PhR^{6c} moiety may form a portion of the covalent warhead C in the General Formula (Ib); and
Xaa7 is a residue of an aminothiol or an amino acid of General Formula (XII), 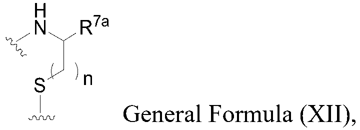
wherein:
R^{7a} is H or -COOH;
n is 1 or 2; and
Yc has a structure of General Formula (XIII), 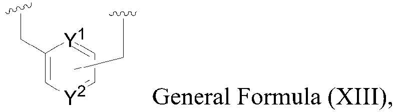
wherein Yc connects the S atom of Xaal and the S atom of Xaa7 by forming two thioether bonds, thereby forming a cyclic structure of General Formula (XIV), 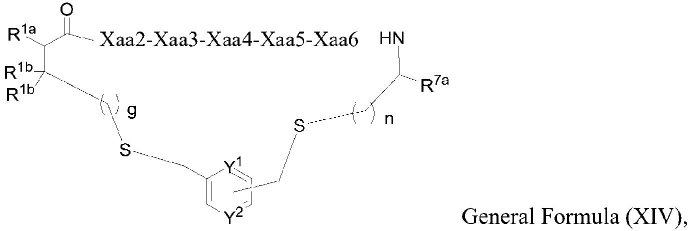
wherein a substitution pattern of the aromatic group in General Formula (XIII) is ortho, para, or meta;
g is 0 or 1;
n is 1 or 2;
Y¹ is CH or N;
Y² is CR^{L1};
R^{L1} is a linker unit connected to another portion of the General Formula (Ib); and
T preferably has Formula (Va):

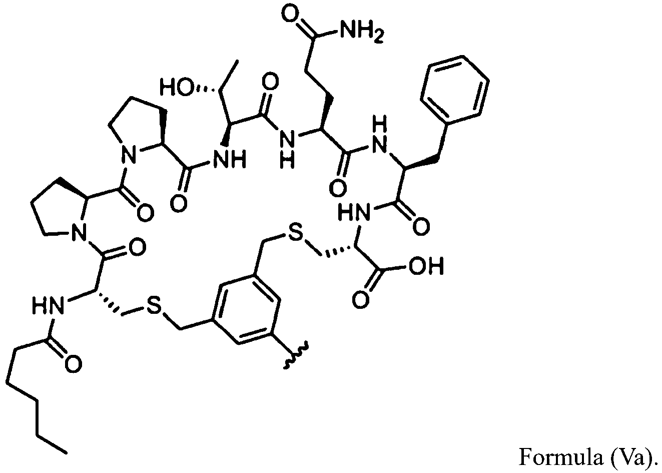

22. The trifunctional compound, or the pharmaceutically acceptable salt, stereoisomer or solvate thereof according to claim 4, wherein the trifunctional compound has a structure of General Formula (Ia) or General Formula (Ib): 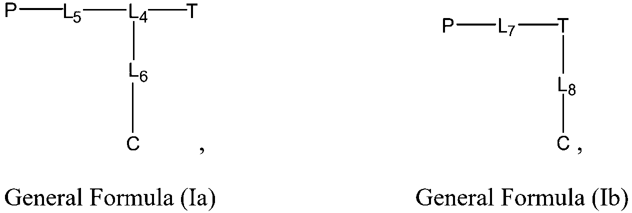 wherein:
L₄ is a trivalent linker moiety;
L₅, L₆, L₇, and L₈ are each independently a divalent linker moiety, and each independently contains 0 to 6 linker units, preferably 0 to 3 linker units;
C is a covalent warhead selected from the group consisting of: 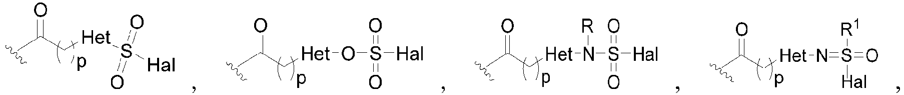 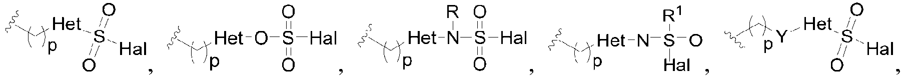 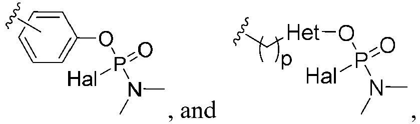
wherein Het is an optionally substituted C₄-C₁₂ heterocyclic group or an optionally substituted C₅-C₁₂ heteroaryl group, wherein when the heteroaryl group contains an N atom, the N atom is present in a free form or an onium salt form;
Hal is F or Cl;
Y is selected from the group consisting of O, S, and NR¹;
each p is independently an integer from 0 to 6;
each R¹ is independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
each R is independently selected from the group consisting of hydrogen, halogen, a nitro group, a cyano group, an optionally substituted mercapto group, an optionally substituted seleno group, an optionally substituted alkyl group, an optionally substituted cycloalkyl group, an optionally substituted heterocycloalkyl group, an optionally substituted aryl group, an optionally substituted heteroaryl group, and an optionally substituted amino group;
T is a targeting moiety that targets fibroblast activation protein-α (FAP-α); and
P is a chelating moiety capable of chelating a radionuclide.

23. The trifunctional compound, or the pharmaceutically acceptable salt, stereoisomer or solvate thereof according to claim 22, wherein the trifunctional compound has a structure of General Formula (Ia) or General Formula (Ib):
wherein:
L₄ is a trivalent linker selected from: 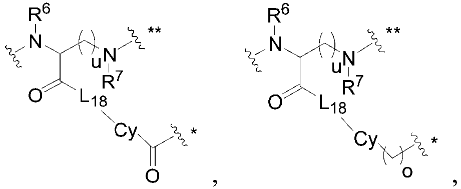 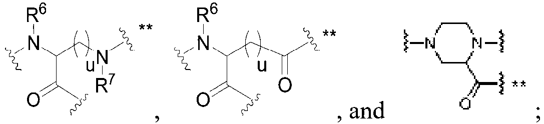
L₅, L₆, L₇, and L₈ are each independently a divalent linker moiety, and each independently contains 0 to 3 linker units, preferably 0 or 1 linker unit, and the linker units are each independently selected from:
a single bond, 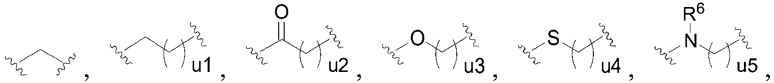 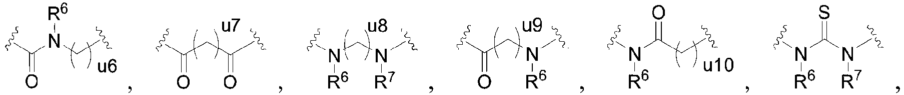 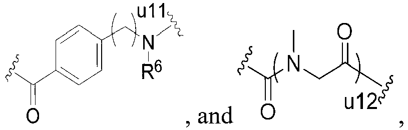
wherein the methylene group in the listed divalent linker units is optionally substituted with one or more -COR^{L2} or -COOR^{L2} groups, wherein each R^{L2} is independently selected from H, a C₁-C₆ alkyl group, a C₆-C₁₀ aryl group, and a C₁-C₆ alkyl group substituted with a C₆-C₁₀ aryl group;
L₁₈ is a single bond, -CH₂-, -NHCH₂-, or 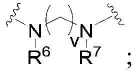
Cy is selected from 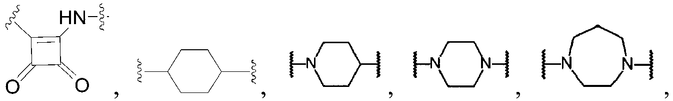 and 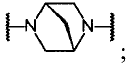
u, u1, u2, u3, u4, u5, u6, u7, u8, u9, u10, u11, u12, v, and o are each independently 0, 1, 2, 3, 4, 5, 6, 7, 8, 9, or 10;
R⁶ and R⁷ are each independently selected from the group consisting of hydrogen, an optionally substituted alkyl group, an optionally substituted cycloalkyl group, an optionally substituted heterocycloalkyl group, an optionally substituted aryl group, and an optionally substituted heteroaryl group;
* is a site for linking T;
** is a site for linking C;
on the condition that in L₅, L₆, L₇, and L₈, the heteroatoms are not directly connected via covalent bonds, wherein the heteroatoms are selected from N, O, and S;
C is selected from the group consisting of: 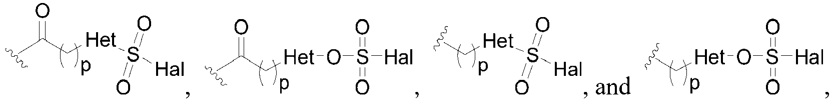
wherein:
Het is a C₄, C₅, or C₆ heterocyclic group or a C₅ or C₆ heteroaryl group, and the heterocyclic group or heteroaryl group contains one or two heteroatoms selected from N, O, or S, wherein when the heteroaryl group contains an N atom, the N atom is present in a free form or an onium salt form, preferably in the free form; preferably, Het is a C₄, C₅, or C₆ heterocyclic group, or a C₅ or C₆ heteroaryl group, and the heterocyclic group or heteroaryl group contains one heteroatom of N, O or S, preferably N; more preferably, Het is an azetidinyl group, a pyridyl group, a pyrrolyl group, or an N-oxidopyridyl group;
Hal is F;
each p is independently an integer from 0 to 4;
T is a targeting moiety that targets fibroblast activation protein-α (FAP-α); and
P is a chelating moiety capable of chelating a radionuclide, selected from:

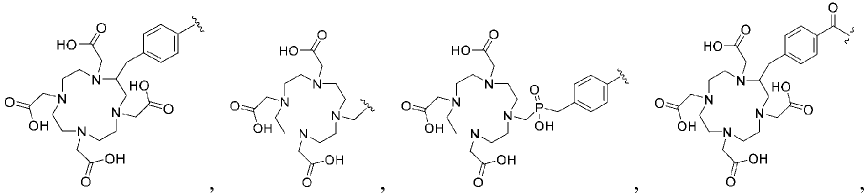

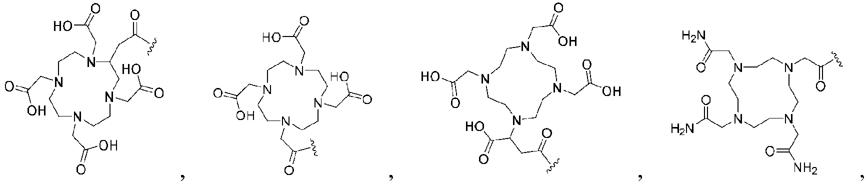

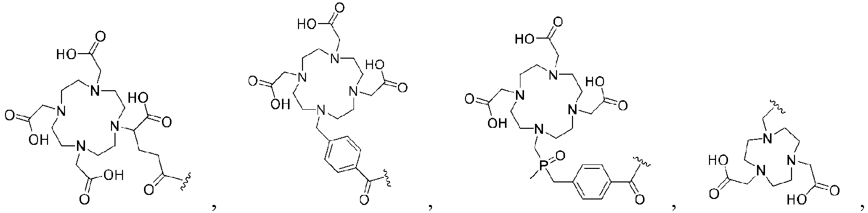

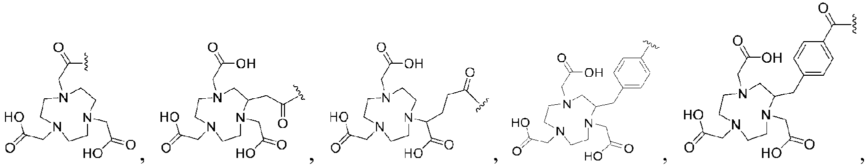

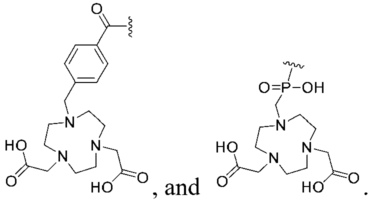

24. The trifunctional compound, or the pharmaceutically acceptable salt, stereoisomer or solvate thereof according to claim 22 or 23, wherein the trifunctional compound has a structure of General Formula (Ia):
wherein:
C and P have the same definitions as in claim 23;
L₄ has the following structure: 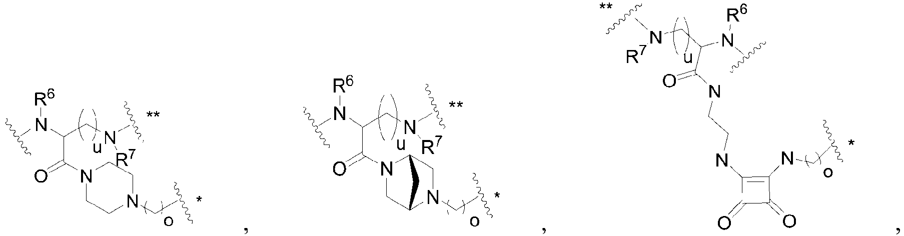 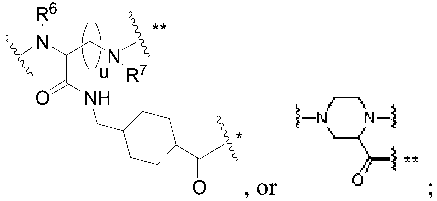
L₅ and L₆ are each independently:
a single bond, 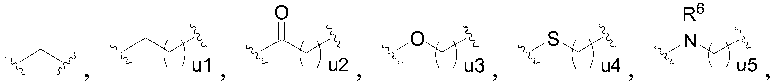 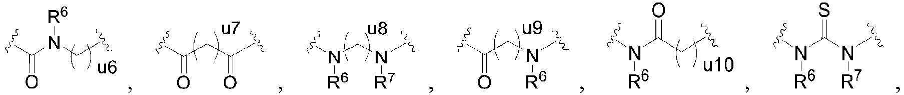 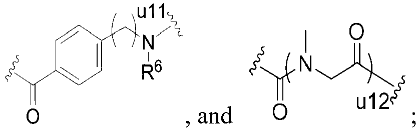
wherein u, u1, u2, u3, u4, u5, u6, u7, u8, u9, u10, u11, u12, v, and o are each independently 0, 1, 2, 3, 4, 5, 6, 7, 8, 9, or 10;
R⁶ and R⁷ are each independently selected from hydrogen and an optionally substituted C₁-C₄ group;
* is a site for linking T;
** is a site for linking C;
on the condition that in L₅ and L₆, the heteroatoms are not directly connected via covalent bonds, wherein the heteroatoms are selected from N, O, and S;
T is represented by General Formula (IV), preferably General Formula (IVa), more preferably, General Formula (IVc), General Formula (IVd), General Formula (IVe), or General Formula (IVf), 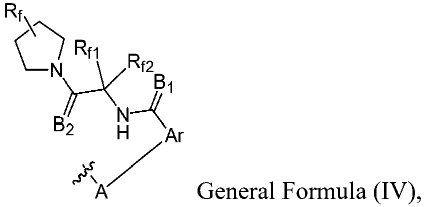
wherein in the General Formula (IV), A is selected from O, S, and NR_{A}, and R_{A} is selected from H and a C₁-C₆ alkyl group;
there are multiple R_{f} groups on the pyrrolidine ring, each R_{f} group is independently selected from H, F, Cl, -CN, -B(OH)₂, a C₁-C₆ alkyl group, and an α-chloroketone group, and optionally, two R_{f} groups on adjacent carbon atoms are connected to form a cycloalkyl group, preferably a C₃-C₇ cycloalkyl group;
B₁ and B₂ are each independently selected from O\ and S;
R_{f1} and R_{f2} are each independently selected from H, D, and a C₁-C₄ alkyl group;
Ar is a C₆-C₁₀ heteroaryl group containing one N atom; 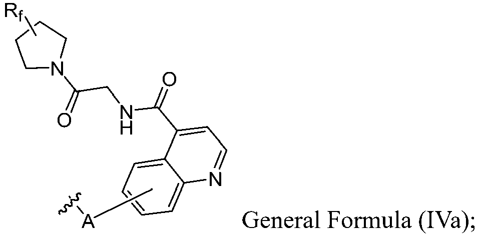
wherein in the General Formula (IVa), A is selected from O, S, and NR_{A}, and R_{A} is selected from H and a C₁-C₆ alkyl group; and
there are multiple R_{f} groups on the pyrrolidine ring, each R_{f} group is independently selected from H, F, Cl, -CN, and a C₁-C₆ alkyl group, and optionally, two R_{f} groups on adjacent carbon atoms are connected to form a cycloalkyl group, preferably a C₃-C₇ cycloalkyl group; 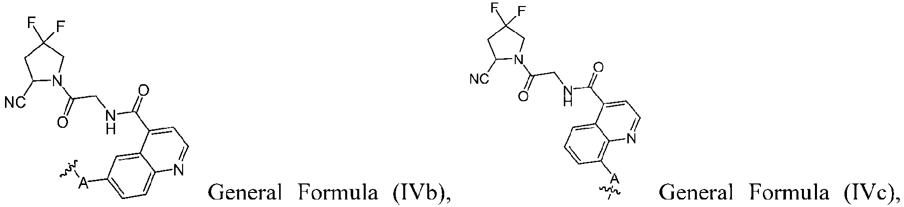 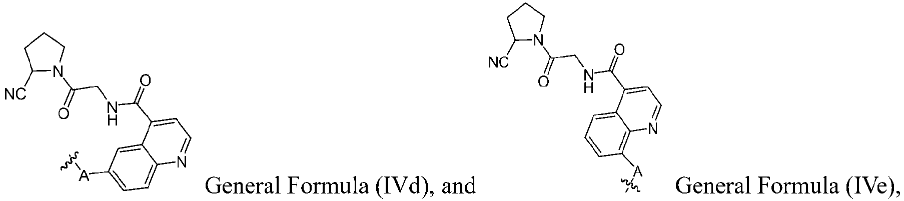
wherein in the General Formula (IVb), (IVc), (IVd) or (IVe), A is selected from O, S, and NR_{A}, and R_{A} is selected from H, a methyl group, an ethyl group, an n-propyl group, and an isopropyl group.

25. The trifunctional compound, or the pharmaceutically acceptable salt, stereoisomer or solvate thereof according to claim 1 or 4, which has one of the following structures: 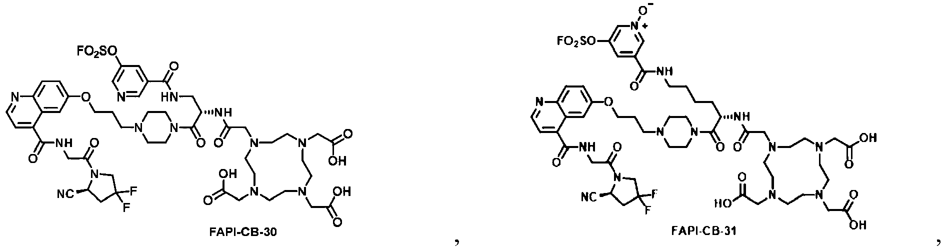 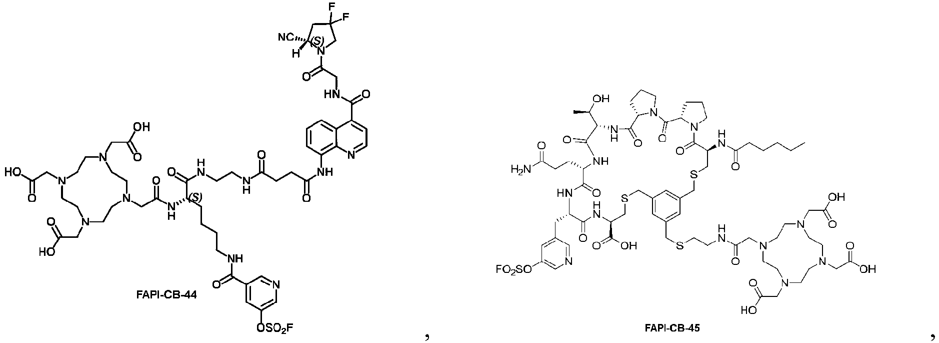 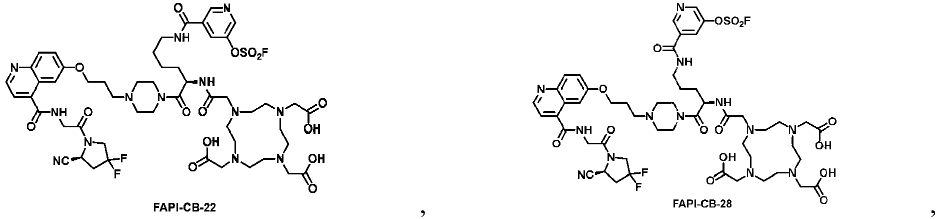 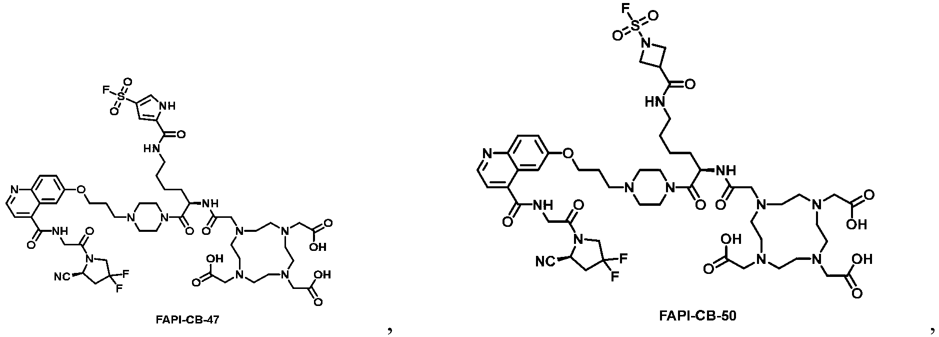 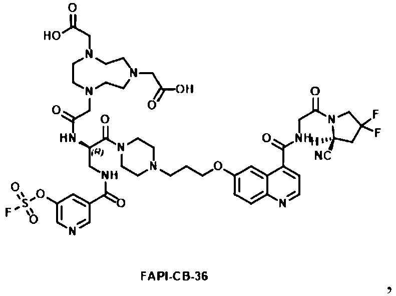 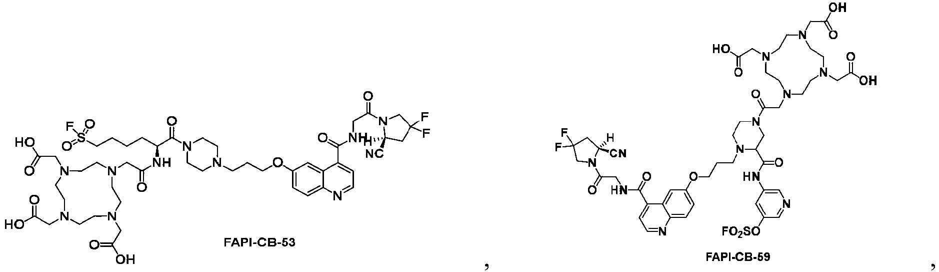 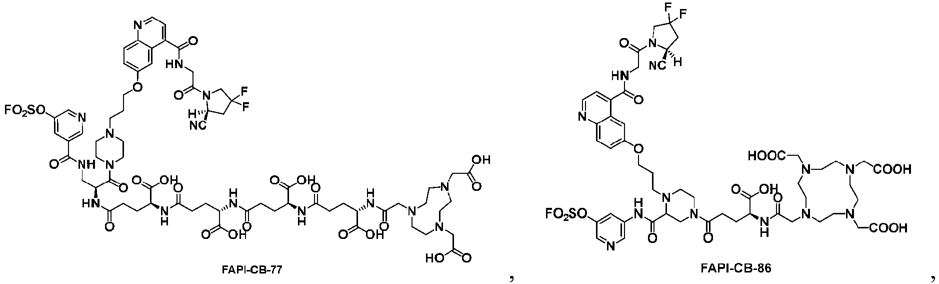 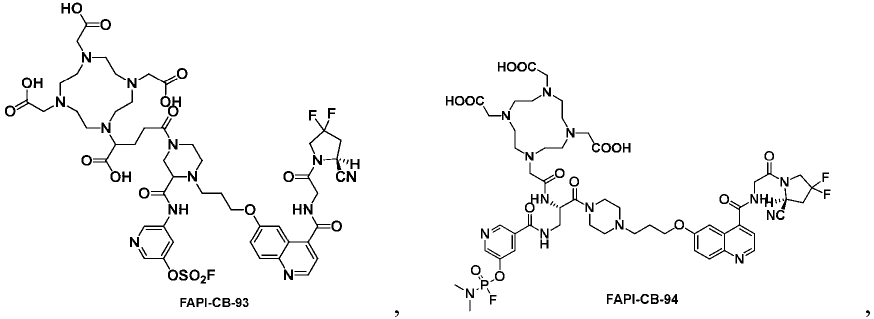 and

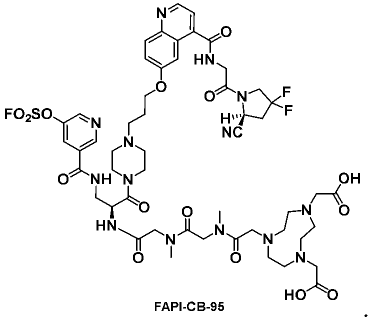

26. The trifunctional compound, or the pharmaceutically acceptable salt, stereoisomer or solvate thereof according to any one of claims 13 to 25, wherein the payload P is a chelating moiety capable of chelating a radionuclide, that is, the payload P chelates a radionuclide.

27. The trifunctional compound, or the pharmaceutically acceptable salt, stereoisomer or solvate thereof according to any one of claims 13 to 26, wherein the radionuclide carried or chelated by the trifunctional compound is a positron nuclide, β emitter, α emitter, an Auger electron-emitting isotope, an X-ray-emitting isotope, a fluorescence-emitting isotope, or a stable metal/non-metal element coordinated with a radionuclide, which is preferably selected from ¹¹C, ¹³N, ¹⁵O, ¹⁸F and its coordinating element, ⁴⁷Sc, ⁵¹Cr, ⁶⁷Ga, ⁶⁸Ga, ⁸⁶Y, ⁹⁰Y, ⁶⁴Cu, ⁶⁷Cu, ⁷²As, ⁷²Se, ⁸⁹Zr, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁴²Pr, ¹⁵¹Eu, ¹⁵³Eu, ¹⁶⁹Eu, ¹⁵⁹Gd, ¹⁶¹Tb, ¹⁷⁷Lu, ¹⁹⁸Au, ¹⁹⁹Ag, ²⁰¹Tl, ²¹¹At, ²⁰³Pb, ²¹²Pb, ²¹²Bi, ²¹³Bi, ²²⁵Ac, ^{99m}Tc, ¹¹¹In, ¹⁴⁹Pm, ¹⁵³Sm, ¹⁶⁵Dy, ¹⁶⁹Er, ¹⁸⁶Re, ¹⁸⁸Re, ¹⁹⁷Hg, ²²⁷Th, ⁶⁷Ga, ⁶⁸Ga, ⁸⁶Y, ⁹⁰Y, ⁵⁵Co, ¹³⁹La, ¹⁴⁰La, ¹⁴⁹Tb, ¹⁵²Tb, ¹⁵⁵Tb, ¹⁶⁶Ho, ¹⁷⁵Yb, ²²⁶Th, ²²³Ra, and ²³⁰U.

28. A pharmaceutical composition comprising the trifunctional compound, or the pharmaceutically acceptable salt, stereoisomer or solvate thereof according to any one of claims 1 to 27, and a pharmaceutically acceptable carrier.

29. A kit comprising or consisting of the trifunctional compound, or the pharmaceutically acceptable salt, stereoisomer or solvate thereof according to any one of claims 1 to 27 or the pharmaceutical composition according to claim 28, and an instruction for diagnosing a disease.

30. A method for diagnosing or treating a disease, preferably a disease involving FAP, wherein the method comprises administering, to a subject, a therapeutically effective dose of the trifunctional compound, or the pharmaceutically acceptable salt, stereoisomer or solvate thereof according to any one of claims 1 to 27, wherein the disease is preferably a central nervous system disease, a metabolic disease, preferably a cardiometabolic disease, or cancer.

31. The method according to claim 30, wherein the cancer is selected from prostate cancer, breast cancer, pancreatic cancer, liver cancer, lung cancer, gastric cancer, renal cancer, ovarian cancer, bladder cancer, esophageal cancer, head and neck cancer, thymic cancer, cervical cancer, endometrial cancer, neuroendocrine tumor, thyroid cancer, intestinal cancer, glioma, and bone metastatic cancer.
